# EUROPEAN PATENT APPLICATION

(11) **EP 4 640 704 A1**
(43) Date of publication of application: **29.10.2025**
(21) Application number: 24172812.0
(22) Date of filing: 26.04.2024
(51) Int. Cl.: C07K 16/24

(54) **ANTIBODIES SPECIFIC TO HUMAN HMGI-C ANTIGEN AND USE THEREOF**

(71) Applicant: PeriCard Check GmbH, 28757 Bremen (DE)
(72) Inventor: ALHOWAIZY, Saman F., 28757 Bremen (DE)
(74) Representative: Peters, Hajo

(57) **Abstract**

The disclosure relates to an (isolated) antibody (or antibody fragment/antigen binding fragment thereof) binding to (human) HMGI-C (anti-HMGI-C-Antibody) as well the nucleic acid encoding such an antibody or host cells comprising these or pharmaceutical composition comprising the antibody. Preferred antibodies are binding to the epitopes of SEQ. ID NOs. 32, 33 or 34, especially the (monoclonal) antibodies that comprise in its heavy chain variable region (VH) the amino acid CDR sequence CDR3 of SEQ ID NO. 4; in its heavy chain variable region (VH) the amino acid CDR sequence CDR3 of SEQ ID NO. 14; or in its heavy chain variable region (VH) the amino acid CDR sequence CDR3 of SEQ ID NO. 24. Especially preferred are also the (monoclonal) antibodies that comprise a heavy chain variable region (VH) having amino acid sequence of SEQ ID NO. 1 and a light chain variable region (VL) having amino acid sequence of SEQ ID NO. 6; that comprise a heavy chain variable region (VH) having amino acid sequence of SEQ ID NO. 11 and a light chain variable region (VL) having amino acid sequence of SEQ ID NO. 16; that comprise a heavy chain variable region (VH) having amino acid sequence of SEQ ID NO. 21 and a light chain variable region (VL) having amino acid sequence of SEQ ID NO. 26. In addition, the disclosure relates to a lateral flow device for the detection of HMGI-C, especially the HMGI-C released during an event leading to an acute aortic syndrome/acute aortic dissection (AAS/AAD) or in relation to cancer in a sample of bodily fluid of a human/patient using an antibody of the disclosure or to the use of the antibody for the treatment of cancer or for cancer prevention by early detection of HMGI-C as a marker for cancer.

## Description

### Field of the Invention

The invention is generally related to HMGI-C belonging to the high mobility group protein relevant both in the area or cardiology, specifically acute aortic dissection (AAD), as well as in the area of cancer in its treatment and detection and to antibodies that specifically bind HMGI-C. Especially, the invention relates to an (isolated) antibody (or antibody fragment/antigen binding fragment thereof) binding to (human) HMGI-C (anti-HMGI-C-Antibody) as well the nucleic acid encoding such an antibody or host cells comprising these or pharmaceutical composition comprising the antibody. Preferred antibodies are binding to the epitopes of SEQ. ID NOs. 32, 33 or 34, especially the (monoclonal) antibodies that comprise in its heavy chain variable region (VH) the amino acid CDR sequence CDR3 of SEQ ID NO. 4; in its heavy chain variable region (VH) the amino acid CDR sequence CDR3 of SEQ ID NO. 14; or that comprise in its heavy chain variable region (VH) the amino acid CDR sequence CDR3 of SEQ ID NO. 24. Especially preferred are also the (monoclonal) antibodies that comprise a heavy chain variable region (VH) having amino acid sequence of SEQ ID NO. 1 and a light chain variable region (VL) having amino acid sequence of SEQ ID NO. 6; that comprise a heavy chain variable region (VH) having amino acid sequence of SEQ ID NO. 11 and a light chain variable region (VL) having amino acid sequence of SEQ ID NO. 16; that comprise a heavy chain variable region (VH) having amino acid sequence of SEQ ID NO. 21 and a light chain variable region (VL) having amino acid sequence of SEQ ID NO. 26.. In addition, the invention relates to a lateral flow device for the detection of HMGI-C especially the HMGI-C released during an event leading to an acute aortic syndrome/acute aortic dissection (AAS/AAD) or in relation to cancer in a sample of bodily fluid of a human/patient using an antibody of the invention or to the use of the antibody for the treatment of cancer or for cancer prevention by early detection of HMGI-C as a marker for cancer.

### Background of the Invention

The invention is generally related to HMGI-C and antibodies that specifically bind HMGI-C. HMGI-C belongs to the high mobility group proteins and is relevant in the area or cardiology as well as in the area of cancer. The first interest of the inventors rested in the field of cardiology with a specific focus on acute aortic dissection (AAD).

Acute aortic dissection (AAD) is a life-threatening clinical entity requiring urgent surgical intervention. It is one aspect of acute aortic syndrome (AAS). The peak incidence of aortic dissection is reported to occur in the sixth and seventh decades of life with 2000 new cases per year in North America and 3000 in Europe, respectively.^{1,2} The initiating event is an intimal tear due to congenital defects of the aortic wall, such as in Marfan syndrome (MFS), Ehlers-Danlos syndrome (a heterogeneous group connective tissue disorders), idiopathic Erdheim Gsell medial necrosis, bicuspid aortic valve (the most common congenital heart disorder), or even acquired intima damage caused by hypertension, thoracic trauma or other unknown mechanism.³⁻⁵ Subintima or media necrosis results in intimal tear, which eventually allows blood to enter into the aortic wall, thus leading to separation of the aortic wall layers and hence to a rapid propagation of the aortic dissection. The separation of the media and the adventitia layers of the aortic wall may lead to malperfusion of numerous organs with devastating complications.^{2,6} An important clinical fact is that aortic dissections occur in both aneurysmatic expanded calibre as well as in normal calibre of the aorta. The most feared complication of aortic dissection is its outward rupture, which is associated with high mortality.⁷ The surgical treatment depends on the location of the initial tear and on the longitudinal propagation of the dissection. To describe dissections various classifications have been proposed, with the DeBakey and the Stanford classification more often used.^{8,9} When the ascending aorta is affected (Stanford type A), the mortality of untreated patients is about 36%-72% within the first 48 hours and 62%-91 % within the first week.¹⁰⁻¹³ Chest pain and other symptoms of ADD are often confused with those of cardiac infarction, thus complicating the diagnosis and delaying the treatment of AAD. Therefore, a better understanding of the molecular mechanisms underlying AAD can be the first step to supporting the future development of a rapid test for monitoring patients at high risk.

As said above, the initiating event is an intimal tear due to defects of the aortic wall. The dissection of these layers occurs after the aortic rupture. After 5-10 min of the onset of pain, the biological marker HMGI-C is released in the blood. The biomarker level remains elevated for 6-10 days since the pain onset, thereby providing a greater one-time window for the detection of aortic wall damage. The high specificity of the biomarker measurements to the aortic wall damage was verified during surgical interventions, which consequently become a biomarker indicative of aortic dissection.

Given that fact it would be a huge benefit to provide a diagnostic antibody that can suitably detect HMGI-C released during an event leading to an acute aortic syndrome/acute aortic dissection in samples from a human/patient, especially in a timely manner as time is all-important in this indication (e.g. a lateral flow test using such an antibody).

In addition, HMGI-C or HMGA2 (high mobility group AT-hook 2) plays a role in cancer.¹⁶ It promotes cancer metastasis by regulating epithelial-mesenchymal transition.¹⁷ Epithelial-mesenchymal transition (EMT) is a complex physiological process that transforms polarized epithelial cells into moving mesenchymal cells and the dysfunction of EMT promotes the invasion and metastasis of cancer. HMGI-C/HMGA2 is highly overexpressed in highly overexpressed in various types of cancer and thus can act as a target for early detection of cancer as well as for cancer treatment.

Thus, highly specific antibodies against HMGI-C are of tremendous benefit also for the treatment of cancer.

The inventors have identified 3 EPITOPES on human HMGI-C and developed antibodies which bind to these EPITOPES. Finding these antibodies was a very difficult task and these antibodies have - at least partly due to the selection of the epitopes and partly due to their specific nature - the benefit of being able to detect all 4 subtypes of HMGI-C, especially in body fluids. The first two groups of antibodies are exemplified by 4-H2-2-G6-E12 and 43-3-C5-A6. These two groups of antibodies are specifically binding to the EPITOPES 1 (QKRGRGRPRKQQQE) and 2 (IFSRDRVSP), respectively. The 3^{rd} group of antibodies - exemplified by 141-3-F2-H4 - is specific to EPITOPE 3 (RGRPRKWAGVQWYNLGSLQ) but has also binding to EPITOPE 1 due to a 6 amino acid overlap. It has a high detection value for HMGI-C in body fluids.

### Summary of the Invention

In principle, the application is drawn to antibodies which specifically bind to the EPITOPES 1 (QKRGRGRPRKQQQE), 2 (IFSRDRVSP) and 3 (RGRPRKWAGVQWYNLGSLQ) of HMGI-C. These epitopes were specifically selected to allow a high level of detection of HMGI-C (belonging to the high mobility group proteins) especially allowing detection of all 4 subtypes, particularly in body fluids.

In one aspect the invention is thus drawn to the first 2 groups of antibodies which are exemplified by 4-H2-2-G6-E12 and 43-3-C5-A6. These are specific to the EPITOPES 1 (QKRGRGRPRKQQQE) and 2 (IFSRDRVSP) of HMGIC, respectively. In another aspect the invention is drawn to a 3^{rd} group of antibodies - exemplified by 141-3-F2-H4 - which is specific to EPITOPE 3 (RGRPRKWAGVQWYNLGSLQ).

In a **first** aspect, the invention is drawn to an (isolated) antibody (**exemplified by 4-H2-2-G6-E12**) (or antibody derivative/antigen binding fragment thereof), wherein the antibody (or antibody derivative/antigen binding fragment thereof) comprises
- in the heavy chain variable region ONE (VH-1) one (the) amino acid CDR sequence CDR3 of SEQ ID NO. 4.

In a preferred embodiment of this **first** aspect this (isolated) antibody (or antibody derivative/antigen binding fragment thereof) according to the invention, the antibody (or antibody derivative/antigen binding fragment thereof) comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;

In another preferred embodiment of this **first** aspect this (isolated) antibody (or antibody derivative/antigen binding fragment thereof) according to the invention, the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 1 and a light chain with at least 50%, 60%, 70%, 80%, 85%, 90%, 95%, 99% or 100% sequence identity to SEQ ID NO: 6, especially a heavy chain with at least 95%, 99% or 100% sequence identity to SEQ ID NO: 1 and a light chain with at least 95%, 99% or 100% sequence identity to SEQ ID NO: 6; preferably a heavy chain with at least 99% or 100% sequence identity to SEQ ID NO: 1 and a light chain with at least 99% or 100% sequence identity to SEQ ID NO: 6; most preferably a heavy chain with 100% sequence identity to SEQ ID NO: 1 and a light chain with 100% sequence identity to SEQ ID NO: 6.

In a **second** aspect, the invention is drawn to an (isolated) antibody (**exemplified by 43-3-C5-A6**) (or antibody derivative/antigen binding fragment thereof), wherein the antibody (or antibody derivative/antigen binding fragment thereof) comprises
- in the heavy chain variable region ONE (VH-1) one (the) amino acid CDR sequence CDR3 of SEQ ID NO. 14.

In a preferred embodiment of this **second** aspect this (isolated) antibody (or antibody derivative/antigen binding fragment thereof) according to the invention, the antibody (or antibody derivative/antigen binding fragment thereof) comprises comprises
- in the heavy chain variable region ONE (VH-1) amino acid sequence CDR1 of SEQ ID NO.12, amino acid sequence CDR2 of SEQ ID NO.13, and amino acid sequence CDR3 of SEQ ID NO.14 and
- in the light chain variable region ONE (VL-1) amino acid sequence CDR1 of SEQ ID NO. 17, amino acid sequence CDR2 of SEQ ID NO. 18 and amino acid sequence CDR3 of SEQ ID NO. 19.

In a preferred embodiment of this **second** aspect this (isolated) antibody (or antibody derivative/antigen binding fragment thereof) according to the invention, the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 11 and a light chain with at least 50%, 60%, 70%, 80%, 85%, 90%, 95%, 99% or 100% sequence identity to SEQ ID NO: 16, especially a heavy chain with at least 95%, 99% or 100% sequence identity to SEQ ID NO: 11 and a light chain with at least 95%, 99% or 100% sequence identity to SEQ ID NO: 16; preferably a heavy chain with at least 99% or 100% sequence identity to SEQ ID NO: 11 and a light chain with at least 99% or 100% sequence identity to SEQ ID NO: 16; most preferably a heavy chain with 100% sequence identity to SEQ ID NO: 11 and a light chain with 100% sequence identity to SEQ ID NO: 16.

In a **third** aspect, the invention is drawn to an (isolated) antibody **(exemplified by 141-3-F2-H4)** (or antibody derivative/antigen binding fragment thereof), wherein the antibody (or antibody derivative/antigen binding fragment thereof) comprises in the heavy chain variable region (VH) (THREE (VH-3)) at least one of the amino acid CDR sequences CDR1 of SEQ ID NO. 22, CDR2 of SEQ ID NO. 23 and CDR3 of SEQ ID NO. 24.

In a preferred embodiment of this **third** aspect this (isolated) antibody (or antibody derivative/antigen binding fragment thereof) according to the invention, the antibody (or antibody derivative/antigen binding fragment thereof) comprises
- in the heavy chain variable region THREE (VH-3) (the three CDRs of) amino acid sequence CDR1 of SEQ ID NO.22, amino acid sequence CDR2 of SEQ ID NO.23, and amino acid sequence CDR3 of SEQ ID NO.24 and
- in the light chain variable region THREE (VL-3) amino acid sequence CDR1 of SEQ ID NO. 27, amino acid sequence CDR2 of SEQ ID NO. 28 and amino acid sequence CDR3 of SEQ ID NO. 29.

In another preferred embodiment of this **third** aspect this (isolated) antibody (or antibody derivative/antigen binding fragment thereof) according to the invention, the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 21 and a light chain with at least 50%, 60%, 70%, 80%, 85%, 90%, 95%, 99% or 100% sequence identity to SEQ ID NO: 26, especially a heavy chain with at least 95%, 99% or 100% sequence identity to SEQ ID NO: 21 and a light chain with at least 95%, 99% or 100% sequence identity to SEQ ID NO: 26; preferably a heavy chain with at least 99% or 100% sequence identity to SEQ ID NO: 21 and a light chain with at least 99% or 100% sequence identity to SEQ ID NO: 26; most preferably a heavy chain with 100% sequence identity to SEQ ID NO: 21 and a light chain with 100% sequence identity to SEQ ID NO: 26.

In other aspects, the invention is drawn to a lateral flow device for the detection of HMGI-C especially the HMGI-C released during an event leading to an acute aortic syndrome/acute aortic dissection (AAS/AAD) or in relation to cancer in a sample of bodily fluid of a human/patient using an antibody of the invention or to the use of the antibody for the treatment of cancer or for cancer prevention by early detection of HMGI-C as a marker for cancer.

Thus, in another aspect the invention relates to a lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
   wherein the 1-AAD-protein is human HMGI-C and the protein antibody binding to said 1-AAD-protein is an antibody of any one of the **first, second orthird** aspects of the invention.

In another aspect the invention relates to an article of manufacture or medical device comprising a container and a composition contained therein, wherein the composition comprises an antibody (or antibody derivative/antigen binding fragment thereof) of any one of the **first, second or third** aspects of the invention, preferably wherein the medical device is a lateral flow device for detection of an antigen, most preferably wherein the medical device is a lateral flow device for detection of the presence of human HMGI-C in a body fluid, like blood, whole blood, blood plasma or serum.

In another aspect the invention relates to a method of treating a HMGI-C positive cancer, comprising administering to a patient suffering from the cancer, a therapeutically effective amount of the antibody (or antibody derivative/antigen binding fragment thereof) of any one of the **first, second or third** aspects of the invention; or
the invention relates to an antibody (or antibody derivative/antigen binding fragment thereof) of any one of the **first, second or third** aspects of the invention for use in a method of treating HMGI-C positive cancer, wherein the method comprises the step of contacting a HMGI-C-positive cancer in a subject with said antibody or antibody derivative/antigen binding fragment thereof.

In another aspect the invention relates to an (isolated) nucleic acid that encodes the antibody (or antibody derivative/antigen binding fragment thereof) of any one of the **first, second or third** aspects of the invention.

In another aspect the invention relates to an expression vector encoding the antibody (or antibody derivative/antigen binding fragment thereof) of any one of the **first, second or third** aspects of the invention.

In another aspect the invention relates to a host cell comprising a nucleic acid according to Embodiment E-B34 or an expression vector according to Embodiment **E-B41.**

In another aspect the invention relates to a method of producing the antibody (or antibody derivative/antigen binding fragment thereof) of any one of the **first, second or third** aspects of the invention, comprising culturing the host cell that produces the antibody and recovering the antibody from the cell culture.

In another aspect the invention relates to a composition comprising the antibody (or antibody derivative/antigen binding fragment thereof) of any one of the **first, second or third** aspects of the invention and a carrier.

### BRIEF DESCRIPTION OF THE FIGURES

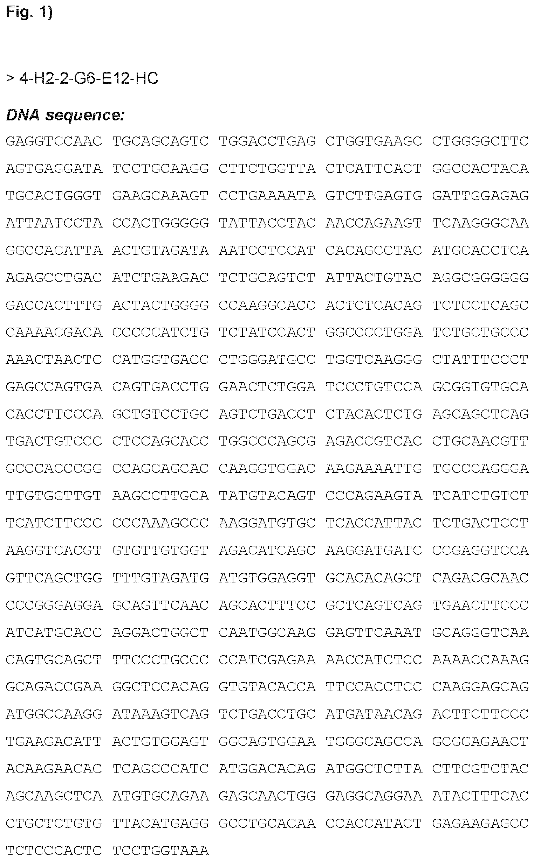

**Fig. 1**) Nucleic acid sequence of the heavy chain variable domain (VH) of the antibody 4-H2 (SEQ ID NO. 5).

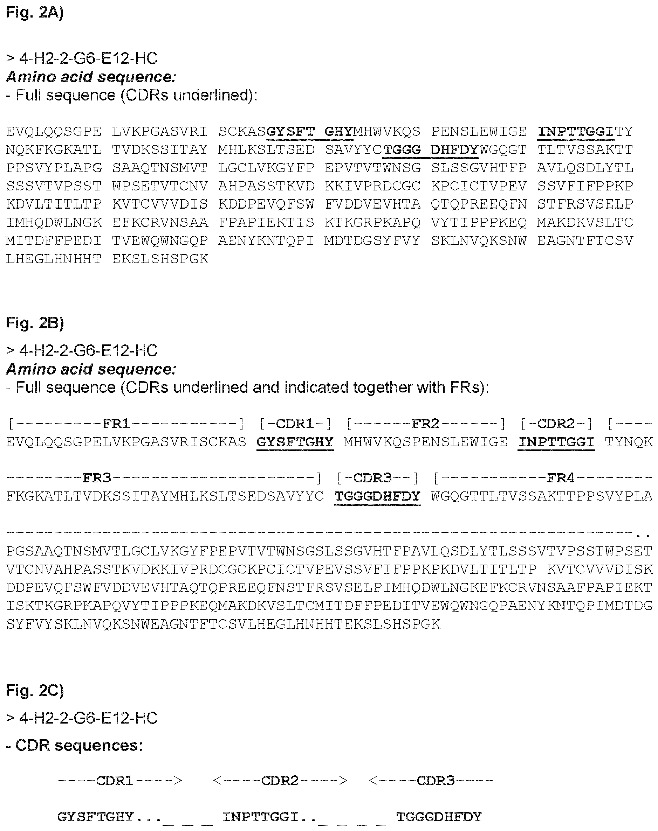

**Fig. 2A**) Amino acid sequence of the heavy chain variable domain (VH) of the antibody 4-H2 (SEQ ID NO. 1). The CDRs of VH of 4-H2 are underlined and are as follow: CDR1 (SEQ ID NO. 2), CDR2 (SEQ ID NO. 3), and CDR3 (SEQ ID NO. 4).
**Fig. 2B**) Amino acid sequence of the heavy chain variable domain (VH) of the antibody 4-H2 (SEQ ID NO. 1). The CDR1 (SEQ ID NO. 2), CDR2 (SEQ ID NO. 3) and CDR3 (SEQ ID NO. 4) in the chain are shown underlined, flanked by the framework regions, FR1 - FR4, as indicated.
**Fig. 2C**) Amino acid sequence of the CDRs [CDR1 (SEQ ID NO. 2), CDR2 (SEQ ID NO. 3) and CDR3 (SEQ ID NO. 4)] of the heavy chain variable domain (VH) of the antibody 4-H2 (SEQ ID NO. 1).

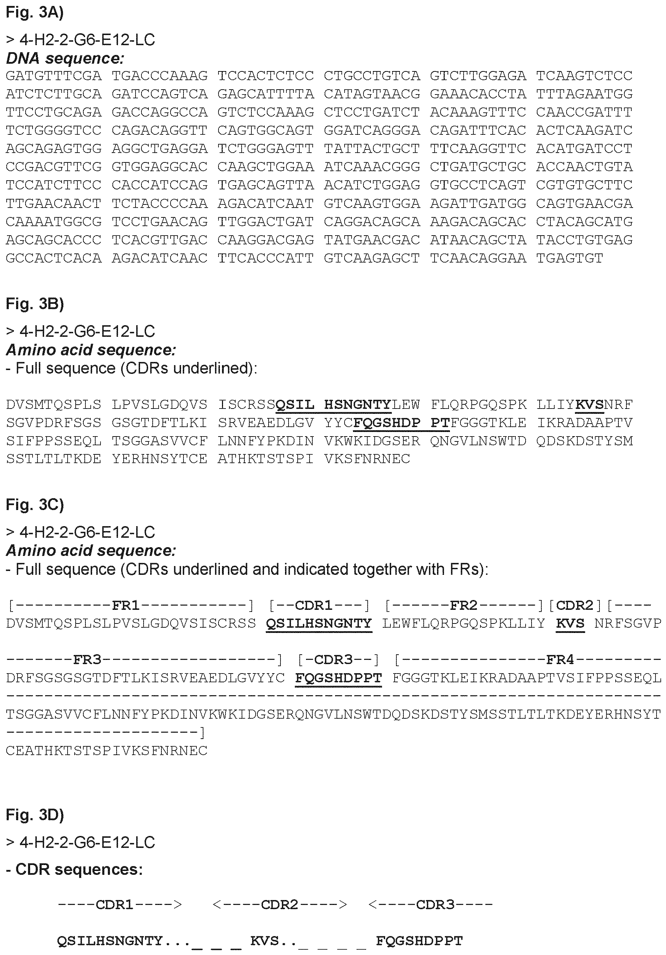

**Fig. 3A**) Nucleic acid sequence of the light chain variable domain (VL) of the antibody 4-H2 (SEQ ID NO. 10).
**Fig. 3B**) Amino acid sequence of the light chain variable domain (VL) of the antibody 4-H2 (SEQ ID NO. 6). The CDRs of VL of 4-H2 are underlined and are as follow: CDR1 (SEQ ID NO. 7), CDR2 (SEQ ID NO. 8), and CDR3 (SEQ ID NO. 9).
**Fig. 3C**) Amino acid sequence of the light chain variable domain (VL) of the antibody 4-H2 (SEQ ID NO. 6). The CDR1 (SEQ ID NO. 7), CDR2 (SEQ ID NO. 8) and CDR3 (SEQ ID NO. 9) in the chain are shown underlined, flanked by the framework regions, FR1 - FR4, as indicated.
**Fig. 3D**) Amino acid sequence of the CDRs [CDR1 (SEQ ID NO. 7), CDR2 (SEQ ID NO. 8) and CDR3 (SEQ ID NO. 9)] of the light chain variable domain (VL) of the antibody 4-H2 (SEQ ID NO. 1).

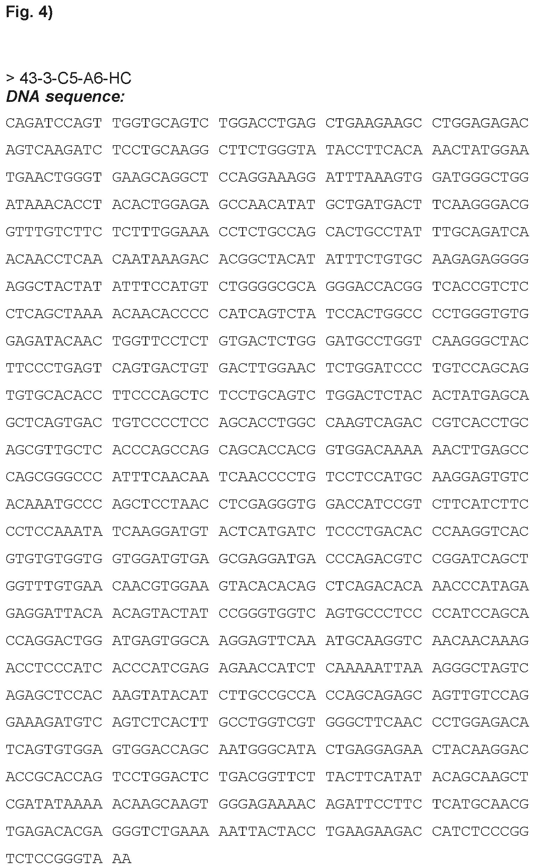

**Fig. 4**) Nucleic acid sequence of the heavy chain variable domain (VH) of the antibody 43-3 (SEQ ID NO. 15).

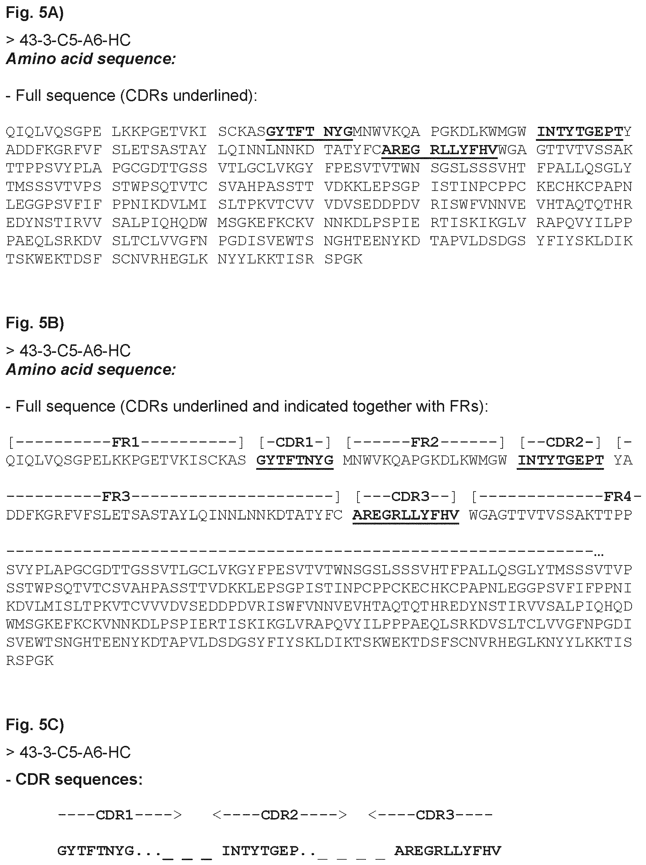

**Fig. 5A**) Amino acid sequence of the heavy chain variable domain (VH) of the antibody 43-3 (SEQ ID NO. 11). The CDRs of VH of 43-3 are underlined and are as follow: CDR1 (SEQ ID NO. 12), CDR2 (SEQ ID NO. 13), and CDR3 (SEQ ID NO. 14).
**Fig. 5B**) Amino acid sequence of the heavy chain variable domain (VH) of the antibody 43-3 (SEQ ID NO. 11). The CDR1 (SEQ ID NO. 12), CDR2 (SEQ ID NO. 13) and CDR3 (SEQ ID NO. 14) in the chain are shown underlined, flanked by the framework regions, FR1 - FR4, as indicated.
**Fig. 5C**) Amino acid sequence of the CDRs [CDR1 (SEQ ID NO.2), CDR2 (SEQ ID NO.3) and CDR3 (SEQ ID NO. 14)] of the heavy chain variable domain (VH) of the antibody 43-3 (SEQ ID NO. 1).

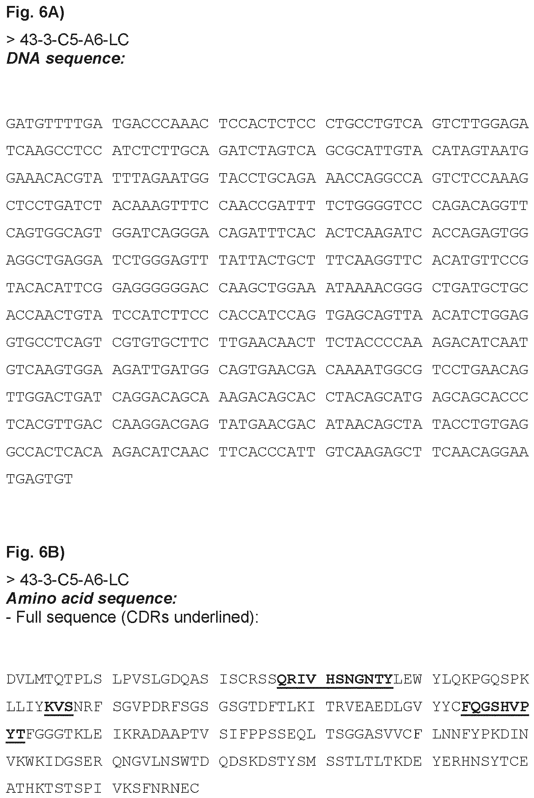

**Fig. 6A**) Nucleic acid sequence of the light chain variable domain (VL) of the antibody 43-3 (SEQ ID NO. 20).
**Fig. 6B**) Amino acid sequence of the light chain variable domain (VL) of the antibody 43-3 (SEQ ID NO. 16). The CDRs of VL of 43-3 are underlined and are as follow: CDR1 (SEQ ID NO. 17), CDR2 (SEQ ID NO. 18), and CDR3 (SEQ ID NO. 19).

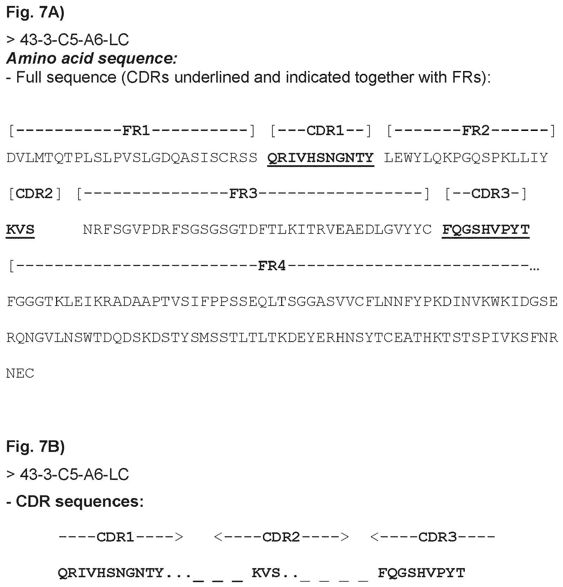

**Fig. 7A**) Amino acid sequence of the light chain variable domain (VL) of the antibody 43-3 (SEQ ID NO. 16). The CDR1 (SEQ ID NO. 17), CDR2 (SEQ ID NO. 18) and CDR3 (SEQ ID NO. 19) in the chain are shown underlined, flanked by the framework regions, FR1 - FR4, as indicated.
**Fig. 7B**) Amino acid sequence of the CDRs [CDR1 (SEQ ID NO. 17), CDR2 (SEQ ID NO. 18) and CDR3 (SEQ ID NO. 19)] of the light chain variable domain (VL) of the antibody 43-3 (SEQ ID NO. 1).

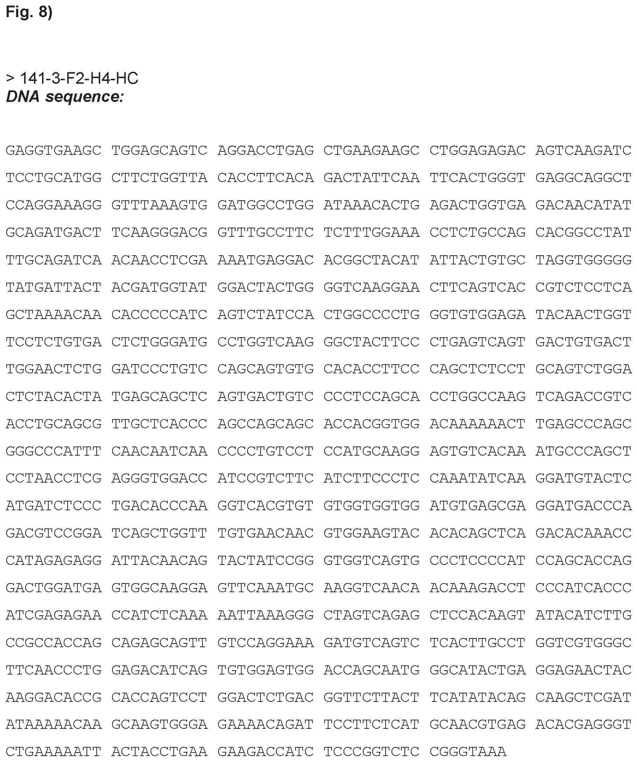

**Fig. 8**) Nucleic acid sequence of the heavy chain variable domain (VH) of the antibody 141-3 (SEQ ID NO. 15).

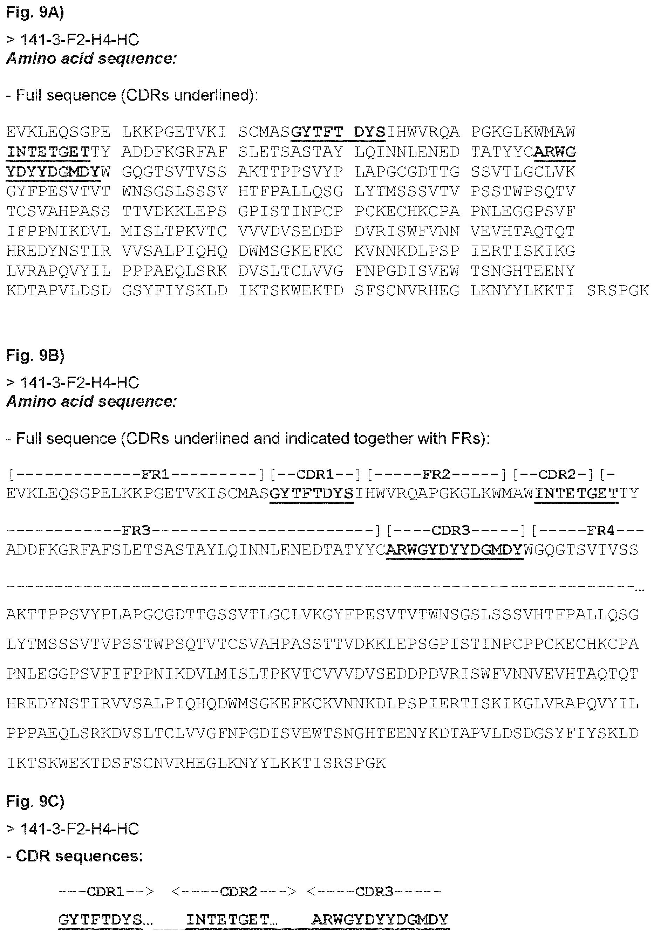

**Fig. 9A**) Amino acid sequence of the heavy chain variable domain (VH) of the antibody 141-3 (SEQ ID NO. 11). The CDRs of VH of 43-3 are underlined and are as follow: CDR1 (SEQ ID NO. 12), CDR2 (SEQ ID NO. 13), and CDR3 (SEQ ID NO. 14).
**Fig. 9B**) Amino acid sequence of the heavy chain variable domain (VH) of the antibody 141-3 (SEQ ID NO. 11). The CDR1 (SEQ ID NO. 12), CDR2 (SEQ ID NO. 13) and CDR3 (SEQ ID NO. 14) in the chain are shown underlined, flanked by the framework regions, FR1 - FR4, as indicated.
**Fig. 9C**) Amino acid sequence of the CDRs [CDR1 (SEQ ID NO.2), CDR2 (SEQ ID NO.3) and CDR3 (SEQ ID NO. 14)] of the heavy chain variable domain (VH) of the antibody 141-3 (SEQ ID NO. 1).

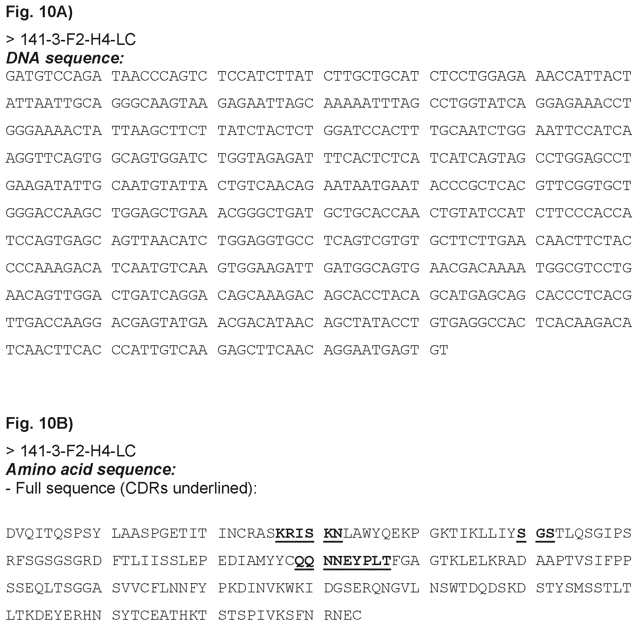

**Fig. 10A**) Nucleic acid sequence of the light chain variable domain (VL) of the antibody 141-3 (SEQ ID NO. 20).
**Fig. 10B**) Amino acid sequence of the light chain variable domain (VL) of the antibody 141-3 (SEQ ID NO. 16). The CDRs of VL of 43-3 are underlined and are as follow: CDR1 (SEQ ID NO. 17), CDR2 (SEQ ID NO. 18), and CDR3 (SEQ ID NO. 19).

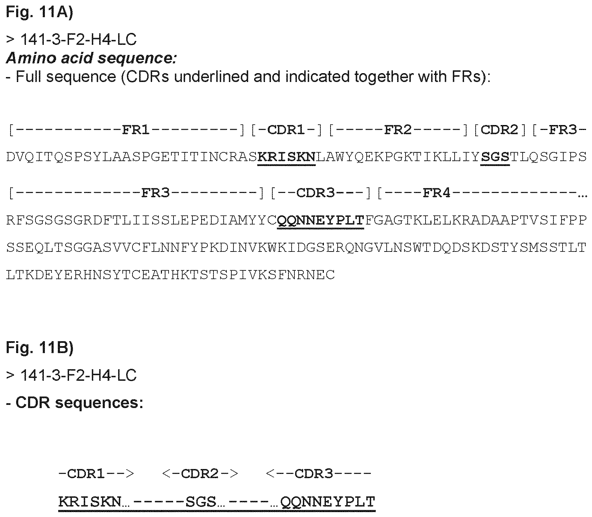

**Fig. 11A**) Amino acid sequence of the light chain variable domain (VL) of the antibody 141-3 (SEQ ID NO. 16). The CDR1 (SEQ ID NO. 17), CDR2 (SEQ ID NO. 18) and CDR3 (SEQ ID NO. 19) in the chain are shown underlined, flanked by the framework regions, FR1 - FR4, as indicated.
**Fig. 11B**) Amino acid sequence of the CDRs [CDR1 (SEQ ID NO. 17), CDR2 (SEQ ID NO. 18) and CDR3 (SEQ ID NO. 19)] of the light chain variable domain (VL) of the antibody 141-3 (SEQ ID NO. 1).
**Fig. 12A**) Amino acid sequence of human HMGI-C (High mobility group protein)
**Fig. 12B**) Amino acid sequence of human HMGI-C (SEQ ID NO. 31) with Epitope 1 and Epitope 2 shown and marked.
**Fig. 12C**) Amino acid sequence of an epitope (designated Epitope 1) of human HMGI-C (SEQ ID NO. 32).
**Fig. 12D**) Amino acid sequence of an epitope (designated Epitope 2) of human HMGI-C (SEQ ID NO. 33).
**Fig. 12E**) Amino acid sequence of an epitope (designated Epitope 3) of human HMGI-C (SEQ ID NO. 34).

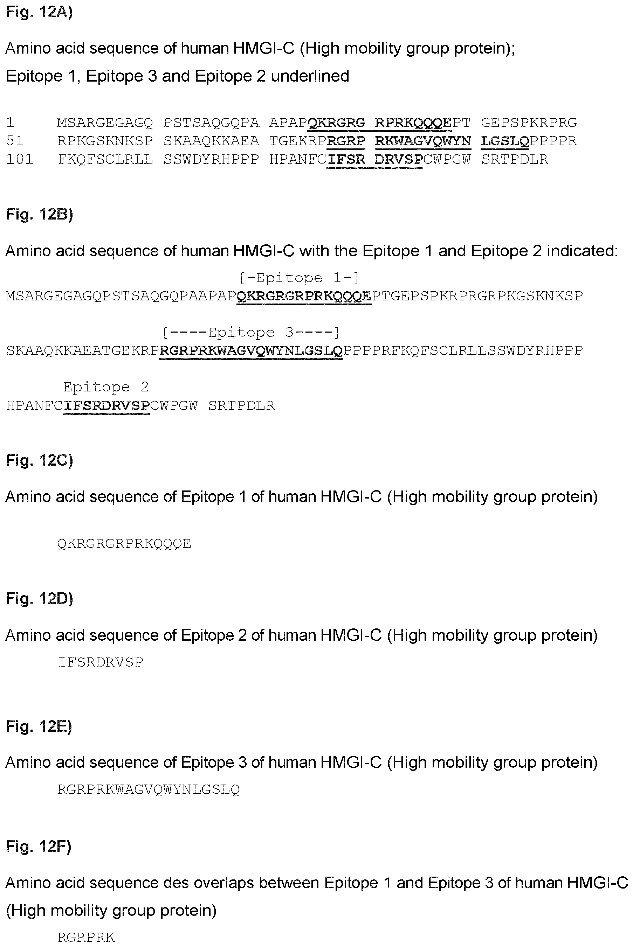

**Fig. 12D**) Amino acid sequence of the overlap between Epitope 1 and Epitope 3 of human HMGI-C (SEQ ID NO. 35).

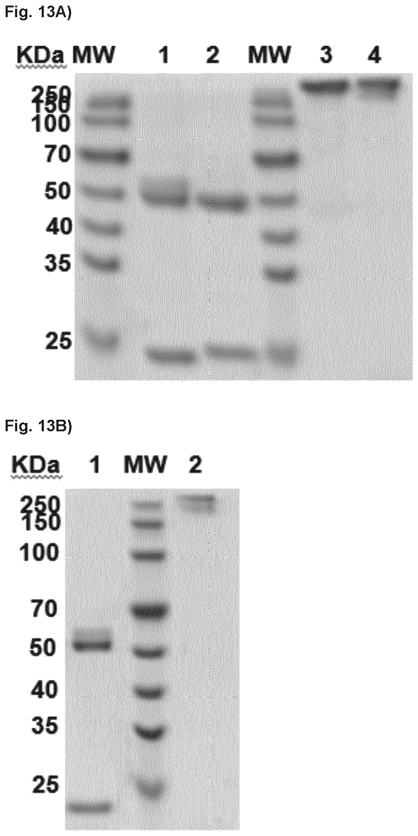

**Fig. 13A**) This figure shows an SDS PAGE and shows the purified antibody production from the final clones 43-3-C5-A6 and 4-H2-2-G6-E12 of Example 1 with
   - lane no. 1 showing antibody-43-3-C5-A6 (2µg, 10% reduce SDS-PAGE)
   - lane no. 2 showing antibody-4-H2-2-G6-E12 (2µg, 10% reduce SDS-PAGE)
   - lane no. 3 showing antibody-43-3-C5-A6 (2µg, 10% non-reduce SDS-PAGE)
   - lane no. 4 showing antibody-4-H2-2-G6-E12 (2µg, 10% non-reduce SDS-PAGE).
**Fig. 13B**) This figure shows an SDS PAGE and shows the purified antibody production from the final clone 141-3-F2-H4 of Example 1 with
   - lane no. 1 showing antibody-141-3-F2-H4 (2µg, 10% reduce SDS-PAGE)
   - lane no. 2 showing antibody-141-3-F2-H4 (2µg, 10% non-reduce SDS-PAGE)

### Detailed Description of the invention

At a main aspect, the invention relates to an (isolated) antibody or an antibody derivative that bind to human HMGI-C, wherein the antibody comprises
- in the heavy chain variable region ONE (VH-1) one amino acid CDR sequence CDR3 of SEQ ID NO. 4;
   OR
- in the heavy chain variable region TWO (VH-2) at least one amino acid CDR sequence CDR3 of SEQ ID NO. 14.

These antibodies - exemplified by 4-H2-2-G6-E12 and 43-3-C5-A6 and binding to EPITOPE 1 (SEQ ID NO: 32) and EPITOPE 2 (SEQ ID NO: 33) - were quite difficult to obtain and have - at least partly due to the selection of the epitopes and partly due to their specific nature - the benefit of being able to detect all 4 subtypes of HMGI-C, especially in body fluids like blood serum or blood.

A preferred embodiment of the invention relates to the (isolated) antibody or antibody derivative of the invention, wherein the antibody or antibody derivative is effective to identify the presence of human HMGI-C in liquids *in vitro*; and/or
wherein the antibody or antibody derivative is effective to treat cancer related to human HMGI-C.

Another preferred embodiment of the invention relates to the (isolated) antibody or antibody derivative of the invention, wherein the antibody or antibody derivative comprises
- in the heavy chain variable region ONE (VH-1) together with the amino acid sequence CDR3 of SEQ ID NO. 4 the amino acid sequence CDR1 of SEQ ID NO. 2 and the amino acid sequence CDR2 of SEQ ID NO. 3;
   OR
- in the heavy chain variable region TWO (VH-2) together with the amino acid sequence CDR3 of SEQ ID NO. 14 the amino acid sequence CDR1 of SEQ ID NO. 12 and the amino acid sequence CDR2 of SEQ ID NO. 13.

Another preferred embodiment of the invention relates to the (isolated) antibody or antibody derivative of the invention, wherein the antibody or antibody derivative comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

Another preferred embodiment of the invention relates to the (isolated) antibody or antibody derivative of the invention, wherein the antibody or antibody derivative comprises:
a) a heavy chain variable region ONE (VH-1) encoded by the nucleic acid sequence of SEQ ID NO. 5 and a light chain variable region ONE (VL-1) encoded by the nucleic acid sequence of SEQ ID NO. 10; or
b) a heavy chain variable region ONE (VH-1) having amino acid sequence of SEQ ID NO. 1 and a light chain variable region ONE (VL-1) having amino acid sequence of SEQ ID NO. 6;
OR
a) a heavy chain variable region TWO (VH-2) encoded by nucleic acid sequences of SEQ ID NO. 15 and a light chain variable region TWO (VL-2) encoded by nucleic acid sequences of SEQ ID NO. 20; or
b) a heavy chain variable region TWO (VH-2) having amino acid sequence of SEQ ID NO. 11 and a light chain variable region TWO (VL-2) having amino acid sequence of SEQ ID NO. 16.

Another preferred embodiment of the invention relates to the (isolated) antibody or antibody derivative of the invention, wherein
- for the antibody with the heavy chain variable region ONE (VH-1) with the CDR sequence CDR3 of SEQ ID NO. 4, the antibody or antibody derivative comprises a heavy chain variable region with at least 95%, 99% or 100% sequence identity to SEQ ID NO: 1 and a light chain variable region with at least 95%, 99% or 100% sequence identity to SEQ ID NO: 6;
   OR
- for the antibody with the heavy chain variable region TWO (VH-2) with the CDR sequence CDR3 of SEQ ID NO. 14, the antibody or antibody derivative comprises a heavy chain variable region with at least 95%, 99% or 100% sequence identity to SEQ ID NO: 11 and a light chain variable region with at least 95%, 99% or 100% sequence identity to SEQ ID NO: 16.

Another preferred main aspect of the invention relates to an (isolated) antibody or an antibody derivative that bind to human HMGI-C, wherein the antibody comprises
- in the heavy chain variable region THREE (VH-3) (the three CDRs of) amino acid sequence CDR1 of SEQ ID NO.22, amino acid sequence CDR2 of SEQ ID NO.23, and amino acid sequence CDR3 of SEQ ID NO.24 and
- in the light chain variable region THREE (VL-3) amino acid sequence CDR1 of SEQ ID NO. 27, amino acid sequence CDR2 of SEQ ID NO. 28 and amino acid sequence CDR3 of SEQ ID NO. 29.

These antibodies - exemplified by exemplified by 141-3-F2-H4 and binding to EPITOPE 3 (SEQ ID NO: 34) - were also quite difficult to obtain and have - at least partly due to the selection of the epitopes and partly due to their specific nature - also the benefit of being able to detect all 4 subtypes of HMGI-C, especially in body fluids like blood serum or blood, but are also binding to EPITOPE 1 (SEQ ID NO: 32) due to a 6 amino acid overlap (SEQ ID NO:35). These antibodies have thus a high detection value for HMGI-C in body fluids.

Another preferred embodiment of this other main aspect of the invention relates to the (isolated) antibody or antibody derivative of the invention, wherein the antibody or antibody derivative comprises a heavy chain variable region with at least 95%, 99% or 100% sequence identity to SEQ ID NO: 21 and a light chain variable region with at least 95%, 99% or 100% sequence identity to SEQ ID NO: 26.

Another preferred embodiment of the invention relates to the (isolated) antibody or antibody derivative of the invention,
- wherein the antibody is a murine antibody, such as an IgG, or
- wherein said antibody is a human or humanized antibody, especially a humanized antibody, in particular wherein said humanized antibody is an IgG and/or
- wherein said antibody is a monoclonal antibody; and/or
- wherein said antibody derivative is a Fab fragment; and/or
- wherein the said antibody or the said antibody derivative
   ∘ which comprises in the heavy chain variable region ONE (VH-1) amino acid CDR sequence CDR3 of SEQ ID NO. 4, binds to Epitope 1 of SEQ ID NO. 32;
   ∘ which comprises in the heavy chain variable region TWO (VH-2) amino acid CDR sequence CDR3 of SEQ ID NO. 14, binds to Epitope 2 of SEQ ID NO. 33.

Another preferred aspect of the invention relates to an isolated nucleic acid or an expression vector that encodes the antibody or antibody derivative according to the invention.

Another preferred aspect of the invention relates to a host cell comprising said nucleic acid or said expression vector according to the invention, preferably wherein the host cell produces the antibody or antibody derivative according to the invention.

Another preferred aspect of the invention relates to a method of producing the antibody or antibody derivative according to the invention, comprising culturing the host cell according to the invention that produces the antibody and recovering the antibody from the cell culture.

Another preferred aspect of the invention relates to a composition comprising the antibody or antibody derivative according to the invention and a carrier, preferably a pharmaceutically acceptable carrier.

Another preferred aspect of the invention relates to an article of manufacture or medical device comprising a container and a composition contained therein, wherein the composition comprises an antibody or antibody derivative according to the invention, preferably wherein the medical device is a lateral flow device for detection of an antigen, most preferably herein the medical device is a lateral flow device for detection of the presence of human HMGI-C in a body fluid, like blood, whole blood, blood plasma or serum.

Another preferred aspect of the invention relates to an antibody according to the invention for use in a method of treating HMGI-C positive cancer, wherein the method comprises the step of contacting a HMGI-C-positive cancer in a subject with said antibody or antibody derivative, preferably wherein said HMGI-C positive cancer is selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.

### Definitions

Throughout this specification, unless the context requires otherwise, the words "comprise," "comprises," and "comprising" will be understood to imply the inclusion of a stated step or element or group of steps or elements but not the exclusion of any other step or element or group of steps or elements. It should also be understood that a term with the words "comprise," "comprises," and "comprising" can be in a limiting way be replaced to mean the words "consists of', "consists of" and "consisting of", and then be understood to imply the inclusion of a stated step or element or group of steps or elements but the exclusion of any other step or element or group of steps or elements.

The term "antibody", also referred to as "immunoglobulin", is a Y-shaped protein of the immune system that specifically identifies foreign objects or antigens, such as the components of bacteria, yeasts, parasites, and viruses. Usually and especially in humans and unless modified (see below), an "antibody" has four polypeptide chains, two identical heavy chains and two identical light chains. Therein, each light chain has a variable domain (VL) and each heavy chain has a variable domain (VH). Inside these variable domains there are three CDRs (CDR1, CDR2 and CDR3), arranged non-consecutively, on the amino acid sequence of the variable domains. Each tip of the 'Y' of an antibody contains an antigen-binding site that is specific for a particular epitope on an antigen, allowing these two structures to bind together with precision. An "antibody" typically comprises all or a portion of an Fc region, to facilitate detection by an Fe-binding protein, and may also comprise one or more antigen-binding sites, to facilitate detection by an antibody-specific binding agent, such as an antigen or antigenic peptide. The antibodies can be, e.g., of IgG, IgE, IgD, IgM, or IgA type.

The term "CDR" is understood in the current invention as a variable sequence of amino acids that folds into loops capable of binding to an antigenic amino acid sequence, also known as an epitope. The acronym "CDR" stands for complementarity determining region. There are three CDRs (CDR1, CDR2 and CDR3), arranged non-consecutively, on the amino acid sequence of a/the variable domain/s of an antibody. Thus, CDRs are part of the variable domains in antibodies (immunoglobulins) where these molecules bind to their specific antigen, forming part of the heavy chains and light chains. The CDRs are sometimes referred to as hypervariable regions and CDR3 is considered the most variable.

The term "heavy chain variable region" or "heavy chain variable region (VH)" is thus understood in the current invention as the variable domain (VH) of a respective heavy chain as described above for the antibody as defined above. This "heavy chain variable region" thus comprises three CDRs (CDR1, CDR2 and CDR3), arranged non-consecutively, on the amino acid sequence of this variable domain of the heavy chain of the antibody. The term e.g. "heavy chain variable region ONE (VH-1)", "heavy chain variable region TWO (VH-2)" or "heavy chain variable region THREE (VH-3)", respectively, are only giving a designation to identify the respective "heavy chain variable region (VH)" of different antibodies, here especially of antibodies of the "1^{st}, 2^{nd} or 3^{rd} Group of Antibodies" or "first, second or third aspect" (see above), respectively.

The term "light chain variable region" or "light chain variable region (VL)" is thus understood in the current invention as the variable domain (VL) of a respective light chain as described above for the antibody as defined above. This "light chain variable region" thus comprises three CDRs (CDR1, CDR2 and CDR3), arranged non-consecutively, on the amino acid sequence of this variable domain of the light chain of the antibody. The term "light chain variable region ONE (VL-1)", "light chain variable region TWO (VL-2)" or "light chain variable region THREE (VL-3)", respectively, are only giving a designation to identify the respective "light chain variable region (VL)" of different antibodies, here especially of antibodies of the "1^{st}, 2^{nd} or 3^{rd} Group of Antibodies" or "first, second or third aspect" (see above), respectively.

In the context of this invention the term "HMGI-C" (also HMGA2) is to be understood as a protein that belongs to the non-histone chromosomal high-mobility group (HMG) protein family. HMG proteins function as architectural factors and are essential components of the enhanceosome. It may act as a transcriptional regulating factor and its expression in adult tissues is commonly associated with both malignant and benign tumor formation. As said above there are 4 subtypes. As said above, HMGI-C can be seen in AAD (acute aortic disruption). ¹⁵ The initiating event is an intimal tear due to defects of the aortic wall. The dissection of these layers occurs after the aortic rupture. After 5-10 min of the onset of pain, the biological marker HMGI-C is released in the blood. The biomarker level remains elevated for 6-10 days since the pain onset, thereby providing a greater one-time window for the detection of aortic wall damage. The high specificity of the biomarker measurements to the aortic wall damage was verified during surgical interventions, which consequently become a biomarker indicative of aortic dissection. In addition, HMGI-C or HMGA2 (high mobility group AT-hook 2) plays a role in cancer.¹⁶ It promotes cancer metastasis by regulating epithelial-mesenchymal transition.¹⁷ Epithelial-mesenchymal transition (EMT) is a complex physiological process that transforms polarized epithelial cells into moving mesenchymal cells and the dysfunction of EMT promotes the invasion and metastasis of cancer. HMGI-C/HMGA2 is highly overexpressed in highly overexpressed in various types of cancer and thus can act as a target for early detection of cancer as well as for cancer treatment.

As used herein, the term "antigen derivative" of an antibody refers to one or more portions of an antibody that contain the antibody's Complementarity Determining Regions ("'CORS") and optionally other parts, and exhibit an ability to specifically bind antigen. Such fragments include: Fab\F(ab1)2, Fv, single chain (ScFv), and mutants thereof, naturally occurring variants, and fusion proteins including the antibody's "variable region" antigen. recognition site and a heterologous protein (e.g., a toxin, an antigen recognition site for a different antigen, an enzyme, a receptor or receptor ligand, etc,).

As used herein, the term "antigen binding fragment" of an antibody refers to one or more portions of an antibody that contain the antibody's Complementarity Determining Regions ("'CORS") and optionally other parts, and exhibit an ability to specifically bind antigen. Such fragments include: Fab\F(ab1)2, Fv, single chain (ScFv), and mutants thereof, naturally occurring variants, and fusion proteins including the antibody's "variable region" antigen. recognition site and a heterologous protein (e.g., a toxin, an antigen recognition site for a different antigen, an enzyme, a receptor or receptor ligand, etc,).

In the context of this invention the term "chimeric antibody" is to be understood as an antibody produced through the joining two or more genes that originally coded for separate proteins.

In the context of this invention the term "epitope" is to be understood as the part of an antigen that is recognized by an antibody.

In the context of this invention the term "cancer related to HMGI-C " is to be understood to be selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.

In the context of this invention the term that "the treatment involves a HMGI-C-positive cancer cell" means that in this treatment involves a cell, which is a human cancer cell to which the cancer cell-associated HMGI-C is associated or the HMGI-C-positive cancer cell. Usually, this is a solid tumor cancer cell, in particular a soft tissue cancer cell, especially an ovarian cancer, like epithelial ovarian cancer cell; nasopharyngeal cancer cell, renal cancer cell, epithelial cancer cell, prostate cancer cell, endometrial cancer cell, pancreatic cancer cell, laryngeal squamous cancer cell, bladder cancer cell, oral squamous cancer cell, cervical cancer cell, head and neck squamous cancer cell, hepatocellular cancer cell, tongue squamous cancer cell, especially oral tongue squamous cancer cell carcinoma; and osteosarcoma cell.

The terms "protein", "protein fragment", "polypeptide", and "peptide" may be used interchangeably herein to refer to a polymer of amino acid residues and to variants and synthetic and naturally occurring analogues of the same. Thus, these terms apply to amino acid polymers in which one or more amino acid residues are synthetic non-naturally occurring amino acids, such as a chemical analogue of a corresponding naturally occurring amino acid, as well as to naturally- occurring amino acid polymers and naturally occurring chemical derivatives thereof.

The term "antigen" means a molecule having distinct surface features or epitopes capable of stimulating a specific immune response. Antibodies (immunoglobulins) are produced by the immune system in response to exposure to antigens. Antigens maybe proteins, carbohydrates or lipids, although only protein antigens are classified as immunogens because carbohydrates and lipids cannot elicit an immune response on their own.

An "antigen-binding site," or "binding portion" of an antibody, refers to the part of the immunoglobulin molecule that participates in antigen binding. The antigen-binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". In an antibody molecule, the three hypervariable regions of a light chain and the three hypervariable regions of a heavy chain are disposed relative to each other in three-dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs."

The term "Fc-binding protein" refers to a protein or polypeptide capable of binding to the fragment crystallizable region (Fc region) of an antibody. The Fc region is the tail region of an antibody that interacts with cell surface Fc receptors and certain proteins of the complement system. In IgG, IgA and IgD antibody isotypes, the Fc region is composed of two identical protein fragments, derived from the second and third constant domains of the antibody's two heavy chains. IgM and IgE Fc regions contain three heavy chain constant domains (CH domains 2-4) in each polypeptide chain. The Fc regions of IgG antibodies bears a highly conserved N-glycosylation site. The N-glycans attached to this site are predominantly core-fucosylated diantennary structures of the complex type. In addition, small amounts of these N-glycans also bear bisecting GlcNAc and. alpha-2,6 linked sialic acid residues. The Fab region of an antibody contains variable sections that define the target-specificity of the antibody, and in contrast, the Fc region of all antibodies in a class are the same for each species; they are constant rather than variable.

The term "nanoparticles" means uniform particles having a size of 1-200 nm.

The term "nanoshells" means nanoparticles that consist of a core and a metallic shell (usually gold).

In the context of this invention the term "acute aortic dissection (AAD)" is to be understood as a clinical entity being part of the "AAS", initiated by an intimal tear due to congenital defects of the aortic wall, such as in Marfan syndrome (MFS), Ehlers-Danlos syndrome (a heterogeneous group connective tissue disorders), idiopathic Erdheim Gsell medial necrosis, bicuspid aortic valve (the most common congenital heart dissection), or even acquired intima damage caused by hypertension, thoracic trauma or other unknown mechanism.³⁻⁵ One complication of aortic dissection is its outward rupture, which is surgically treated. With the Stanford type A the mortality of untreated patients is about 36%-72% within the first 48 hours and 62%-91% within the first week.¹⁰⁻¹³.

In the context of this invention the term "acute aortic syndrome (AAS)" is to be understood as a broader term including the AAD. The AAS (as well as the AAD) can include the following syndroms: Penetrating Aortic Ulcer and Intramural Hematoma, Thoracic Aortic Aneurysm, Chronic Dissection-Indications with Treatment with Branched and Fenestrated Stent-grafts, Different Therapeutic Modalities for Aortic Arch Dissection Combined with Kommerell's Diverticulum, Secondary Interventions After Endovascular Repair of Aortic Dissections, False Aneurysms Complicating Internal Carotid Artery Dissection, Established Indications for the Invasive Treatment of a Thoracic Aortic Aneurysm, Aortic Arch Dissection: A Controversy of Classification, Treatment Algorithms for Patients with (Sub)acute Type B Aortic Dissections, Descending Thoracic Aortic Dissections, Differences in Aortopathy in Patients with a Bicuspid Aortic Valve with or Without Aortic Coarctation, Angina Chest pain, Dyspnea Accompanied by Hypertension Crisis, Sudden Coma or Shock of Unknown Etiology.

In the context of this invention the term "1-AAD-protein" is HMGI-C. It is released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "1-AAD-protein/s" is/are released in the blood. This biomarker ("1-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection.

In the context of this invention the term "2-AAD-protein" is to be understood as one or more from a group of proteins of which one or more are released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "2-AAD-protein/s" is/are released in the blood. The biomarker ("2-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection. The "2-AAD-proteins" can be e.g. DISAMIN: D-dimer smooth muscle actin myosin isozyme CK-MB and nucleic acid component; but it could also be aggrecan, smooth muscle myosin heavy chain, or soluble aortic elastin.

In the context of this invention the term "X-AAD-protein" is to be understood as one or more from a group of proteins of which one or more are released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "X-AAD-protein/s" is/are released in the blood. The biomarker ("X-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection. The "X-AAD-proteins" can be e.g. DISAMIN: D-dimer smooth muscle actin myosin isozyme CK-MB and nucleic acid component; but it could also be aggrecan, smooth muscle myosin heavy chain, or soluble aortic elastin.

In the context of this invention the term "biomarker" is to be understood as any compound of any biological origin whose detection signals the occurrence of a event in an organism. In the context of this invention "biomarker" is especially understood as one or more proteins, especially enzymes, whose presence in a bio-sample for acute aortic dissection (AAD)more especially the above-described "1-AAD-protein/s", "2-AAD-protein/s" or "X-AAD-protein/s".

In the context of this invention the term "sample pad" is to be understood as a stretch on (or a part of) the strip onto which a fluid sample or analyte, preferably a sample of body fluids, especially of blood, e.g. of blood from a human/ a patient is applied. This sample pad can e.g. comprise or consist of polymers or paper or cotton, like sintered polymer, cotton, porous paper or a microstructured polymer or nitrocellulose. The sample pad should be constructed so that the bodily fluid can be - e.g. by way of an opening in the lateral flow device - applied to it. The sample pad can also act as a sponge and is often able to hold excess fluid.

In the context of this invention the term "bodily fluid" is to be understood as any fluid inside a body, especially that of a mammal, like a human or a patient. A body fluid or bodily fluid can be blood, urine, semen, sputum, saliva etc., most preferably is blood like whole blood, blood serum etc.

In the context of this invention the term "strip" is to be understood as a material or combination of materials enabling capillary flow of fluid along - at least - a portion of the strip. The strip may have - and preferably has - the capacity to spontaneously transport the fluid, like bodily fluid, like blood. The strip can comprise or consist of polymers or paper, like sintered polymer, a microstructured polymer, nitrocellulose, porous paper, especially porous paper like nitrocellulose.

In the context of this invention the term "conjugate" is to be understood as a protein antibody binding to a biomarker (see above) that has been conjugated to a detection agent. In a preferred embodiment said protein antibody is an antibody that binds to a 1-AAD-protein, 2-AAD-protein or X-AAD-protein (see above) and which is conjugated to a detection agent (a Tag) like gold, colored latex etc. Often, the antibody is freeze-dried and/or is a monoclonal antibody. Preferably the antibody in the conjugate is an antibody binding to HMGI-C, more preferably is a monoclonal antibody binding to HMGI-C, most preferably is a freeze-dried monoclonal antibody binding to HMGI-C. Preferably in the conjugate this antibody is conjugated to a gold-particle or a colored latex-particle, more preferably a gold particle. Most preferably the conjugate is a monoclonal antibody binding to HMGI-C conjugated with a gold-particle.

In the context of this invention the term "conjugate pad" is to be understood as a stretch on (or a part of) the strip into which the fluid sample or analyte, previously applied to the sample pad, is transported via capillary flow, e.g. in the strip. It does contain (comprise) a conjugate (see above). This conjugate pad can e.g. consist of polymers or paper, like sintered polymer, porous paper or a microstructured polymer or nitrocellulose. When the sample or analyte flows under capillary action through the strip from the sample pad to the conjugate pad, it mobilizes the conjugate contained (comprised) in the conjugate pad.

In the context of this invention the term "mobilize" is to be understood as being displaced from its current position, e.g. inside the conjugate pad. Preferably, this is applied to the movement of the conjugate (see above) contained inside the conjugate pad further along the strip along with the sample/fluid, e.g. by capillary forces, e.g. towards/to the "detection band". Most preferably, this is applied to the movement of the conjugate (see above) contained inside the conjugate pad which is bound through its protein antibody to the biomarker (1-AAD-protein, 2-AAD-protein, or X-AAD-protein) from the sample/analyte further along the strip along with the sample/fluid.

In the context of this invention "the 1-AAD-protein antibody conjugate" (or the "2-AAD-protein antibody conjugate") is the conjugate (see above) of the "protein antibody binding to" the "biomarker", 1-AAD-protein (or 2-AAD-protein), with a "detection agent". This also covers the conjugate (see above) of the "protein antibody binding to" the "biomarker", 1-AAD-protein (or 2-AAD-protein), with a "detection agent" being bound through its protein antibody to the biomarker 1-AAD-protein (or 2-AAD-protein).

In the context of this invention "the mobilized 1-AAD-protein antibody conjugate" (or the mobilized 2-AAD-protein antibody conjugate) is the conjugate (see above) of the "protein antibody binding to" the "biomarker", 1-AAD-protein (or 2-AAD-protein), with a "detection agent", being mobilized e.g. from the conjugate pad and moved along with the sample. This also covers the conjugate (see above) of the "protein antibody binding to" the "biomarker", 1-AAD-protein (or 2-AAD-protein), with a "detection agent" being bound through its protein antibody to the biomarker 1-AAD-protein (or 2-AAD-protein), being mobilized e.g. from the conjugate pad and moved along with the sample.

In the context of this invention the term "protein antibody" is to be understood as an antibody being a protein that binds to a biomarker (see above). In a preferred embodiment said protein antibody is an antibody that binds to a 1-AAD-protein, 2-AAD-protein or X-AAD-protein (see above). Often, the antibody is freeze-dried and/or is a monoclonal antibody. Preferably the protein antibody is an antibody binding to HMGI-C, more preferably is a monoclonal antibody binding to HMGI-C. It can also be preferred that the protein antibody is an antibody binding to D-Dimer, more preferably is a monoclonal antibody binding to D-Dimer. It can also be preferred that the protein antibody is an antibody binding to the antigen of the amino acid sequence of SEQ ID NO: 1 or a fragment thereof SEQ ID No. 1, more preferably is a monoclonal antibody binding to the antigen of the amino acid sequence of SEQ ID NO: 1 or a fragment thereof.

In the context of this invention the term "protein antibody binding to" is to be understood as a protein antibody wherein it is clarified to which antigen it binds. Usually the "protein antibody binding to" binds to a biomarker (see above) and in a preferred embodiment said protein antibody binds to a 1-AAD-protein, 2-AAD-protein or X-AAD-protein (see above) as its antigen. Preferably it is a protein antibody binding to HMGI-C, more preferably is a monoclonal antibody binding to HMGI-C.

In the context of this invention the term "detection agent" is to be understood as a tag like gold, colored latex, or gold and colored latex-particles or magnetic beads. Preferably the detection agent is understood as part of the conjugate described above, being preferably a gold or latex particle being conjugated to the protein antibody (see above).

In the context of this invention the term "detection band" is to be understood as a stretch on the strip into which the fluid sample or analyte together with the mobilized conjugate (which comprises the "protein antibody"), contained in the conjugate pad, is transported via capillary flow, e.g. in the strip. The detection band is located substantially perpendicular to the direction of flow of the sample along the strip. The detection band does comprise (contain) a (first or second) conjugate-binding protein (see below). The (first or second) conjugate-binding protein is immobilized within the strip (the detection band), preferably along a band (the detection band) located substantially perpendicular to the direction of flow of the sample along the strip. Thus, when the flow of the sample together with the mobilized (1- or 2-)AAD-protein antibody conjugate in the sample reaches the detection band, it contacts (or - at least some AAD-protein antibody conjugate - binds to) the first conjugate-binding protein comprised (contained) within the detection band. This leads to that the presence of said (1- or 2-)AAD-protein in the sample is indicated by a visible color change. This detection band can e.g. consist of polymers or paper, like sintered polymer, porous paper or a microstructured polymer or nitrocellulose.

In the context of this invention the term "immobilized" is to be understood as first of all meaning that it is comprised/contained within the detection band and within the strip. Preferably, it means that it is not mobilized when fluid contacts the immobilized entity, even if continues to flow by capillary flow. Thus, it means that it (e.g. the immobilized (first or second) conjugate-binding protein) is not mobilized by the fluid sample if it (together with the mobilized (1- or 2-) AAD-protein antibody conjugate in the sample) reaches (and contacts) the "immobilized" (first or second) conjugate-binding protein in the detection band.

In the context of this invention the term "contact" or "contacts" is to be understood as one entity getting in contact with another entity. It also includes "binding" or "binds". Thus, "contacts" also includes if a "conjugate-binding-protein" binds to a (mobilized) "AAD-protein antibody conjugate" like a "mobilized 1-AAD-protein antibody conjugate".

In the context of this invention the term "conjugate binding site" is to be understood as covering two different embodiments: a) the "conjugate-binding protein" (as described directly below) or b) a construct in which a surface-structure (site) of the biomarker, the 1-AAD-protein, 2-AAD-Protein or X-AAD-protein is presented to the fluid, e.g. of the sample. In this, the conjugate binding site is immobilized within the strip, usually at a detection band, usually the first detection band, but it could also be immobilized in another (2^{nd}) detection band. In that, in a sample containing the mobilized 1-AAD-protein antibody conjugate (the mobilized 2-AAD-protein antibody conjugate), the mobilized 1-AAD-protein antibody conjugate (the mobilized 2-AAD-protein antibody conjugate) could bind to the conjugate binding site if the "protein antibody" is not already bound to the biomarker/AAD-protein.

In the context of this invention the term "conjugate-binding protein" is to be understood as a protein that is binding to a conjugate comprising an antibody. Preferably, the conjugate-binding protein is binding to the "protein antibody" (described above). The "conjugate-binding protein" binds a) either directly to the "protein antibody" (described above) being part of the "conjugate" (descirbed above) or b) indirectly to the "protein antibody" (described above) by binding to the biomarker as an antigen (1-AAD-protein, 2-AAD-protein, or X-AAD-protein) which is bound via the "protein antibody" of the "conjugate" (described above). Most importantly, though, the "conjugate-binding protein" is - highly preferably - only binding to the protein antibody of the conjugate - whether directly or indirectly - if the protein antibody of the conjugate is - preferably already - bound to the biomarker/AAD-protein. This would be the case in a preferred embodiment of the invention in which the LFA is executed as a Sandwich assay. It is to be understood that the term "contact" also includes "binding" or "binds" in the context of "conjugate-binding protein". The "conjugate-binding protein" is immobilized within the strip, usually at a detection band, usually the first detection band, but it could also be immobilized in another (2^{nd}) detection band. In a preferred embodiment, the "conjugate-binding protein" is directly binding to a biomarker as antigen, which is also bound to the protein antibody of the conjugate; the conjugate being mobilized from the conjugate pad by the fluid from the sample with the protein antibody of the conjugate bound to the biomarker. In a very preferred embodiment, the "conjugate-binding protein" is directly binding to HMGI-C, which is also bound to the protein antibody of the conjugate; the conjugate being mobilized from the conjugate pad by the fluid from the sample with the protein antibody of the conjugate bound to HMGI-C. As an alternative the "conjugate-binding protein" is an FC-binding protein, preferably wherein the FC-binding protein is Protein G, Protein A or a Protein A/G fusion protein.

If the "conjugate-binding protein" binds to the same biomarker as the "protein antibody" (in the "conjugate"), the "conjugate-binding protein" can bind to the same epitope on the same biomarker as the "protein antibody" (in the "conjugate") or the "conjugate-binding protein" can bind to a different epitope on the same biomarker than the "protein antibody" (in the "conjugate").

In the context of this invention the term "further conjugate-binding protein" is to be understood as a protein that is binding to a mobilized MI-protein antibody sample conjugate comprising an antibody to an MI-protein (see below). The further conjugate-binding protein" is different from the first and second immobilized conjugate-binding protein. The "further conjugate-binding protein" binds a) either directly to the "MI-protein antibody" (described below) being part of a conjugate or b) indirectly to the "MI-protein antibody" (described below) by binding to the MI-protein, 2-AAD-protein, or X-AAD-protein) which is bound via the "MI-protein antibody" being conjugated. The "further conjugate-binding protein" is only binding to the MI-protein antibody being conjugated - whether directly or indirectly - if the MI-protein antibody being conjugated is bound to the MI-protein.

In the context of this invention the term "the presence or absence of color" is to be understood as either a) a "visible color change" of the detection band or b) no change of color in the detection band when the mobilized (1-; or 2-)AAD-protein antibody conjugate in the sample contacts the (first or second) conjugate-binding protein in the (first or second) detection band.

In the context of this invention the term "visible color change" is to be understood as a change of color that is - usually - detectable by the naked eye in the detection band when the mobilized (1-; or 2-)AAD-protein antibody conjugate in the sample contacts the (first or second) conjugate-binding protein in the (first or second) detection band. An example is the color change from white to a red color if the "conjugate" is comprising gold particles or from white to a blue color if the "conjugate" is comprising blue Latex particles.

In the context of this invention the term "DISAMIN" is an acronym covering D-dimer smooth muscle actin myosin isozyme CK-MB and nucleic acid component. Accordingly, the group of biomarkers for AAD, denominated by "DISAMIN" includes D-dimer smooth muscle actin myosin isozyme CK-MB and nucleic acid component, and thus especially "D-dimer", "smooth muscle actin" "myosin heavy chain" and also "isozyme CK-MB".

In the context of this invention the term "D-dimer" is to be understood as a specifc degradation product of cross-linked fibrin that is normally produced by plasmin hydrolysis. A cardiac "D-dimer" assay is used for detection of pulmonary embolism and deep vein thrombosis.

In the context of this invention the term "smooth muscle actin" is to be understood as an actin protein that is a globular multi-functional protein that form microfilaments and is involved in the contractile apparatus of smooth muscle.

In the context of this invention the term "smooth muscle myosin heavy chain" is to be understood as the heavy chain of myosin from smooth muscle cells,

In the context of this invention the term "cardiac myosin light chain" is to be understood as the light chain of myosin from heart muscle cells.

In the context of this invention the term "isozyme CK-MB" is to be understood as the creatinekinase creatine kinase myocardial band being the bound combination of two variants (isoenzymes CKM and CKB) of the enzyme phosphocreatine kinase.

In the context of this invention the term "creatinine phosphokinase (CK) or its MB isozyme" (also CPK) is to be understood as synonymous to creatine kinase (CK) wherein MB signifies "myocardial band".

In the context of this invention the term "troponin T (TnT)" is to be understood as a part of troponin (or the troponin complex). It is integral to muscle contraction in skeletal muscle and cardiac muscle, but not smooth muscle.

In the context of this invention the term "Aggrecan" is to be understood as a proteoglycan able to form large aggregates in the cartilage tissue. It is am member of the chondroitin sulfate proteoglycan family and is an integral part of the extracellular matrix in cartilagenous tissue.

In the context of this invention the term "soluble aortic elastin" is to be understood as and elastin that soluble and present at the aorta. Elastin is a key component of the extracellular matrix. It is highly elastic and present in connective tissue allowing many tissues in the body to resume their shape after stretching or contracting. Elastin is used in places where mechanical energy is required to be stored.

In the context of this invention the term "elastin degradation product" is to be understood as the degradation product of elastin.

In the context of this invention the term "human heart fatty acids binding protein" (hFABP) also known as mammary-derived growth inhibitor is a small cytoplasmic protein released from cardiac myocytes following an ischemic episode. It is involved in active fatty acid metabolism where it transports fatty acids from the cell membrane to mitochondria for oxidation.

In the context of this invention the term "acute myocardial infarction" is to be understood as a medical complication that occurs when blood flow decreases or stops to the coronary artery of the heart, causing damage to the heart muscle. It is also commonly known as a heart attack.

In the context of this invention the term "MI-protein" is to be understood as a protein indicative for acute myocardial infarction. Examples include creatinine phosphokinase (CK) or its MB isozyme, cardiac myosin light chain, troponin T (TnT) and human heart fatty acids binding protein (MI-protein).

In the context of this invention the term "MI-detection band" is to be understood as a stretch on the strip into which the fluid sample or analyte together with the mobilized MI-protein antibody (sample) conjugate is transported via capillary flow. The MI-detection band is located substantially perpendicular to the direction of flow of the sample along the strip and is usually distanced from the (first r second) detection band. The MI-detection band does comprise (contain) a further conjugate-binding protein (see below). The further conjugate-binding protein is immobilized within the strip (the MI-detection band), preferably along a band (the MI-detection band) located substantially perpendicular to the direction of flow of the sample along the strip. Thus, when the flow of the sample together with the mobilized MI-protein antibody being conjugated in the sample reaches the MI-detection band, it contacts (or - at least some MI-protein antibody conjugate - binds to) the further conjugate-binding protein comprised (contained) within the MI-detection band. This leads to that the presence of said MI-protein in the sample is indicated by a visible color change. This detection band can e.g. consist of polymers or paper, like sintered polymer, porous paper or a microstructured polymer or nitrocellulose.

In the context of this invention the term "wicking pad" is to be understood as a stretch on (or a part of) the strip into which the fluid sample or analyte is transported via capillary flow, e.g. in the strip. It is at the end of the strip (membrane) opposite the sample pad. It holds the fluids (sample or analyte) and reagents that have migrated via capillary action through the membrane (strip) and acts more or less as a waste container. This wicking pad can e.g. consist of polymers or paper, like sintered polymer, porous paper or a microstructured polymer or nitrocellulose.

In the context of this invention the term "Protein G, Protein A or a Protein A/G fusion protein" is to be understood as a group of proteins that bind to (especially human) IgG, polyclonal or monoclonal antibodies as well as to IgA, IgE, IgM and IgD. Protein A/G is a recombinant fusion protein that combines IgG binding domains of both Protein A and Protein G.

### EMBODIMENTS:

### PART A:

### (Drawn to 2 Groups of antibodies - exemplified by 2 preferred antibodies, 4-H2-2-G6-E12 and 43-3-C5-A6)

**E-A01** An antibody or antibody derivative, wherein the antibody or antibody derivative comprises
- in the heavy chain variable region ONE (VH-1) one (the) amino acid CDR sequence CDR3 of SEQ ID NO. 4;
   OR
- in the heavy chain variable region TWO (VH-2) one (the) amino acid CDR sequence CDR3 of SEQ ID NO. 14.

### (As an alternative, the claim of Embodiment E-A01 could also read:

**E-A01** An antibody, wherein the antibody comprises
- in the heavy chain variable region ONE (VH-1) one (the) amino acid CDR sequence CDR3 of SEQ ID NO. 4;
   OR
- in the heavy chain variable region TWO (VH-2) one (the) amino acid CDR sequence CDR3 of SEQ ID NO. 14.

**E-A02** The antibody (or antibody derivative) according to Embodiment E-A01, wherein the antibody (or antibody derivative) binds to HMGI-C, preferably binds to human HMGI-C.

**E-A03** The antibody (or antibody derivative) according to Embodiments E-A01 or E-A02, wherein the antibody (or antibody derivative)
- which comprises in the heavy chain variable region ONE (VH-1) amino acid CDR sequence CDR3 of SEQ ID NO. 4 binds to Epitope 1 of SEQ ID NO. 32, especially specifically binds to Epitope 1 of SEQ ID NO. 32 of human HMGI-C;
- which comprises in the heavy chain variable region TWO (VH-2) amino acid CDR sequence CDR3 of SEQ ID NO. 14 binds to Epitope 2 of SEQ ID NO. 33, especially specifically binds to Epitope 1 of SEQ ID NO. 33 of human HMGI-C.

**E-A04** The antibody (or antibody derivative) according to any one of Embodiments E-A01 to E-A03, wherein the antibody is a single chain antibody (single-chain variable fragment), a single domain antibody, or a chimeric antibody.

**E-A05** The antibody or antibody derivative according to any one of Embodiments E-A01 to E-A04, wherein said antibody derivative is a Fab fragment.

**E-A06** The antibody or antibody derivative for use according to any one of Embodiments E-A01 to E-A05, wherein said antibody is a murine antibody, such as an IgG, or wherein said antibody is a human or humanized antibody, in particular wherein said humanized antibody is an IgG.

**E-A07** The antibody (or antibody derivative) according to any one of Embodiment E-A01 to E-A06, wherein the antibody is a monoclonal antibody.

**E-A08** The antibody (or antibody derivative) according to any one of Embodiments E-A01 to E-A07, wherein the antibody is a humanized antibody.

**E-A09** The antibody (or antibody derivative) according to any one of Embodiments E-A01 to E-A08, wherein the antibody (or antibody derivative) is effective to detect HMGI-C, preferably is effective to detect HMGI-C in a fluid.

**E-A10** The antibody (or antibody derivative) according to Embodiment E-A09, wherein the fluid, is a body fluid, preferably is a body fluid of human origin.

**E-A11** The antibody (or antibody derivative) according to any one of Embodiments E-A09 or E-A10, wherein the fluid is selected from blood, urine, semen, sputum, saliva; preferably is blood including whole blood, blood serum or blood plasma.

**E-A12** The antibody (or antibody derivative) according to any one of Embodiments E-A01 to E-A11, wherein the antibody (or antibody derivative) binds (to) human HMGI-C and is effective to identify the presence of human HMGI-C in liquids, especially *in vitro.*

**E-A13** The antibody (or antibody derivative) according to any one of Embodiments E-A01 to E-A12 that binds (to) human HMGI-C, wherein the antibody (or antibody derivative) is effective to identify the presence of human HMGI-C in liquids *in vitro,* wherein the antibody comprises
- in the heavy chain variable region ONE (VH-1) one (the) amino acid CDR sequence CDR3 of SEQ ID NO. 4;
   OR
- in the heavy chain variable region TWO (VH-2) one (the) amino acid CDR sequence CDR3 of SEQ ID NO. 14.

**E-A14** The antibody (or antibody derivative) according to any one of Embodiments E-A01 to E-A08, wherein the antibody (or antibody derivative) is effective to treat cancer related to HMGI-C, preferably is effective to treat cancer related to human HMGI-C *in vivo.*

**E-A15** The antibody (or an antibody derivative) according to any one of Embodiments E-A01 to E-A08 and E-A14 that binds (to) human HMGI-C, wherein the antibody or antibody derivative is effective to treat cancer related to human HMGI-C *in vivo,* the antibody comprising
- in the heavy chain variable region ONE (VH-1) one (the) amino acid CDR sequence CDR3 of SEQ ID NO. 4;
   OR
- in the heavy chain variable region TWO (VH-2) one (the) amino acid CDR sequence CDR3 of SEQ ID NO. 14.

**E-A16** The antibody (or antibody derivative) according to any one of Embodiments E-A14 to E-A15, wherein cancer related to HMGI-C is selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.

**E-A17** The antibody (or antibody derivative) according to any one of Embodiments E-A14 to E-A15, wherein the treatment involves a HMGI-C-positive cancer cell.

**E-A18** The antibody (or antibody derivative) according to any one of Embodiments E-A01 to E-A08, wherein the antibody (or antibody derivative) binds to a cancer cell-associated HMGI-C, preferably binds to a human cancer cell-associated HMGI-C.

**E-A19** The antibody (or antibody derivative) according to any one of Embodiments E-A01 to E-A08, wherein the antibody (or antibody derivative) binds to a human cancer cell-associated HMGI-C, the antibody comprising
- in the heavy chain variable region ONE (VH-1) one (the) amino acid CDR sequence CDR3 of SEQ ID NO. 4;
   OR
- in the heavy chain variable region TWO (VH-2) one (the) amino acid CDR sequence CDR3 of SEQ ID NO. 14.

**E-A20** The antibody (or antibody derivative) according to any one of Embodiments E-A17 to E-A19, wherein the human cancer cell to which the cancer cell-associated HMGI-C is associated or the HMGI-C-positive cancer cell is a solid tumor cancer cell, in particular a soft tissue cancer cell, especially an ovarian cancer, like epithelial ovarian cancer cell; nasopharyngeal cancer cell, renal cancer cell, epithelial cancer cell, prostate cancer cell, endometrial cancer cell, pancreatic cancer cell, laryngeal squamous cancer cell, bladder cancer cell, oral squamous cancer cell, cervical cancer cell, head and neck squamous cancer cell, hepatocellular cancer cell, tongue squamous cancer cell, especially oral tongue squamous cancer cell carcinoma; and osteosarcoma cell.

**E-A21** The antibody (or antibody derivative) according to any one of Embodiments E-A01 to E-A20, wherein the antibody (or antibody derivative) comprises
- in the heavy chain variable region ONE (VH-1) together with the amino acid sequence CDR3 of SEQ ID NO. 4 at least one amino acid CDR sequence selected from amino acid sequence CDR1 of SEQ ID NO. 2 or amino acid sequence CDR2 of SEQ ID NO. 3;
   OR
- in the heavy chain variable region TWO (VH-2) together with the amino acid sequence CDR3 of SEQ ID NO. 14 at least one amino acid CDR sequence selected from amino acid sequence CDR1 of SEQ ID NO. 12 or amino acid sequence CDR2 of SEQ ID NO. 13.

**E-A22** The antibody (or antibody derivative) according to any one of Embodiments E-A01 to E-A20, wherein the antibody (or antibody derivative) comprises
- in the heavy chain variable region ONE (VH-1) together with the amino acid sequence CDR3 of SEQ ID NO. 4 the amino acid sequence CDR1 of SEQ ID NO. 2 and the amino acid sequence CDR2 of SEQ ID NO. 3;
   OR
- in the heavy chain variable region TWO (VH-2) together with the amino acid sequence CDR3 of SEQ ID NO. 14 the amino acid sequence CDR1 of SEQ ID NO. 12 and the amino acid sequence CDR2 of SEQ ID NO. 13.

**E-A23** The antibody (or antibody derivative) according to any one of Embodiments E-A01 to E-A22, wherein the antibody (or antibody derivative) comprises
- for the antibody or antibody derivative, which comprises in the heavy chain variable region ONE (VH-1) the amino acid sequence CDR3 of SEQ ID NO. 4,
   ∘ in the light chain variable region ONE (VL-1) at least one amino acid CDR sequence selected from amino acid sequence CDR1 of SEQ ID NO. 7, amino acid sequence CDR2 of SEQ ID NO. 8 and amino acid sequence CDR3 of SEQ ID NO. 9;
   OR
- for the antibody (or antibody derivative), which comprises in the heavy chain variable region TWO (VH-2) the amino acid CDR3 sequence of SEQ ID NO. 14,
   ∘ in the light chain variable region TWO (VL-2) at least one amino acid CDR sequence selected from amino acid sequence CDR1 of SEQ ID NO. 17, amino acid sequence CDR2 of SEQ ID NO. 18 and amino acid sequence CDR3 of SEQ ID NO. 19.

**E-A24** The antibody (or antibody derivative) according to any one of Embodiments E-A01 to E-A22, wherein the antibody (or antibody derivative) comprises
- for the antibody or antibody derivative, which comprises in the heavy chain variable region ONE (VH-1) the amino acid CDR3 sequence of SEQ ID NO. 4,
   ∘ in the light chain variable region ONE (VL-1) at least two amino acid CDR sequences selected from amino acid sequence CDR1 of SEQ ID NO. 7, amino acid sequence CDR2 of SEQ ID NO. 8 and amino acid sequence CDR3 of SEQ ID NO. 9;
   OR
- for the antibody or antibody derivative), which comprises in the heavy chain variable region TWO (VH-2) the amino acid CDR3 sequence of SEQ ID NO. 14,
   ∘ in the light chain variable region TWO (VL-2) at least two amino acid CDR sequences selected from amino acid sequence CDR1 of SEQ ID NO. 17, amino acid sequence CDR2 of SEQ ID NO. 18 and amino acid sequence CDR3 of SEQ ID NO. 19.

**E-A25** The antibody (or antibody derivative) according to any one of Embodiments E-A01 to E-A22, wherein the antibody (or antibody derivative) comprises
- for the antibody (or antibody derivative), which comprises in the heavy chain variable region ONE (VH-1) the amino acid CDR3 sequence of SEQ ID NO. 4,
   ∘ in the light chain variable region TWO (VL-2) amino acid sequence CDR1 of SEQ ID NO. 7, amino acid sequence CDR2 of SEQ ID NO. 8, and amino acid sequence CDR3 of SEQ ID NO. 9;
   OR
- for the antibody or antibody derivative, which comprises in the heavy chain variable region TWO (VH-2) the amino acid sequence CDR3 of SEQ ID NO. 14,
   ∘ in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-A26** The antibody (or antibody derivative) according to any one of Embodiments E-A01 to E-A25, wherein the antibody (or antibody derivative) comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-A27** The antibody (or antibody derivative) according to any one of Embodiments E-A01 to E-A25, wherein the antibody (or antibody derivative) comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid CDR3 sequence of SEQ ID NO. 9.

**E-A28** The antibody (or antibody derivative) according to any one of Embodiments E-A01 to E-A25, wherein the antibody (or antibody derivative) comprises
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-A29** The antibody (or antibody derivative) according to any one of Embodiments E-A01 to E-A22, wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ in the light chain variable region ONE (VL-1) the amino acid sequence of SEQ ID NO.6 or an amino acid sequence of at least 80% identity thereof;
   OR
- for the antibody (or antibody derivative), which comprises in the heavy chain variable region TWO (VH-2) the amino acid sequence CDR3 of SEQ ID NO: 14,
   ∘ in the light chain variable region TWO (VL-2) the amino acid sequence of SEQ ID NO.16 or an amino acid sequence of at least 80% identity thereof.

**E-A30** The antibody (or antibody derivative) according to Embodiment E-A29, wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ in the light chain variable region ONE (VL-1) the amino acid sequence of SEQ ID NO.6 or an amino acid sequence of at least 85% identity thereof, preferably of at least 90% identity thereof, more preferably of at least 95% identity thereof, most preferably of at least 98% identity thereof;
   OR
- for the antibody (or antibody derivative), which comprises in the heavy chain variable region TWO (VH-2) the amino acid sequence CDR3 of SEQ ID NO: 14,
   ∘ in the light chain variable region TWO (VL-2) the amino acid sequence of SEQ ID NO.16 or an amino acid sequence of at least 85% identity thereof, preferably of at least 90% identity thereof, more preferably of at least 95% identity thereof, most preferably of at least 98% identity thereof.

**E-A31** The antibody (or antibody derivative) according to any one of Embodiments E-A01 to E-A28, wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a light chain variable region ONE (VL-1) the amino acid sequence of SEQ ID NO.6;
   OR
- for the antibody (or antibody derivative), which comprises in the heavy chain variable region TWO (VH-2) the amino acid sequence CDR3 of SEQ ID NO: 14,
   ∘ in the light chain variable region TWO (VL-2) the amino acid sequence of SEQ ID NO.16.

**E-A32** The antibody (or antibody derivative) according to any one of Embodiments E-A01 to E-A31, wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a heavy chain variable region ONE (VH-1) having amino acid sequence of SEQ ID NO. 1, and
   ∘ in the light chain variable region ONE (VL-1) having amino acid sequence of SEQ ID NO.6;
   OR
- for the antibody (or antibody derivative), which comprises in the heavy chain variable region TWO (VH-2) the amino acid sequence CDR3 of SEQ ID NO: 14,
   ∘ a heavy chain variable region TWO (VH-2) having amino acid sequence of SEQ ID NO. 11, and
   ∘ a light chain variable region TWO (VL-2) having amino acid sequence of SEQ ID NO. 16.

**E-A33** The antibody (or antibody derivative) according to any one of Embodiments E-A01 to E-A31, wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a heavy chain variable region ONE (VH-1) encoded by nucleic acid sequence of SEQ ID NO. 5, and
   ∘ a light chain variable region ONE (VL-1) encoded by nucleic acid sequence of SEQ ID NO. 10;
   OR
- for the antibody (or antibody derivative), which comprises in the heavy chain variable region TWO (VH-2) the amino acid sequence CDR3 of SEQ ID NO: 14,
   ∘ a heavy chain variable region TWO (VH-2) encoded by nucleic acid sequence of SEQ ID NO. 15, and
   ∘ a light chain variable region TWO (VL-2) encoded by nucleic acid sequence of SEQ ID NO. 20.

**E-A34** The antibody (or antibody derivative) according to any one of Embodiments E-A01 to E-A31, wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a heavy chain variable region ONE (VH-1) having amino acid sequence of SEQ ID NO. 1, and a light chain variable region ONE (VL-1) having amino acid sequence of SEQ ID NO.6; or
   ∘ a heavy chain variable region ONE (VH-1) encoded by nucleic acid sequence of SEQ ID NO. 5, and a light chain variable region ONE (VL-1) encoded by nucleic acid sequence of SEQ ID NO. 10;
   OR
- for the antibody (or antibody derivative), which comprises in the heavy chain variable region TWO (VH-2) the amino acid sequence CDR3 of SEQ ID NO: 14,
   ∘ a heavy chain variable region TWO (VH-2) having amino acid sequence of SEQ ID NO. 11, and a light chain variable region TWO (VL-2) having amino acid sequence of SEQ ID NO. 16, or
   ∘ a heavy chain variable region TWO (VH-2) encoded by nucleic acid sequence of SEQ ID NO. 15, and a light chain variable region TWO (VL-2) encoded by nucleic acid sequence of SEQ ID NO. 20.

**E-A35** An isolated nucleic acid that encodes the antibody (or antibody derivative) according to any one of Embodiments E-A01 to E-A34.

**E-A36** An expression vector encoding the antibody (or antibody derivative) according to any one of Embodiments E-A01 to E-A34.

**E-A37** A host cell comprising a nucleic acid according to Embodiment E-A35 or an expression vector according to Embodiment E-A36.

**E-A38** The host cell of Embodiment E-A37 that produces the antibody (or antibody derivative) according to any one of Embodiments E-A01 to E-A34.

**E-A39** A method of producing the antibody (or antibody derivative) according to any one of Embodiments E-A01 to E-A34, comprising culturing the host cell that produces the antibody according to Embodiment E-A37 and recovering the antibody from the cell culture.

**E-A40** A composition comprising the antibody (or antibody derivative) according to any one of Embodiments E-A01 to E-A34 and a carrier.

**E-A41** The composition of Embodiment E-A40 wherein the carrier is a pharmaceutically acceptable carrier.

**E-A42** An article of manufacture or medical device comprising a container and a composition contained therein, wherein the composition comprises an antibody or antibody derivative according to any of the Embodiments **E-A01** to **E-A34,** preferably wherein the medical device is a lateral flow device for detection of an antigen, most preferably wherein the medical device is a lateral flow device for detection of the presence of human HMGI-C in a body fluid, like blood, whole blood, blood plasma or serum.

**E-A43** A method of treating a HMGI-C positive cancer, comprising administering to a patient suffering from the cancer, a therapeutically effective amount of the antibody (or antibody derivative) according to any one of Embodiments E-A01 to E-A34.

**E-A44** An antibody (or antibody derivative) according to any one of Embodiments E-A01 to E-A34 for use in a method of treating HMGI-C positive cancer, wherein the method comprises the step of contacting a HMGI-C-positive cancer in a subject with said antibody or antibody derivative.

**E-A45** A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
      wherein the 1-AAD-protein is human HMGI-C and the protein antibody binding to said 1-AAD-protein is an antibody of any one of the Embodiments E-A01 to E-A34.

**E-A46** An antibody (or antibody derivative) according to any one of Embodiments E-A01 to E-A34, wherein the antibody (or antibody derivative) binds to human HMGI-C
- at Epitope 1 of SEQ ID NO. 32
   OR
- at Epitope 2 of SEQ ID NO. 33.

### PART B:

### (Drawn to the 1^{st} Group of Antibodies - exemplified by preferred antibody 4-H2-2-G6-E12 ("4-H2"))

These antibodies -binding to EPITOPE 1 (SEQ ID NO: 32) - were quite difficult to obtain and have - at least partly due to the selection of this epitope and partly due to their specific nature - the benefit of being able to detect all 4 subtypes of HMGI-C, especially in body fluids like blood serum or blood.

**E-B01** An (isolated) antibody or antibody derivative/antigen binding fragment thereof, wherein the antibody or antibody derivative/antigen binding fragment thereof comprises
- in the heavy chain variable region ONE (VH-1) one (the) amino acid CDR sequence CDR3 of SEQ ID NO. 4.

### (As an alternative, the claim of Embodiment E-B01 could also read:

**E-B01** An (isolated) antibody, wherein the antibody comprises
- in the heavy chain variable region ONE (VH-1) one (the) amino acid CDR sequence CDR3 of SEQ ID NO. 4.

**E-B02** The (isolated) antibody (or antibody derivative/antigen binding fragment thereof) according to Embodiment **E-B01,** wherein the antibody (or antibody derivative) binds to HMGI-C, preferably binds to human HMGI-C.

**E-B03** The (isolated) antibody (or antibody derivative/antigen binding fragment thereof) according to Embodiments **E-B01** or **E-B02,** wherein the antibody (or antibody derivative) binds to Epitope 1 of SEQ ID NO. 32, especially specifically binds to Epitope 1 of SEQ ID NO. 32 of human HMGI-C.

**E-B04** The (isolated) antibody (or antibody derivative/antigen binding fragment thereof) according to any one of Embodiments **E-B01** to **E-B03,** wherein the antibody is a single chain antibody, a single domain antibody, or a chimeric antibody.

**E-B05** The (isolated) antibody or antibody derivative/antigen binding fragment thereof according to any one of Embodiments **E-B01** to **E-B04,** wherein said antibody derivative is a Fab fragment.

**E-B06** The (isolated) antibody (or antibody derivative/antigen binding fragment thereof) for use according to any one of Embodiments **E-B01** to **E-B05,** wherein said antibody is a murine antibody, such as an IgG, or wherein said antibody is a human or humanized antibody, in particular wherein said humanized antibody is an IgG.

**E-B07** The (isolated) antibody (or antibody derivative/antigen binding fragment thereof) according to any one of Embodiment **E-B01** to **E-B06,** wherein the antibody is a monoclonal antibody.

**E-B08** The (isolated) antibody (or antibody derivative/antigen binding fragment thereof) according to any one of Embodiments **E-B01** to **E-B07,** wherein the antibody is a humanized antibody.

**E-B09** The (isolated) antibody (or antibody derivative/antigen binding fragment thereof) according to any one of Embodiments **E-B01** to **E-B08,** wherein the antibody (or antibody derivative/antigen binding fragment thereof) is effective to detect HMGI-C, preferably is effective to detect HMGI-C in a fluid.

**E-B10** The (isolated) antibody (or antibody derivative/antigen binding fragment thereof) according to Embodiment **E-B09,** wherein the fluid, is a body fluid, preferably is a body fluid of human origin.

**E-B11** The (isolated) antibody (or antibody derivative/antigen binding fragment thereof) according to any one of Embodiments **E-B09** or **E-B10,** wherein the fluid is selected from blood, urine, semen, sputum, saliva; preferably is blood including whole blood, blood serum or blood plasma.

**E-B12** The (isolated) antibody (or antibody derivative/antigen binding fragment thereof) according to any one of Embodiments **E-B01** to **E-B11,** wherein the antibody (or antibody derivative/antigen binding fragment thereof) binds (to) human HMGI-C and is effective to identify the presence of human HMGI-C in liquids, especially *in vitro.*

**E-B13** The (isolated) antibody (or antibody derivative/antigen binding fragment thereof) according to any one of Embodiments **E-B01** to **E-B12** that binds (to) human HMGI-C, wherein the antibody (or antibody derivative/antigen binding fragment thereof) is effective to identify the presence of human HMGI-C in liquids *in vitro,* wherein the antibody comprises
- in the heavy chain variable region ONE (VH-1) one (the) amino acid CDR sequence CDR3 of SEQ ID NO. 4.

**E-B14** The (isolated) antibody (or antibody derivative/antigen binding fragment thereof) according to any one of Embodiments **E-B01** to **E-B08,** wherein the antibody (or antibody derivative/antigen binding fragment thereof) is effective to treat cancer related to HMGI-C, preferably is effective to treat cancer related to human HMGI-C *in vivo.*

**E-B15** The (isolated) antibody (or an antibody derivative) according to any one of Embodiments **E-B01** to **E-B08** and **E-B14** that binds (to) human HMGI-C, wherein the antibody or antibody derivative is effective to treat cancer related to human HMGI-C *in vivo,* the antibody comprising
- in the heavy chain variable region ONE (VH-1) one (the) amino acid CDR sequence CDR3 of SEQ ID NO. 4.

**E-B16** The (isolated) antibody (or antibody derivative/antigen binding fragment thereof) according to any one of Embodiments **E-B14** to **E-B15,** wherein cancer related to HMGI-C is selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.

**E-B17** The (isolated) antibody (or antibody derivative/antigen binding fragment thereof) according to any one of Embodiments **E-B14** to **E-B15,** wherein the treatment involves a HMGI-C-positive cancer cell.

**E-B18** The (isolated) antibody (or antibody derivative/antigen binding fragment thereof) according to any one of Embodiments **E-B01** to **E-B08,** wherein the antibody (or antibody derivative/antigen binding fragment thereof) binds to a cancer cell-associated HMGI-C, preferably binds to a human cancer cell-associated HMGI-C.

**E-B19** The (isolated) antibody (or antibody derivative/antigen binding fragment thereof) according to any one of Embodiments **E-B01** to **E-B08,** wherein the antibody (or antibody derivative/antigen binding fragment thereof) binds to a human cancer cell-associated HMGI-C, the antibody comprising
- in the heavy chain variable region ONE (VH-1) one (the) amino acid CDR sequence CDR3 of SEQ ID NO. 4.

**E-B20** The (isolated) antibody (or antibody derivative/antigen binding fragment thereof) according to any one of Embodiments **E-B17** to **E-B19,** wherein the human cancer cell to which the cancer cell-associated HMGI-C is associated or the HMGI-C-positive cancer cell is a solid tumor cancer cell, in particular a soft tissue cancer cell, especially an ovarian cancer, like epithelial ovarian cancer cell; nasopharyngeal cancer cell, renal cancer cell, epithelial cancer cell, prostate cancer cell, endometrial cancer cell, pancreatic cancer cell, laryngeal squamous cancer cell, bladder cancer cell, oral squamous cancer cell, cervical cancer cell, head and neck squamous cancer cell, hepatocellular cancer cell, tongue squamous cancer cell, especially oral tongue squamous cancer cell carcinoma; and osteosarcoma cell.

**E-B21** The (isolated) antibody (or antibody derivative/antigen binding fragment thereof) according to any one of Embodiments **E-B01** to **E-B20,** wherein the antibody (or antibody derivative/antigen binding fragment thereof) comprises
- in the heavy chain variable region ONE (VH-1) together with the amino acid sequence CDR3 of SEQ ID NO. 4 at least one amino acid CDR sequence selected from amino acid sequence CDR1 of SEQ ID NO. 2 or amino acid sequence CDR2 of SEQ ID NO. 3.

**E-B22** The (isolated) antibody (or antibody derivative/antigen binding fragment thereof) according to any one of Embodiments **E-B01** to **E-B20,** wherein the antibody (or antibody derivative/antigen binding fragment thereof) comprises
- in the heavy chain variable region ONE (VH-1) together with the amino acid sequence CDR3 of SEQ ID NO. 4 the amino acid sequence CDR1 of SEQ ID NO. 2 and the amino acid sequence CDR2 of SEQ ID NO. 3.

**E-B23** The (isolated) antibody (or antibody derivative/antigen binding fragment thereof) according to any one of Embodiments **E-B01** to **E-B22,** wherein the antibody (or antibody derivative/antigen binding fragment thereof) comprises
∘ in the light chain variable region ONE (VL-1) at least one amino acid CDR sequence selected from amino acid sequence CDR1 of SEQ ID NO. 7, amino acid sequence CDR2 of SEQ ID NO. 8 and amino acid sequence CDR3 of SEQ ID NO. 9.

**E-B24** The (isolated) antibody (or antibody derivative/antigen binding fragment thereof) according to any one of Embodiments **E-B01** to **E-B22,** wherein the antibody (or antibody derivative/antigen binding fragment thereof) comprises
∘ in the light chain variable region ONE (VL-1) at least two amino acid CDR sequences selected from amino acid sequence CDR1 of SEQ ID NO. 7, amino acid sequence CDR2 of SEQ ID NO. 8 and amino acid sequence CDR3 of SEQ ID NO. 9.

**E-B25** The (isolated) antibody (or antibody derivative/antigen binding fragment thereof) according to any one of Embodiments **E-B01** to **E-B22,** wherein the antibody (or antibody derivative/antigen binding fragment thereof) comprises
∘ in the light chain variable region ONE (VL-1) amino acid sequence CDR1 of SEQ ID NO. 7, amino acid sequence CDR2 of SEQ ID NO. 8, and amino acid sequence CDR3 of SEQ ID NO. 9.

**E-B26** The (isolated) antibody (or antibody derivative/antigen binding fragment thereof) according to any one of Embodiments **E-B01** to **E-B25,** wherein the antibody (or antibody derivative/antigen binding fragment thereof) comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;

**E-B27** The (isolated) antibody (or antibody derivative/antigen binding fragment thereof) according to Embodiment **E-B26,** wherein the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 1, especially a heavy chain with at least 95%, 99% or 100% sequence identity to SEQ ID NO: 1; preferably a heavy chain with at least 99% or 100% sequence identity to SEQ ID NO: 1; most preferably a heavy chain with 100% sequence identity to SEQ ID NO: 1.

**E-B28** The (isolated) antibody (or antibody derivative/antigen binding fragment thereof) according to Embodiments **E-B26** or **E-B27,** wherein the antibody (or antibody derivative/antigen binding fragment thereof) comprises a light chain (or light chain variable region LCVR) with at least 50%, 60%, 70%, 80%, 85%, 90%, 95%, 99% or 100% sequence identity to SEQ ID NO: 6; especially a light chain with at least 95%, 99% or 100% sequence identity to SEQ ID NO: 6; preferably a light chain with at least 99% or 100% sequence identity to SEQ ID NO: 6; most preferably a light chain with 100% sequence identity to SEQ ID NO: 6.

**E-B29** The (isolated) antibody (or antibody derivative/antigen binding fragment thereof) according to any one of Embodiments **E-B26** to **E-B28,** wherein the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 1 and a light chain with at least 50%, 60%, 70%, 80%, 85%, 90%, 95%, 99% or 100% sequence identity to SEQ ID NO: 6, especially a heavy chain with at least 95%, 99% or 100% sequence identity to SEQ ID NO: 1 and a light chain with at least 95%, 99% or 100% sequence identity to SEQ ID NO: 6; preferably a heavy chain with at least 99% or 100% sequence identity to SEQ ID NO: 1 and a light chain with at least 99% or 100% sequence identity to SEQ ID NO: 6; most preferably a heavy chain with 100% sequence identity to SEQ ID NO: 1 and a light chain with 100% sequence identity to SEQ ID NO: 6.

**E-B30** The (isolated) antibody (or antibody derivative/antigen binding fragment thereof) according to any one of Embodiments **E-B01** to **E-B26,** wherein the antibody (or antibody derivative) comprises
∘ in the light chain variable region ONE (VL-1) the amino acid sequence of SEQ ID NO.6 or an amino acid sequence of at least 80% identity thereof.

**E-B31** The (isolated) antibody (or antibody derivative/antigen binding fragment thereof) according to Embodiment **E-B30,** wherein the antibody (or antibody derivative/antigen binding fragment thereof) comprises
∘ in the light chain variable region ONE (VL-1) the amino acid sequence of SEQ ID NO.6 or an amino acid sequence of at least 85% identity thereof, preferably of at least 90% identity thereof, more preferably of at least 95% identity thereof, most preferably of at least 98% identity thereof;

**E-B32** The (isolated) antibody (or antibody derivative/antigen binding fragment thereof) according to any one of Embodiments **E-B01** to **E-B26,** wherein the antibody (or antibody derivative/antigen binding fragment thereof) comprises
∘ a light chain variable region ONE (VL-1) the amino acid sequence of SEQ ID NO.6.

**E-B33** The (isolated) antibody (or antibody derivative/antigen binding fragment thereof) according to any one of Embodiments **E-B01** to **E-B26,** wherein the antibody (or antibody derivative/antigen binding fragment thereof) comprises
∘ a heavy chain variable region ONE (VH-1) having amino acid sequence of SEQ ID NO. 1, and
∘ in the light chain variable region ONE (VL-1) having amino acid sequence of SEQ ID NO.6.

**E-B34** The (isolated) antibody (or antibody derivative/antigen binding fragment thereof) according to any one of Embodiments **E-B01** to **E-B26,** wherein the antibody (or antibody derivative/antigen binding fragment thereof) comprises
∘ a heavy chain variable region ONE (VH-1) encoded by nucleic acid sequence of SEQ ID NO. 5, and
∘ a light chain variable region ONE (VL-1) encoded by nucleic acid sequence of SEQ ID NO. 10.

**E-B35** The (isolated) antibody (or antibody derivative/antigen binding fragment thereof) according to any one of Embodiments **E-B01** to **E-B26,** wherein the antibody (or antibody derivative/antigen binding fragment thereof) comprises
∘ a heavy chain variable region ONE (VH-1) having amino acid sequence of SEQ ID NO. 1, and a light chain variable region ONE (VL-1) having amino acid sequence of SEQ ID NO.6; or
∘ a heavy chain variable region ONE (VH-1) encoded by nucleic acid sequence of SEQ ID NO. 5, and a light chain variable region ONE (VL-1) encoded by nucleic acid sequence of SEQ ID NO. 10.

**E-B36** An (isolated) antibody (or antibody derivative/antigen binding fragment thereof) binding to HMGI-C, wherein the antibody (or antibody derivative/antigen binding fragment) comprises the three heavy chain CDRs CDR1, CDR2 and CDR3 contained within a heavy chain (or heavy chain variable region HCVR) with at least 98%, 99% or 100% sequence identity to SEQ ID NO: 1 and three light chain CDRs CDR1, CDR2 and CDR3 contained within a light chain (or light chain variable region LCVR) with at least 98%, 99% or 100% sequence identity to SEQ ID NO: 6.

**E-B37** The (isolated) antibody (or antibody derivative/antigen binding fragment thereof) according to Embodiment **E-B36,** binding to HMGI-C, wherein the antibody (or antibody derivative/antigen binding fragment thereof) comprises the three heavy chain CDRs CDR1, CDR2 and CDR3 contained within the heavy chain Variable region of SEQ ID NO: 1 and three light chain CDRs CDR1, CDR2 and CDR3 contained within a heavy chain with at least 98%, 99% or 100% sequence identity to SEQ ID NO: 6.

**E-B38** The (isolated) antibody (or antibody derivative/antigen binding fragment thereof) according to Embodiments **E-B36,** or **E-B37**,binding to HMGI-C,
- wherein the antibody is a murine antibody, such as an IgG, or
- wherein said antibody is a human or humanized antibody, especially a humanized antibody, in particular wherein said humanized antibody is an IgG and/or
- wherein said antibody is a monoclonal antibody; and/or
- wherein said antibody derivative is a Fab fragment.

**E-B39** An (isolated) antibody (or antibody derivative/antigen binding fragment thereof) according to any one of Embodiments **E-B01** to **E-B38,** wherein the antibody (or antibody derivative/antigen binding fragment thereof) binds to human HMGI-C at Epitope 1 of SEQ ID NO. 32.

**E-B40** An (isolated) nucleic acid that encodes the antibody (or antibody derivative/antigen binding fragment thereof) according to any one of Embodiments **E-B01** to **E-B39..**

**E-B41** An expression vector encoding the antibody (or antibody derivative/antigen binding fragment thereof) according to any one of Embodiments **E-B01** to **E-B39.**

**E-B42** A host cell comprising a nucleic acid according to Embodiment E-B34 or an expression vector according to Embodiment **E-B41.**

**E-B43** The host cell of Embodiment E-B35 that produces the antibody (or antibody derivative/antigen binding fragment thereof) according to any one of Embodiments **E-B01** to **E-B39.**

**E-B44** A method of producing the antibody (or antibody derivative/antigen binding fragment thereof) according to any one of Embodiments **E-B01** to **E-B39,** comprising culturing the host cell that produces the antibody according to Embodiment **E-B43** and recovering the antibody from the cell culture.

**E-B45** A composition comprising the antibody (or antibody derivative/antigen binding fragment thereof) according to any one of Embodiments **E-B01** to **E-B39** and a carrier.

**E-B46** The composition of Embodiment **E-B45** wherein the carrier is a pharmaceutically acceptable carrier.

**E-B47** An article of manufacture or medical device comprising a container and a composition contained therein, wherein the composition comprises an antibody (or antibody derivative/antigen binding fragment thereof) according to any of the Embodiments **E-B01** to **E-B39,** preferably wherein the medical device is a lateral flow device for detection of an antigen, most preferably wherein the medical device is a lateral flow device for detection of the presence of human HMGI-C in a body fluid, like blood, whole blood, blood plasma or serum.

**E-B48** A method of treating a HMGI-C positive cancer, comprising administering to a patient suffering from the cancer, a therapeutically effective amount of the antibody (or antibody derivative/antigen binding fragment thereof) according to any one of Embodiments **E-B01** to **E-B39.**

**E-B49** An antibody (or antibody derivative/antigen binding fragment thereof) according to any one of Embodiments **E-B01** to **E-B39** for use in a method of treating HMGI-C positive cancer, wherein the method comprises the step of contacting a HMGI-C-positive cancer in a subject with said antibody or antibody derivative/antigen binding fragment thereof.

**E-B50** A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
      wherein the 1-AAD-protein is human HMGI-C and the protein antibody binding to said 1-AAD-protein is an antibody of any one of the Embodiments **E-B01** to **E-B39**.

### PART C:

### (Drawn to the 2^{nd} Group of Antibodies - exemplified by preferred antibody 43-3-C5-A6 ("43-3"))

These antibodies -binding to EPITOPE 2 (SEQ ID NO: 33) - were quite difficult to obtain and have - at least partly due to the selection of this epitope and partly due to their specific nature - the benefit of being able to detect all 4 subtypes of HMGI-C, especially in body fluids like blood serum or blood.

**E-C01** An (isolated) antibody or antibody derivative/antigen binding fragment thereof, wherein the antibody or antibody derivative comprises
- in the heavy chain variable region ONE (VH-1) one (the) amino acid CDR sequence CDR3 of SEQ ID NO. 14.

### (As an alternative, the claim of Embodiment E-C01 could also read:

**E-C01** An (isolated) antibody, wherein the antibody comprises
- in the heavy chain variable region ONE (VH-1) one (the) amino acid CDR sequence CDR3 of SEQ ID NO. 14.

**E-C02** The (isolated) antibody (or antibody derivative/antigen binding fragment thereof) according to Embodiment **E-C01,** wherein the antibody (or antibody derivative) binds to HMGI-C, preferably binds to human HMGI-C.

**E-C03** The (isolated) antibody (or antibody derivative/antigen binding fragment thereof) according to Embodiments **E-C01** or **E-C02,** wherein the antibody (or antibody derivative) binds to Epitope 2 of SEQ ID NO. 33, especially specifically binds to Epitope 2 of SEQ ID NO. 33 of human HMGI-C.

**E-C04** The (isolated) antibody (or antibody derivative/antigen binding fragment thereof) according to any one of Embodiments **E-C01** to **E-C03,** wherein the antibody is a single chain antibody, a single domain antibody, or a chimeric antibody.

**E-C05** The (isolated) antibody or antibody derivative/antigen binding fragment thereof according to any one of Embodiments **E-C01** to **E-C04,** wherein said antibody derivative is a Fab fragment.

**E-C06** The (isolated) antibody (or antibody derivative/antigen binding fragment thereof) for use according to any one of Embodiments **E-C01** to **E-C05,** wherein said antibody is a murine antibody, such as an IgG, or wherein said antibody is a human or humanized antibody, in particular wherein said humanized antibody is an IgG.

**E-C07** The (isolated) antibody (or antibody derivative/antigen binding fragment thereof) according to any one of Embodiment **E-C01** to **E-C06**, wherein the antibody is a monoclonal antibody.

**E-C08** The (isolated) antibody (or antibody derivative/antigen binding fragment thereof) according to any one of Embodiments **E-C01** to **E-C07,** wherein the antibody is a humanized antibody.

**E-C09** The (isolated) antibody (or antibody derivative/antigen binding fragment thereof) according to any one of Embodiments **E-C01** to **E-C08,** wherein the antibody (or antibody derivative/antigen binding fragment thereof) is effective to detect HMGI-C, preferably is effective to detect HMGI-C in a fluid.

**E-C10** The (isolated) antibody (or antibody derivative/antigen binding fragment thereof) according to Embodiment **E-C09,** wherein the fluid, is a body fluid, preferably is a body fluid of human origin.

**E-C11** The (isolated) antibody (or antibody derivative/antigen binding fragment thereof) according to any one of Embodiments **E-C09** or **E-C10**, wherein the fluid is selected from blood, urine, semen, sputum, saliva; preferably is blood including whole blood, blood serum or blood plasma.

**E-C12** The (isolated) antibody (or antibody derivative/antigen binding fragment thereof) according to any one of Embodiments **E-C01** to **E-C11,** wherein the antibody (or antibody derivative/antigen binding fragment thereof) binds (to) human HMGI-C and is effective to identify the presence of human HMGI-C in liquids, especially *in vitro.*

**E-C13** The (isolated) antibody (or antibody derivative/antigen binding fragment thereof) according to any one of Embodiments **E-C01** to **E-C12** that binds (to) human HMGI-C, wherein the antibody (or antibody derivative/antigen binding fragment thereof) is effective to identify the presence of human HMGI-C in liquids *in vitro,* wherein the antibody comprises
- in the heavy chain variable region ONE (VH-1) one (the) amino acid CDR sequence CDR3 of SEQ ID NO. 14.

**E-C14** The (isolated) antibody (or antibody derivative/antigen binding fragment thereof) according to any one of Embodiments **E-C01** to **E-C08,** wherein the antibody (or antibody derivative/antigen binding fragment thereof) is effective to treat cancer related to HMGI-C, preferably is effective to treat cancer related to human HMGI-C *in vivo.*

**E-C15** The (isolated) antibody (or an antibody derivative) according to any one of Embodiments **E-BC1** to **E-C08** and **E-C14** that binds (to) human HMGI-C, wherein the antibody or antibody derivative is effective to treat cancer related to human HMGI-C *in vivo,* the antibody comprising
- in the heavy chain variable region ONE (VH-1) one (the) amino acid CDR sequence CDR3 of SEQ ID NO. 14.

**E-C16** The (isolated) antibody (or antibody derivative/antigen binding fragment thereof) according to any one of Embodiments **E-C14** to **E-C15,** wherein cancer related to HMGI-C is selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.

**E-C17** The (isolated) antibody (or antibody derivative/antigen binding fragment thereof) according to any one of Embodiments **E-C14** to **E-C15,** wherein the treatment involves a HMGI-C-positive cancer cell.

**E-C18** The (isolated) antibody (or antibody derivative/antigen binding fragment thereof) according to any one of Embodiments **E-C01** to **E-C08,** wherein the antibody (or antibody derivative/antigen binding fragment thereof) binds to a cancer cell-associated HMGI-C, preferably binds to a human cancer cell-associated HMGI-C.

**E-C19** The (isolated) antibody (or antibody derivative/antigen binding fragment thereof) according to any one of Embodiments **E-C01** to **E-C08,** wherein the antibody (or antibody derivative/antigen binding fragment thereof) binds to a human cancer cell-associated HMGI-C, the antibody comprising
- in the heavy chain variable region ONE (VH-1) one (the) amino acid CDR sequence CDR3 of SEQ ID NO. 14.

**E-C20** The (isolated) antibody (or antibody derivative/antigen binding fragment thereof) according to any one of Embodiments **E-C17** to **E-C19,** wherein the human cancer cell to which the cancer cell-associated HMGI-C is associated or the HMGI-C-positive cancer cell is a solid tumor cancer cell, in particular a soft tissue cancer cell, especially an ovarian cancer, like epithelial ovarian cancer cell; nasopharyngeal cancer cell, renal cancer cell, epithelial cancer cell, prostate cancer cell, endometrial cancer cell, pancreatic cancer cell, laryngeal squamous cancer cell, bladder cancer cell, oral squamous cancer cell, cervical cancer cell, head and neck squamous cancer cell, hepatocellular cancer cell, tongue squamous cancer cell, especially oral tongue squamous cancer cell carcinoma; and osteosarcoma cell.

**E-C21** The (isolated) antibody (or antibody derivative/antigen binding fragment thereof) according to any one of Embodiments **E-C01** to **E-C20,** wherein the antibody (or antibody derivative/antigen binding fragment thereof) comprises
- in the heavy chain variable region ONE (VH-1) together with the amino acid sequence CDR3 of SEQ ID NO. 14 at least one amino acid CDR sequence selected from amino acid sequence CDR1 of SEQ ID NO. 12 or amino acid sequence CDR2 of SEQ ID NO. 13.

**E-C22** The (isolated) antibody (or antibody derivative/antigen binding fragment thereof) according to any one of Embodiments **E-C01** to **E-C20,** wherein the antibody (or antibody derivative/antigen binding fragment thereof) comprises
- in the heavy chain variable region ONE (VH-1) together with the amino acid sequence CDR3 of SEQ ID NO. 14 the amino acid sequence CDR1 of SEQ ID NO. 12 and the amino acid sequence CDR2 of SEQ ID NO. 13.

**E-C23** The (isolated) antibody (or antibody derivative/antigen binding fragment thereof) according to any one of Embodiments **E-C01** to **E-C22,** wherein the antibody (or antibody derivative/antigen binding fragment thereof) comprises
∘ in the light chain variable region ONE (VL-1) at least one amino acid CDR sequence selected from amino acid sequence CDR1 of SEQ ID NO. 17, amino acid sequence CDR2 of SEQ ID NO. 18 and amino acid sequence CDR3 of SEQ ID NO. 19.

**E-C24** The (isolated) antibody (or antibody derivative/antigen binding fragment thereof) according to any one of Embodiments **E-C01** to **E-C22,** wherein the antibody (or antibody derivative/antigen binding fragment thereof) comprises
∘ in the light chain variable region ONE (VL-1) at least two amino acid CDR sequences selected from amino acid sequence CDR1 of SEQ ID NO. 17, amino acid sequence CDR2 of SEQ ID NO. 18 and amino acid sequence CDR3 of SEQ ID NO. 19.

**E-C25** The (isolated) antibody (or antibody derivative/antigen binding fragment thereof) according to any one of Embodiments **E-C01** to **E-C22,** wherein the antibody (or antibody derivative/antigen binding fragment thereof) comprises ∘ in the light chain variable region ONE (VL-1) amino acid sequence CDR1 of SEQ ID NO. 17, amino acid sequence CDR2 of SEQ ID NO. 18, and amino acid sequence CDR3 of SEQ ID NO. 19.

**E-C26** The (isolated) antibody (or antibody derivative/antigen binding fragment thereof) according to any one of Embodiments **E-C01** to **E-C25,** wherein the antibody (or antibody derivative/antigen binding fragment thereof) comprises
- in the heavy chain variable region ONE (VH-1) amino acid sequence CDR1 of SEQ ID NO.12, amino acid sequence CDR2 of SEQ ID NO.13, and amino acid sequence CDR3 of SEQ ID NO. 14 and
- in the light chain variable region ONE (VL-1) amino acid sequence CDR1 of SEQ ID NO. 17, amino acid sequence CDR2 of SEQ ID NO. 18 and amino acid sequence CDR3 of SEQ ID NO. 19.

**E-C27** The (isolated) antibody (or antibody derivative/antigen binding fragment thereof) according to Embodiment **E-C26,** wherein the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 11, especially a heavy chain with at least 95%, 99% or 100% sequence identity to SEQ ID NO: 11; preferably a heavy chain with at least 99% or 100% sequence identity to SEQ ID NO: 11; most preferably a heavy chain with 100% sequence identity to SEQ ID NO: 11.

**E-C28** The (isolated) antibody (or antibody derivative/antigen binding fragment thereof) according to Embodiments **E-C26** or **E-C27,** wherein the antibody (or antibody derivative/antigen binding fragment thereof) comprises a light chain (or light chain variable region LCVR) with at least 50%, 60%, 70%, 80%, 85%, 90%, 95%, 99% or 100% sequence identity to SEQ ID NO: 16; especially a light chain with at least 95%, 99% or 100% sequence identity to SEQ ID NO: 16; preferably a light chain with at least 99% or 100% sequence identity to SEQ ID NO: 16; most preferably a light chain with 100% sequence identity to SEQ ID NO: 16.

**E-C29** The (isolated) antibody (or antibody derivative/antigen binding fragment thereof) according to any one of Embodiments **E-C26** to **E-C28,** wherein the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 11 and a light chain with at least 50%, 60%, 70%, 80%, 85%, 90%, 95%, 99% or 100% sequence identity to SEQ ID NO: 16, especially a heavy chain with at least 95%, 99% or 100% sequence identity to SEQ ID NO: 11 and a light chain with at least 95%, 99% or 100% sequence identity to SEQ ID NO: 16; preferably a heavy chain with at least 99% or 100% sequence identity to SEQ ID NO: 11 and a light chain with at least 99% or 100% sequence identity to SEQ ID NO: 16; most preferably a heavy chain with 100% sequence identity to SEQ ID NO: 11 and a light chain with 100% sequence identity to SEQ ID NO: 16.

**E-C30** The (isolated) antibody (or antibody derivative/antigen binding fragment thereof) according to any one of Embodiments **E-C01** to **E-C26,** wherein the antibody (or antibody derivative) comprises
∘ in the light chain variable region ONE (VL-1) the amino acid sequence of SEQ ID NO. 16 or an amino acid sequence of at least 80% identity thereof.

**E-C31** The (isolated) antibody (or antibody derivative/antigen binding fragment thereof) according to Embodiment **E-C30,** wherein the antibody (or antibody derivative/antigen binding fragment thereof) comprises
∘ in the light chain variable region ONE (VL-1) the amino acid sequence of SEQ ID NO. 16 or an amino acid sequence of at least 85% identity thereof, preferably of at least 90% identity thereof, more preferably of at least 95% identity thereof, most preferably of at least 98% identity thereof;

**E-C32** The (isolated) antibody (or antibody derivative/antigen binding fragment thereof) according to any one of Embodiments **E-C01** to **E-C26,** wherein the antibody (or antibody derivative/antigen binding fragment thereof) comprises
∘ a light chain variable region ONE (VL-1) the amino acid sequence of SEQ ID NO.16.

**E-C33** The (isolated) antibody (or antibody derivative/antigen binding fragment thereof) according to any one of Embodiments **E-C01** to **E-C26,** wherein the antibody (or antibody derivative/antigen binding fragment thereof) comprises
∘ a heavy chain variable region ONE (VH-1) having amino acid sequence of SEQ ID NO. 11, and
∘ in the light chain variable region ONE (VL-1) having amino acid sequence of SEQ ID NO.16.

**E-C34** The (isolated) antibody (or antibody derivative/antigen binding fragment thereof) according to any one of Embodiments **E-C01** to **E-C26,** wherein the antibody (or antibody derivative/antigen binding fragment thereof) comprises
∘ a heavy chain variable region ONE (VH-1) encoded by nucleic acid sequence of SEQ ID NO. 15, and
∘ a light chain variable region ONE (VL-1) encoded by nucleic acid sequence of SEQ ID NO. 20.

**E-C35** The (isolated) antibody (or antibody derivative/antigen binding fragment thereof) according to any one of Embodiments **E-C01** to **E-C26,** wherein the antibody (or antibody derivative/antigen binding fragment thereof) comprises
∘ a heavy chain variable region ONE (VH-1) having amino acid sequence of SEQ ID NO. 11, and a light chain variable region ONE (VL-1) having amino acid sequence of SEQ ID NO.16; or
∘ a heavy chain variable region ONE (VH-1) encoded by nucleic acid sequence of SEQ ID NO. 15, and a light chain variable region ONE (VL-1) encoded by nucleic acid sequence of SEQ ID NO. 20.

**E-C36** An (isolated) antibody (or antibody derivative/antigen binding fragment thereof) binding to HMGI-C, wherein the antibody (or antibody derivative/antigen binding fragment) comprises the three heavy chain CDRs CDR1, CDR2 and CDR3 contained within a heavy chain (or heavy chain variable region HCVR) with at least 98%, 99% or 100% sequence identity to SEQ ID NO: 11 and three light chain CDRs CDR1, CDR2 and CDR3 contained within a light chain (or light chain variable region LCVR) with at least 98%, 99% or 100% sequence identity to SEQ ID NO: 16.

**E-C37** The (isolated) antibody (or antibody derivative/antigen binding fragment thereof) according to Embodiment **E-C36,** binding to HMGI-C, wherein the antibody (or antibody derivative/antigen binding fragment thereof) comprises the three heavy chain CDRs CDR1, CDR2 and CDR3 contained within the heavy chain Variable region of SEQ ID NO: 11 and three light chain CDRs CDR1, CDR2 and CDR3 contained within a heavy chain with at least 98%, 99% or 100% sequence identity to SEQ ID NO: 16.

**E-C38** The (isolated) antibody (or antibody derivative/antigen binding fragment thereof) according to Embodiments **E-C36,** or **E-C37**,binding to HMGI-C,
- wherein the antibody is a murine antibody, such as an IgG, or
- wherein said antibody is a human or humanized antibody, especially a humanized antibody, in particular wherein said humanized antibody is an IgG and/or
- wherein said antibody is a monoclonal antibody; and/or
- wherein said antibody derivative is a Fab fragment.

**E-C39** An (isolated) antibody (or antibody derivative/antigen binding fragment thereof) according to any one of Embodiments **E-C01** to **E-C38,** wherein the antibody (or antibody derivative/antigen binding fragment thereof) binds to human HMGI-C at Epitope 2 of SEQ ID NO. 33.

**E-C40** An (isolated) nucleic acid that encodes the antibody (or antibody derivative/antigen binding fragment thereof) according to any one of Embodiments **E-C01** to **E-C39.**

**E-C41** An expression vector encoding the antibody (or antibody derivative/antigen binding fragment thereof) according to any one of Embodiments **E-C01** to **E-C39.**

**E-C42** A host cell comprising a nucleic acid according to Embodiment E-B34 or an expression vector according to Embodiment **E-C41.**

**E-C43** The host cell of Embodiment E-B35 that produces the antibody (or antibody derivative/antigen binding fragment thereof) according to any one of Embodiments **E-C01** to **E-C39.**

**E-C44** A method of producing the antibody (or antibody derivative/antigen binding fragment thereof) according to any one of Embodiments **E-C01** to **E-C39,** comprising culturing the host cell that produces the antibody according to Embodiment **E-C43** and recovering the antibody from the cell culture.

**E-C45** A composition comprising the antibody (or antibody derivative/antigen binding fragment thereof) according to any one of Embodiments **E-C01** to **E-C39** and a carrier. **E-C46** The composition of Embodiment **E-C45** wherein the carrier is a pharmaceutically acceptable carrier.

**E-C47** An article of manufacture or medical device comprising a container and a composition contained therein, wherein the composition comprises an antibody (or antibody derivative/antigen binding fragment thereof) according to any of the Embodiments **E-C01** to **E-C39,** preferably wherein the medical device is a lateral flow device for detection of an antigen, most preferably wherein the medical device is a lateral flow device for detection of the presence of human HMGI-C in a body fluid, like blood, whole blood, blood plasma or serum.

**E-C48** A method of treating a HMGI-C positive cancer, comprising administering to a patient suffering from the cancer, a therapeutically effective amount of the antibody (or antibody derivative/antigen binding fragment thereof) according to any one of Embodiments **E-C01** to **E-C39.**

**E-C49** An antibody (or antibody derivative/antigen binding fragment thereof) according to any one of Embodiments **E-C01** to **E-C39** for use in a method of treating HMGI-C positive cancer, wherein the method comprises the step of contacting a HMGI-C-positive cancer in a subject with said antibody or antibody derivative/antigen binding fragment thereof.

**E-C50** A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
      wherein the 1-AAD-protein is human HMGI-C and the protein antibody binding to said 1-AAD-protein is an antibody of any one of the Embodiments **E-C01** to **E-C39.**

### PART D:

### (Drawn to preferred claims to antibodies)

**E-D01** An antibody or an antibody derivative that bind to human HMGI-C, wherein the antibody comprises
- in the heavy chain variable region ONE (VH-1) one amino acid CDR sequence CDR3 of SEQ ID NO. 4;
   OR
- in the heavy chain variable region TWO (VH-2) at least one amino acid CDR sequence CDR3 of SEQ ID NO. 14.

**E-D02** The antibody or antibody derivative according to Embodiment **E-D01,** wherein the antibody or antibody derivative is effective to identify the presence of human HMGI-C in liquids *in vitro.*

**E-D03** The antibody or antibody derivative according to Embodiment **E-D01,** wherein the antibody or antibody derivative is effective to treat cancer related to human HMGI-C.

**E-D04** The antibody or antibody derivative according to any one of Embodiments **E-D01** to **E-D03,** wherein the antibody or antibody derivative comprises
- in the heavy chain variable region ONE (VH-1) together with the amino acid sequence CDR3 of SEQ ID NO. 4 at least one amino acid CDR sequence selected from amino acid sequence CDR1 of SEQ ID NO. 2 or amino acid sequence CDR2 of SEQ ID NO. 3;
   OR
- in the heavy chain variable region TWO (VH-2) together with the amino acid sequence CDR3 of SEQ ID NO. 14 at least one amino acid CDR sequence selected from amino acid sequence CDR1 of SEQ ID NO. 12 or amino acid sequence CDR2 of SEQ ID NO. 13.

**E-D05** The antibody or antibody derivative according to any one of Embodiments **E-D01** to **E-D03,** wherein the antibody or antibody derivative comprises
- in the heavy chain variable region ONE (VH-1) together with the amino acid sequence CDR3 of SEQ ID NO. 4 the amino acid sequence CDR1 of SEQ ID NO. 2 and the amino acid sequence CDR2 of SEQ ID NO. 3;
   OR
- in the heavy chain variable region TWO (VH-2) together with the amino acid sequence CDR3 of SEQ ID NO. 14 the amino acid sequence CDR1 of SEQ ID NO. 12 and the amino acid sequence CDR2 of SEQ ID NO. 13.

**E-D06** The antibody or antibody derivative according to any one of Embodiments **E-D01** to **E-D05,** wherein the antibody or antibody derivative comprises
- for the antibody or antibody derivative, which comprises in the heavy chain variable region ONE (VH-1) the amino acid sequence CDR3 of SEQ ID NO. 4,
   ∘ in the light chain variable region ONE (VL-1) at least one amino acid CDR sequence selected from amino acid sequence CDR1 of SEQ ID NO. 7, amino acid sequence CDR2 of SEQ ID NO. 8 and amino acid sequence CDR3 of SEQ ID NO. 9;
   OR
- for the antibody or antibody derivative, which comprises in the heavy chain variable region TWO (VH-2) the amino acid CDR3 sequence of SEQ ID NO. 14,
   ∘ in the light chain variable region TWO (VL-2) at least one amino acid CDR sequence selected from amino acid sequence CDR1 of SEQ ID NO. 17, amino acid sequence CDR2 of SEQ ID NO. 18 and amino acid sequence CDR3 of SEQ ID NO. 19.

**E-D07** The antibody or antibody derivative according to any one of Embodiments **E-D01** to **E-D05,** wherein the antibody or antibody derivative comprises
- for the antibody or antibody derivative, which comprises in the heavy chain variable region ONE (VH-1) the amino acid sequence CDR3 of SEQ ID NO. 4
   ∘ in the light chain variable region ONE (VL-1) at least two amino acid CDR sequences selected from amino acid sequence CDR1 of SEQ ID NO. 7, amino acid sequence CDR2 of SEQ ID NO. 8 and amino acid sequence CDR3 of SEQ ID NO. 9;
   OR
- for the antibody or antibody derivative, which comprises in the heavy chain variable region TWO (VH-2) the amino acid sequence CDR3 of SEQ ID NO. 14
   ∘ in the light chain variable region TWO (VL-2) at least two amino acid CDR sequences selected from amino acid sequence CDR1 of SEQ ID NO. 17, amino acid sequence CDR2 of SEQ ID NO. 18 and amino acid sequence CDR3 sequence of SEQ ID NO. 19.

**E-D08** The antibody or antibody derivative according to any one of Embodiments **E-D01** to **E-D05,** wherein the antibody or antibody derivative comprises
- for the antibody or antibody derivative, which comprises in the heavy chain variable region ONE (VH-1) the amino acid sequence CDR3 of SEQ ID NO. 4
   ∘ in the light chain variable region ONE (VL-1) amino acid sequence CDR1 of SEQ ID NO. 7, amino acid sequence CDR2 of SEQ ID NO. 8, and amino acid sequence CDR3 of SEQ ID NO. 9;
   OR
- in the antibody or antibody derivative, which comprises in the heavy chain variable region TWO (VH-2) the amino acid sequence CDR3 of SEQ ID NO. 14
   ∘ in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-D09** The antibody or antibody derivative according to any one of Embodiments **E-D01** to **E-D08,** wherein the antibody or antibody derivative comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-D10** The antibody or antibody derivative according to any one of Embodiments **E-D01** to **E-D08,** wherein the antibody or antibody derivative comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9.

**E-D11** The antibody or antibody derivative according to any one of Embodiments **E-D01** to **E-D08,** wherein the antibody or antibody derivative comprises
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-D12** The antibody or antibody derivative according to any one of Embodiments **E-D01** to **E-D11,** wherein the antibody or antibody derivative comprises:
a) a heavy chain variable region ONE (VH-1) encoded by the nucleic acid sequence of SEQ ID NO. 5 and a light chain variable region ONE (VL-1) encoded by the nucleic acid sequence of SEQ ID NO. 10; or
b) a heavy chain variable region ONE (VH-1) having amino acid sequence of SEQ ID NO. 1 and a light chain variable region ONE (VL-1) having amino acid sequence of SEQ ID NO. 6;
OR
a) a heavy chain variable region TWO (VH-2) encoded by nucleic acid sequences of SEQ ID NO. 15 and a light chain variable region TWO (VL-2) encoded by nucleic acid sequences of SEQ ID NO. 20; or
b) a heavy chain variable region TWO (VH-2) having amino acid sequence of SEQ ID NO. 11 and a light chain variable region TWO (VL-2) having amino acid sequence of SEQ ID NO. 16.

**E-D13** The antibody or antibody derivative according to any one of Embodiments **E-D01** to **E-D12,**
- wherein the antibody is a murine antibody, such as an IgG, or
- wherein said antibody is a human or humanized antibody, especially a humanized antibody, in particular wherein said humanized antibody is an IgG and/or
- wherein said antibody is a monoclonal antibody; and/or
- wherein said antibody derivative is a Fab fragment.

**E-D14** The antibody (or antibody derivative) according to any one of Embodiments **E-D01** to **E-D13,** wherein the antibody (or antibody derivative)
- which comprises in the heavy chain variable region ONE (VH-1) amino acid CDR sequence CDR3 of SEQ ID NO. 4 binds to Epitope 1 of SEQ ID NO. 32, especially specifically binds to Epitope 1 of SEQ ID NO. 32 of human HMGI-C;
- which comprises in the heavy chain variable region TWO (VH-2) amino acid CDR sequence CDR3 of SEQ ID NO. 14 binds to Epitope 2 of SEQ ID NO. 33, especially specifically binds to Epitope 1 of SEQ ID NO. 33 of human HMGI-C.

**E-D15** An antibody or an antibody derivative that bind to human HMGI-C, wherein the antibody comprises
- in the heavy chain variable region THREE (VH-3) (the three CDRs of) amino acid sequence CDR1 of SEQ ID NO.22, amino acid sequence CDR2 of SEQ ID NO.23, and amino acid sequence CDR3 of SEQ ID NO.24 and
- in the light chain variable region THREE (VL-3) amino acid sequence CDR1 of SEQ ID NO. 27, amino acid sequence CDR2 of SEQ ID NO. 28 and amino acid sequence CDR3 of SEQ ID NO. 29.

**E-D16** The antibody (or antibody derivative) according to Embodiment **E-D15,** wherein the antibody or antibody derivative comprises a heavy chain variable region with at least 95%, 99% or 100% sequence identity to SEQ ID NO: 21 and a light chain variable region with at least 95%, 99% or 100% sequence identity to SEQ ID NO: 26.

**E-D17** An isolated nucleic acid or an expression vector that encodes the antibody or antibody derivative according to any one of the Embodiments **E-D01** to **E-D16.**

**E-D18** A host cell comprising the nucleic acid or the expression vector according to Embodiment **E-D17,** preferably wherein the host cell produces the antibody or antibody derivative according to any one of the preceding claims.

**E-D19** A method of producing the antibody or antibody derivative according to any one of the Embodiments **E-D01** to **E-D16,** comprising culturing the host cell according to Embodiment **E-D18** that produces the antibody and recovering the antibody from the cell culture.

**E-D20** A composition comprising the antibody or antibody derivative according to any one of the Embodiments **E-D01** to **E-D16** and a carrier, preferably a pharmaceutically acceptable carrier.

**E-D21** An article of manufacture or medical device comprising a container and a composition contained therein, wherein the composition comprises an antibody or antibody derivative according to any of the Embodiments **E-D01** to **E-D16,** preferably wherein the medical device is a lateral flow device for detection of an antigen, most preferably wherein the medical device is a lateral flow device for detection of the presence of human HMGI-C in a body fluid, like blood, whole blood, blood plasma or serum.

**E-D22** An antibody according to any one of Embodiments **E-D01** to **E-D16** for use in a method of treating HMGI-C positive cancer, wherein the method comprises the step of contacting a HMGI-C-positive cancer in a subject with said antibody or antibody derivative, preferably wherein said HMGI-C positive cancer is selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.

**E-D23** A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
      wherein the 1-AAD-protein is human HMGI-C and the protein antibody binding to said 1-AAD-protein is an antibody of any of the Embodiments **E-D01** to **E-D16.**

### PART E:

### (Drawn to a lateral Flow Device employing the antibodies of the invention)

**E-E01** A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
   wherein the 1-AAD-protein is HMGI-C and the -protein antibody binding to said 1-AAD-protein is an antibody or antibody or antibody derivative, wherein the antibody or antibody derivative comprises
   - in the heavy chain variable region ONE (VH-1) one (the) amino acid CDR sequence CDR3 of SEQ ID NO. 4;
      OR
   - in the heavy chain variable region TWO (VH-2) one (the) amino acid CDR sequence CDR3 of SEQ ID NO. 14.

**E-E02** The lateral flow device according to Embodiment E-E01, wherein said antibody (or antibody derivative) binds to human HMGI-C.

**E-E03** The lateral flow device according to Embodiments **E-E01** or **E-E02,** wherein said antibody is a murine antibody, such as an IgG, or wherein said antibody is a human or humanized antibody, in particular wherein said humanized antibody is an IgG.

**E-E04** The lateral flow device according to any one of the Embodiments **E-E01** to **E-E03,** wherein said antibody is a monoclonal antibody.

**E-E05** The lateral flow device according to any one of the Embodiments **E-E01** to **E-E04,** wherein said antibody is a humanized antibody.

**E-E06** The lateral flow device according to any one of the Embodiments **E-E01** to **E-E05,** wherein said antibody (or antibody derivative) comprises
- in the heavy chain variable region ONE (VH-1) together with the amino acid sequence CDR3 of SEQ ID NO. 4 at least one amino acid CDR sequence selected from amino acid sequence CDR1 of SEQ ID NO. 2 or amino acid sequence CDR2 of SEQ ID NO. 3;
   OR
- in the heavy chain variable region TWO (VH-2) together with the amino acid sequence CDR3 of SEQ ID NO. 14 at least one amino acid CDR sequence selected from amino acid sequence CDR1 of SEQ ID NO. 12 or amino acid sequence CDR2 of SEQ ID NO. 13.

**E-E07** The lateral flow device according to any one of the Embodiments **E-E01** to **E-E06,** wherein said antibody (or antibody derivative) comprises
- in the heavy chain variable region ONE (VH-1) together with the amino acid sequence CDR3 of SEQ ID NO. 4 the amino acid sequence CDR1 of SEQ ID NO. 2 and the amino acid sequence CDR2 of SEQ ID NO. 3;
   OR
- in the heavy chain variable region TWO (VH-2) together with the amino acid sequence CDR3 of SEQ ID NO. 14 the amino acid sequence CDR1 of SEQ ID NO. 12 and the amino acid sequence CDR2 of SEQ ID NO. 13.

**E-E08** The lateral flow device according to any one of the Embodiments **E-E01** to **E-E07,** wherein said antibody (or antibody derivative) comprises
- for the antibody or antibody derivative, which comprises in the heavy chain variable region ONE (VH-1) the amino acid sequence CDR3 of SEQ ID NO. 4,
   ∘ in the light chain variable region ONE (VL-1) at least one amino acid CDR sequence selected from amino acid sequence CDR1 of SEQ ID NO. 7, amino acid sequence CDR2 of SEQ ID NO. 8 and amino acid sequence CDR3 of SEQ ID NO. 9;
   OR
- for the antibody (or antibody derivative), which comprises in the heavy chain variable region TWO (VH-2) the amino acid CDR3 sequence of SEQ ID NO. 14,
   ∘ in the light chain variable region TWO (VL-2) at least one amino acid CDR sequence selected from amino acid sequence CDR1 of SEQ ID NO. 17, amino acid sequence CDR2 of SEQ ID NO. 18 and amino acid sequence CDR3 of SEQ ID NO. 19.

**E-E09** The lateral flow device according to any one of the Embodiments **E-E01** to **E-E08,** wherein said antibody (or antibody derivative) comprises
- for the antibody or antibody derivative, which comprises in the heavy chain variable region ONE (VH-1) the amino acid CDR3 sequence of SEQ ID NO. 4,
   ∘ in the light chain variable region ONE (VL-1) at least two amino acid CDR sequences selected from amino acid sequence CDR1 of SEQ ID NO. 7, amino acid sequence CDR2 of SEQ ID NO. 8 and amino acid sequence CDR3 of SEQ ID NO. 9;
   OR
- for the antibody or antibody derivative), which comprises in the heavy chain variable region TWO (VH-2) the amino acid CDR3 sequence of SEQ ID NO. 14,
   ∘ in the light chain variable region TWO (VL-2) at least two amino acid CDR sequences selected from amino acid sequence CDR1 of SEQ ID NO. 17, amino acid sequence CDR2 of SEQ ID NO. 18 and amino acid sequence CDR3 of SEQ ID NO. 19.

**E-E10** The lateral flow device according to any one of the Embodiments **E-E01** to **E-E09,** wherein said antibody (or antibody derivative) comprises
- for the antibody (or antibody derivative), which comprises in the heavy chain variable region ONE (VH-1) the amino acid CDR3 sequence of SEQ ID NO. 4,
   ∘ in the light chain variable region TWO (VL-2) amino acid sequence CDR1 of SEQ ID NO. 7, amino acid sequence CDR2 of SEQ ID NO. 8, and amino acid sequence CDR3 of SEQ ID NO. 9;
   OR
- for the antibody or antibody derivative, which comprises in the heavy chain variable region TWO (VH-2) the amino acid sequence CDR3 of SEQ ID NO. 14,
   ∘ in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-E11** The lateral flow device according to any one of the Embodiments **E-E01** to **E-E10,** wherein said antibody (or antibody derivative) comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-E12** The lateral flow device according to any one of the Embodiments **E-E01** to **E-E10,** wherein said antibody (or antibody derivative) comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid CDR3 sequence of SEQ ID NO. 9.

**E-E13** The lateral flow device according to any one of the Embodiments **E-E01** to **E-E10,** wherein said antibody (or antibody derivative) comprises
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

**E-E14** The lateral flow device according to any one of the Embodiments **E-E01** to **E-E07,** wherein said antibody (or antibody derivative) comprises
- for the antibody (or antibody derivative), which comprises in the heavy chain variable region ONE (VH-1) the amino acid sequence CDR3 of SEQ ID NO. 4,
   ∘ in the light chain variable region ONE (VL-1) the amino acid sequence of SEQ ID NO.6 or an amino acid sequence of at least 80% identity thereof;
   OR
- for the antibody (or antibody derivative), which comprises in the heavy chain variable region TWO (VH-2) the amino acid sequence CDR3 of SEQ ID NO: 14,
   ∘ in the light chain variable region TWO (VL-2) the amino acid sequence of SEQ ID NO. 16 or an amino acid sequence of at least 80% identity thereof.

**E-E15** The lateral flow device according to Embodiment **E-E14,** wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ in the light chain variable region ONE (VL-1) the amino acid sequence of SEQ ID NO.6 or an amino acid sequence of at least 85% identity thereof, preferably of at least 90% identity thereof, more preferably of at least 95% identity thereof, most preferably of at least 98% identity thereof;
   OR
- for the antibody (or antibody derivative), which comprises in the heavy chain variable region TWO (VH-2) the amino acid sequence CDR3 of SEQ ID NO: 14,
   ∘ in the light chain variable region TWO (VL-2) the amino acid sequence of SEQ ID NO. 16 or an amino acid sequence of at least 85% identity thereof, preferably of at least 90% identity thereof, more preferably of at least 95% identity thereof, most preferably of at least 98% identity thereof.

**E-E16** The lateral flow device according to any one of the Embodiments **E-E01 to E-E15,** wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a light chain variable region ONE (VL-1) the amino acid sequence of SEQ ID NO.6;
   OR
- for the antibody (or antibody derivative), which comprises in the heavy chain variable region TWO (VH-2) the amino acid sequence CDR3 of SEQ ID NO: 14,
   ∘ in the light chain variable region TWO (VL-2) the amino acid sequence of SEQ ID NO.16.

**E-E17** The lateral flow device according to any one of the Embodiments **E-E01** to **E-E16**, wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a heavy chain variable region ONE (VH-1) having amino acid sequence of SEQ ID NO. 1, and
   ∘ in the light chain variable region ONE (VL-1) having amino acid sequence of SEQ ID NO.6;
   OR
- for the antibody (or antibody derivative), which comprises in the heavy chain variable region TWO (VH-2) the amino acid sequence CDR3 of SEQ ID NO: 14,
   ∘ a heavy chain variable region TWO (VH-2) having amino acid sequence of SEQ ID NO. 11, and
   ∘ a light chain variable region TWO (VL-2) having amino acid sequence of SEQ ID NO. 16.

**E-E18** The lateral flow device according to any one of the Embodiments **E-E01** to **E-E17,** wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a heavy chain variable region ONE (VH-1) encoded by nucleic acid sequence of SEQ ID NO. 5, and
   ∘ a light chain variable region ONE (VL-1) encoded by nucleic acid sequence of SEQ ID NO. 10;
   OR
- for the antibody (or antibody derivative), which comprises in the heavy chain variable region TWO (VH-2) the amino acid sequence CDR3 of SEQ ID NO: 14,
   ∘ a heavy chain variable region TWO (VH-2) encoded by nucleic acid sequence of SEQ ID NO. 15, and
   ∘ a light chain variable region TWO (VL-2) encoded by nucleic acid sequence of SEQ ID NO. 20.

**E-E19** The lateral flow device according to any one of the Embodiments **E-E01 to E-E18,** wherein the antibody (or antibody derivative) comprises
- for the antibody (or antibody derivative), which comprises in the heavy chain variable region ONE (VH-1) the amino acid sequence CDR3 of SEQ ID NO. 4,
   ∘ a heavy chain variable region ONE (VH-1) having amino acid sequence of SEQ ID NO. 1, and a light chain variable region ONE (VL-1) having amino acid sequence of SEQ ID NO.6; or
   ∘ a heavy chain variable region ONE (VH-1) encoded by nucleic acid sequence of SEQ ID NO. 5, and a light chain variable region ONE (VL-1) encoded by nucleic acid sequence of SEQ ID NO. 10;
   OR
- for the antibody (or antibody derivative), which comprises in the heavy chain variable region TWO (VH-2) the amino acid sequence CDR3 of SEQ ID NO: 14,
   ∘ a heavy chain variable region TWO (VH-2) having amino acid sequence of SEQ ID NO. 11, and a light chain variable region TWO (VL-2) having amino acid sequence of SEQ ID NO. 16, or
   ∘ a heavy chain variable region TWO (VH-2) encoded by nucleic acid sequence of SEQ ID NO. 15, and a light chain variable region TWO (VL-2) encoded by nucleic acid sequence of SEQ ID NO. 20.

**E-E20** A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
   wherein the 1-AAD-protein is HMGI-C and the -protein antibody binding to said 1-AAD-protein is an antibody or antibody or antibody derivative, wherein the antibody or antibody derivative comprises
   - in the heavy chain variable region THREE (VH-3) (the three CDRs of) amino acid sequence CDR1 of SEQ ID NO.22, amino acid sequence CDR2 of SEQ ID NO.23, and amino acid sequence CDR3 of SEQ ID NO.24 and
   - in the light chain variable region THREE (VL-3) amino acid sequence CDR1 of SEQ ID NO. 27, amino acid sequence CDR2 of SEQ ID NO. 28 and amino acid sequence CDR3 of SEQ ID NO. 29.

**E-E21** The antibody (or antibody derivative) according to Embodiment **E-E20,** wherein the antibody or antibody derivative comprises a heavy chain variable region with at least 95%, 99% or 100% sequence identity to SEQ ID NO: 21 and a light chain variable region with at least 95%, 99% or 100% sequence identity to SEQ ID NO: 26.

**E-E22** The lateral flow device according to any one of the Embodiments **E-E01 to E-E21,** wherein the lateral flow device further comprises one or more further protein antibody/ies binding to one or more further protein/s potentially also released during said event leading to an acute aortic dissection (X-AAD-protein/s).

**E-E23** The lateral flow device according to Embodiment **E-E22,** wherein the X-AAD-protein/s is/are selected from a group of biomarkers for AAD including D-dimer, smooth muscle actin, myosin, isozyme CK-MB and nucleic acid component (DISAMIN), and especially from aggrecan, smooth muscle myosin heavy chain, soluble aortic elastin or D-dimer preferably is D-Dimer
or
wherein the X-AAD-proteins are D-Dimer and isozyme CK-MB
   or
wherein the X-AAD-protein is D-Dimer.

**E-E24** The lateral flow device according to any one of the Embodiments **E-E22 or E-E23,** wherein said one or more further protein antibody/ies to the X-AAD-protein/s is/are conjugated to the same detection agent as the one conjugated to said antibody to the 1-AAD-protein and said conjugate with the one or more further protein antibody/ies to the X-AAD-protein/s is/are located in said first conjugate pad and the first conjugate-binding protein in the first detection band will when the one or more further mobilized X-AAD-protein/s antibody/ies conjugate/s in the sample contact/s the first conjugate-binding protein in the first detection band, the presence of the one or more X-AAD-protein/s in the sample is/are indicated by a visible color change.

**E-E25** The lateral flow device according to any one of the Embodiments **E-E01 or E-E24,** further comprising at least one MI-protein antibody binding to a protein indicative for acute myocardial infarction (MI-protein) together with a separate MI-detection band comprising a further conjugate-binding protein different from the first and second immobilized conjugate-binding protein immobilized within the strip along a band located substantially perpendicular to the direction of flow of the sample along the strip so that when an at least one mobilized MI-protein antibody sample conjugate in the sample contacts the further conjugate-binding protein in the MI-detection band the presence of said MI-protein in the sample is indicated by a visible color change; preferably wherein the MI-protein is selected from the group consisting of creatinine phosphokinase (CK) or its MB isozyme, cardiac myosin light chain, troponin T (TnT) or troponin I (Thl) and human heart fatty acids binding protein;
most preferably wherein the lateral flow device further comprises a MI-protein antibody binding to the MI-protein being troponin, like T (TnT) or troponin I (Thl);
   or
most preferably wherein the lateral flow device further comprises two MI-protein antibodies with one antibody binding to the MI-protein troponin, like T (TnT) or troponin I (Thl), and the other antibody binding to the MI-protein creatinine phosphokinase (CK) and especially its CK-MB isozyme.

**E-E26** The lateral flow device according to any one of the Embodiments **E-E01 or E-E25,** wherein the acute aortic dissection (AAD) is acute aortic syndrome which comprises aortic dissection, including acute type A aortic dissection, and/or one or more of the following aortic dissections/acute aortic syndroms:
- Penetrating Aortic Ulcer and Intramural Hematoma
- Thoracic Aortic Aneurysm
- Chronic Dissection-Indications with Treatment with Branched and Fenestrated Stent-grafts
- Different Therapeutic Modalities for Aortic Arch Dissection Combined with Kommerell's Diverticulum
- Secondary Interventions After Endovascular Repair of Aortic Dissections
- False Aneurysms Complicating Internal Carotid Artery Dissection
- Established Indications for the Invasive Treatment of a Thoracic Aortic Aneurysm
- Aortic Arch Dissection: A Controversy of Classification
- Treatment Algorithms for Patients with (Sub)acute Type B Aortic Dissections
- Descending Thoracic Aortic Dissections
- Differences in Aortopathy in Patients with a Bicuspid Aortic Valve with or Without Aortic Coarctation
- Angina Chest pain
- Dyspnea Accompanied by Hypertension Crisis
- Sudden Coma or
- Shock of Unknown Etiology.

**E-E27** The lateral flow device according to any one of the Embodiments **E-E01 or E-E26,** wherein the conjugate-binding protein is an FC-binding protein, preferably wherein the FC-binding protein is Protein G, Protein A or a Protein A/G fusion protein.

**E-E28** The lateral flow device according to any one of the Embodiments **E-E01 or E-E27,** wherein the detection agent conjugated to the antibody is selected from the group comprising metallic nanoparticles or nanoshells, non-metallic nanoparticles or nanoshells, enzymes, and fluorescent molecules, preferably wherein the detection agent conjugated to the antibodies comprises nanoparticles or nanoshells of metallic gold.

**E-E29** The lateral flow device according to any one of the Embodiments **E-E01 or E-E28,** further comprising a control line located substantially perpendicular to the direction of flow of the sample along the strip, preferably wherein the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line, preferably wherein deposited at the control line is an antibody capable of binding to the mobilized immunoglobin conjugate as it crosses the control line, with optionally said binding at the control line being indicated by a visible color change; even more preferably wherein the immunoglobulin in the conjugate is from an animal species other than the mammal species from which the sample of bodily fluid is derived.

**E-E30** A method for the detection of HMGI-C in a body fluid of a mammal comprising using a lateral flow device of any one of the Embodiments **E-E01 or E-E29.**

**E-E31** A method for the detection of HMGI-C released during an event leading to an acute aortic dissection in a sample of bodily fluid of a mammal, the method comprising:
a) placing the sample onto a sample pad of a lateral flow assay device comprising:
   a. a strip formed of a material enabling capillary flow of fluid along a portion of the strip;
   b. the sample pad located proximal to one end of the strip for receiving the sample of the bodily fluid;
   c. a first conjugate pad located in the strip so that in operation the sample flows under capillary action through the strip from the sample pad to the conjugate pad and mobilizes a conjugate comprised in the conjugate pad, the conjugate comprises a protein antibody binding to HMGI-C (HMGI-C antibody) that has been conjugated to a detection agent (HMGI-C antibody conjugate), and
   d. a first detection band comprising a first conjugate-binding protein immobilized within the strip along a band located substantially perpendicular to the direction of flow of the sample along the strip so that when the mobilized HMGI-C antibody conjugate in the sample contacts the first conjugate-binding protein in the first detection band the presence of HMGI-C in in the sample is indicated by a visible color change,
   wherein said HMGI-C antibody is an antibody defined in any one of the Embodiments **E-E01 to E-E21,**
b) interrogating the detection region for the presence of label particles to detect whether said AAD protein is present in the sample.

**E-E32** A kit for detecting HNGI-C released during an event leading to an acute aortic dissection in a sample of bodily fluid of a mammal, the kit comprising:
a) a lateral flow assay device of any one of the Embodiments **E-E01** to **E-E27;** and
b) an instruction information on how to use the device of in any one of the Embodiments **E-E01 to E-E27.**

**E-E31** A method for the immunoassay determination of acute aortic dissection (AAD) in a patient, said method comprising:
a) incubating a body fluid sample of the patient with
   a. at least one antibody binding to HMGI-C released during an event leading to an acute aortic dissection and
   b. a binding partner B for either HMGI-C or the antibody
      wherein either the antibody or the binding partner B is conjugated to a detection agent,
      to form an immunological complex containing the detection agent;
b) isolating the immunological complex from the remaining sample; and
c) determining the detection agent either in the isolated complex or in the remaining sample as an indicator of the presence of HMGI-C in the sample;

thereby determining the occurrence of AAD in the patient,
wherein said antibody binding to HMGI-C is an antibody defined in any one of the Embodiments **E-E01 to E-E21.**

### PART F:

### (Drawn to the 3^{rd} Group of Antibodies - exemplified by preferred antibody 141-3-F2-H4 ("141-3"))

These antibodies - binding to EPITOPE 3 (SEQ ID NO: 34) - were also quite difficult to obtain and have - at least partly due to the selection of this epitope and partly due to their specific nature - also the benefit of being able to detect all 4 subtypes of HMGI-C, especially in body fluids like blood serum or blood, but are also binding to EPITOPE 1 (SEQ ID NO: 32) due to a 6 amino acid overlap (SEQ ID NO:35). These antibodies have thus a high detection value for HMGI-C in body fluids.

**E-F01** An antibody or antibody derivative, wherein the antibody or antibody derivative binds to Epitope 3 of SEQ ID NO. 34

### (As an alternative, the claim of Embodiment E-A01 could also read:

**E-F01** An antibody, wherein the antibody binds to.

**E-F02** The antibody (or antibody derivative) according to Embodiment E-F01, wherein the antibody (or antibody derivative) binds to Epitope 3 of SEQ ID NO. 34 of HMGI-C, preferably of human HMGI-C.

**E-F03** The antibody (or antibody derivative) according to Embodiments **E-F01** or **E-F02,** wherein the antibody (or antibody derivative) also binds to Epitope 1 of SEQ ID NO. 32, preferably also binds to Epitope 1 of SEQ ID NO. 32 of HMGI-C, preferably of human HMGI-C.

**E-F04** The antibody (or antibody derivative) according to Embodiments **E-F01** to **E-F03,** wherein the antibody (or antibody derivative) binds to Epitope 3 of SEQ ID NO. 34 of human HMGI-C and binds also to Epitope 1 of SEQ ID NO. 32 of human HMGI-C.

**E-F05** The antibody (or antibody derivative) according to Embodiments **E-F01** to **E-F04,** wherein the antibody (or antibody derivative) binds to the amino acid sequence of the overlap between Epitope 1 and Epitope 3 of human HMGI-C of SEQ ID NO. 35.

**E-F06** The antibody (or antibody derivative) according to any one of Embodiments **E-F01** to **E-F05,** wherein the antibody is a single chain antibody, a single domain antibody, or a chimeric antibody.

**E-F07** The antibody or antibody derivative according to any one of Embodiments **E-F01** to **E-F05,** wherein said antibody derivative is a Fab fragment.

**E-F08** The antibody or antibody derivative for use according to any one of Embodiments **E-F01** to **E-F05,** wherein said antibody is a murine antibody, such as an IgG, or wherein said antibody is a human or humanized antibody, in particular wherein said humanized antibody is an IgG.

**E-F09** The antibody (or antibody derivative) according to any one of Embodiment **E-F01** to **E-F08,** wherein the antibody is a monoclonal antibody.

**E-F10** The antibody (or antibody derivative) according to any one of Embodiments **E-F01** to **E-F09,** wherein the antibody is a humanized antibody.

**E-F11** An isolated nucleic acid that encodes the antibody (or antibody derivative) according to any one of Embodiments **E-F01** to **E-F10.**

**E-F12** An expression vector encoding the antibody (or antibody derivative) according to any one of Embodiments **E-F01** to **E-F10.**

**E-F13** A host cell comprising a nucleic acid according to Embodiment **E-F11** or an expression vector according to Embodiment **E-F12.**

**E-F14** The host cell of Embodiment **E-F13** that produces the antibody (or antibody derivative) according to any one of Embodiments **E-F01** to **E-F10.**

**E-F15** A method of producing the antibody (or antibody derivative) according to any one of Embodiments **E-F01** to **E-F10,** comprising culturing the host cell that produces the antibody according to Embodiment **E-F14** and recovering the antibody from the cell culture.

**E-F16** A composition comprising the antibody (or antibody derivative) according to any one of Embodiments **E-F01** to **E-F10** and a carrier.

**E-F17** The composition of Embodiment **E-F16** wherein the carrier is a pharmaceutically acceptable carrier.

**E-F18** An article of manufacture or medical device comprising a container and a composition contained therein, wherein the composition comprises an antibody or antibody derivative according to any of the Embodiments **E-F01** to **E-F10,** preferably wherein the medical device is a lateral flow device for detection of an antigen, most preferably wherein the medical device is a lateral flow device for detection of the presence of human HMGI-C in a body fluid, like blood, whole blood, blood plasma or serum.

**E-F19** A method of treating a HMGI-C positive cancer, comprising administering to a patient suffering from the cancer, a therapeutically effective amount of the antibody (or antibody derivative) according to any one of Embodiments **E-F01** to **E-F18.**

**E-F20** An antibody (or antibody derivative) according to any one of Embodiments **E-F01** to **E-F10** for use in a method of treating HMGI-C positive cancer, wherein the method comprises the step of contacting a HMGI-C-positive cancer in a subject with said antibody or antibody derivative.

E-F21 A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
      wherein the 1-AAD-protein is human HMGI-C and the protein antibody binding to said 1-AAD-protein is an antibody of any one of the Embodiments **E-F01** to **E-F10.**

### PART G:

### (Drawn to the 3^{rd} Group of Antibodies - exemplified by preferred antibody ("141-3"))

**E-G01** An (isolated) antibody or antibody derivative/antigen binding fragment thereof, wherein the antibody or antibody derivative/antigen binding fragment thereof comprises in the heavy chain variable region (VH) (THREE (VH-3)) at least one of the amino acid CDR sequences CDR1 of SEQ ID NO. 22, CDR2 of SEQ ID NO. 23 and CDR3 of SEQ ID NO. 24.

### (As an alternative, the claim of Embodiment E-F01 could also read:

**E-G01** An (isolated) antibody, wherein the antibody comprises in the heavy chain variable region (VH) (THREE (VH-3)) at least one of the amino acid CDR sequences CDR1 of SEQ ID NO. 22, CDR2 of SEQ ID NO. 23 and CDR3 of SEQ ID NO. 24.

**E-G02** The (isolated) antibody (or antibody derivative/antigen binding fragment thereof) according to Embodiment **E-G01,** wherein the antibody (or antibody derivative/antigen binding fragment thereof) binds to HMGI-C, preferably binds to human HMGI-C.

**E-G03** The (isolated) antibody (or antibody derivative/antigen binding fragment thereof) according to Embodiments **E-G01** or **E-G02,** wherein the antibody (or antibody derivative/antigen binding fragment thereof) comprises in the heavy chain variable region THREE (VH-3) one (the) amino acid CDR sequence CDR3 of SEQ ID NO. 24.

**E-G04** The (isolated) antibody (or antibody derivative/antigen binding fragment thereof) according to any one of Embodiments **E-G01** to **E-G03,** wherein the antibody (or antibody derivative/antigen binding fragment thereof) comprises in the heavy chain variable region THREE (VH-3) together with the amino acid sequence CDR3 of SEQ ID NO. 24 at least one amino acid CDR sequence selected from amino acid sequence CDR1 of SEQ ID NO. 22 or amino acid sequence CDR2 of SEQ ID NO. 23.

**E-G05** The (isolated) antibody (or antibody derivative/antigen binding fragment thereof) according to any one of Embodiments **E-G01** to **E-G04,** wherein the antibody (or antibody derivative/antigen binding fragment thereof) comprises in the heavy chain variable region THREE (VH-3) together with the amino acid sequence CDR3 of SEQ ID NO. 24 the amino acid sequence CDR1 of SEQ ID NO. 22 and the amino acid sequence CDR2 of SEQ ID NO. 23.

**E-G06** The (isolated) antibody (or antibody derivative//antigen binding fragment thereof) according to any one of Embodiments **E-G01** to **E-G05,** wherein the antibody (or antibody derivative/antigen binding fragment thereof) comprises in the light chain variable region THREE (VL-3) at least one amino acid CDR sequence selected from amino acid sequence CDR1 of SEQ ID NO. 27, amino acid sequence CDR2 of SEQ ID NO. 28 and amino acid sequence CDR3 of SEQ ID NO. 29.

**E-G07** The (isolated) antibody (or antibody derivative/antigen binding fragment thereof) according to any one of Embodiments **E-G01** to **E-G06,** wherein the antibody (or antibody derivative/antigen binding fragment thereof) comprises in the light chain variable region THREE (VL-3) at least two amino acid CDR sequences selected from amino acid sequence CDR1 of SEQ ID NO. 27, amino acid sequence CDR2 of SEQ ID NO. 28 and amino acid sequence CDR3 of SEQ ID NO. 29.

**E-G08** The (isolated) antibody (or antibody derivative/antigen binding fragment thereof) according to any one of Embodiments **E-G01** to **E-G07,** wherein the antibody (or antibody derivative/antigen binding fragment thereof) comprises in the light chain variable region THREE (VL-3) amino acid sequence CDR1 of SEQ ID NO. 27, amino acid sequence CDR2 of SEQ ID NO. 28, and amino acid sequence CDR3 of SEQ ID NO. 29.

**E-G09** The (isolated) antibody (or antibody derivative/antigen binding fragment thereof) according to any one of Embodiments **E-G01** to **E-G08,** wherein the antibody (or antibody derivative) comprises
- in the heavy chain variable region THREE (VH-3) (the three CDRs of) amino acid sequence CDR1 of SEQ ID NO.22, amino acid sequence CDR2 of SEQ ID NO.23, and amino acid sequence CDR3 of SEQ ID NO.24 and
- in the light chain variable region THREE (VL-3) amino acid sequence CDR1 of SEQ ID NO. 27, amino acid sequence CDR2 of SEQ ID NO. 28 and amino acid sequence CDR3 of SEQ ID NO. 29.

**E-G10** The (isolated) antibody (or antibody derivative/antigen binding fragment thereof) according to Embodiment **E-G09,** wherein the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 21, especially a heavy chain with at least 95%, 99% or 100% sequence identity to SEQ ID NO: 21; preferably a heavy chain with at least 99% or 100% sequence identity to SEQ ID NO: 21; most preferably a heavy chain with 100% sequence identity to SEQ ID NO: 21.

**E-G11** The (isolated) antibody (or antibody derivative/antigen binding fragment thereof) according to Embodiments **E-G09** or **E-G10,** wherein the antibody (or antibody derivative/antigen binding fragment thereof) comprises a light chain (or light chain variable region LCVR) with at least 50%, 60%, 70%, 80%, 85%, 90%, 95%, 99% or 100% sequence identity to SEQ ID NO: 26; especially a light chain with at least 95%, 99% or 100% sequence identity to SEQ ID NO: 26; preferably a light chain with at least 99% or 100% sequence identity to SEQ ID NO: 26; most preferably a light chain with 100% sequence identity to SEQ ID NO: 26.

**E-G12** The (isolated) antibody (or antibody derivative/antigen binding fragment thereof) according to any one of Embodiments **E-G09** to **E-G11,** wherein the antibody (or antibody derivative/antigen binding fragment thereof) comprises a heavy chain (or heavy chain variable region HCVR) with at least 50%, 60%, 70%, 80%, 85%, 90%, 95%, 99% or 100% sequence identity to SEQ ID NO: 21 and a light chain with at least 50%, 60%, 70%, 80%, 85%, 90%, 95%, 99% or 100% sequence identity to SEQ ID NO: 26, especially a heavy chain with at least 95%, 99% or 100% sequence identity to SEQ ID NO: 21 and a light chain with at least 95%, 99% or 100% sequence identity to SEQ ID NO: 26; preferably a heavy chain with at least 99% or 100% sequence identity to SEQ ID NO: 21 and a light chain with at least 99% or 100% sequence identity to SEQ ID NO: 26; most preferably a heavy chain with 100% sequence identity to SEQ ID NO: 21 and a light chain with 100% sequence identity to SEQ ID NO: 26.

**E-G13** An (isolated) antibody (or antibody derivative/antigen binding fragment thereof) binding to HMGI-C, wherein the antibody (or antibody derivative/antigen binding fragment) comprises the three heavy chain CDRs CDR1, CDR2 and CDR3 contained within a heavy chain (or heavy chain variable region HCVR) with at least 98%, 99% or 100% sequence identity to SEQ ID NO: 21 and three light chain CDRs CDR1, CDR2 and CDR3 contained within a light chain (or light chain variable region LCVR) with at least 98%, 99% or 100% sequence identity to SEQ ID NO: 26.

**E-G14** The (isolated) antibody (or antibody derivative/antigen binding fragment thereof) according to Embodiment **E-G13,** binding to HMGI-C, wherein the antibody (or antibody derivative/antigen binding fragment thereof) comprises the three heavy chain CDRs CDR1, CDR2 and CDR3 contained within the heavy chain Variable region of SEQ ID NO: 21 and three light chain CDRs CDR1, CDR2 and CDR3 contained within a heavy chain with at least 98%, 99% or 100% sequence identity to SEQ ID NO: 26.

**E-G15** The (isolated) antibody (or antibody derivative/antigen binding fragment thereof) according to any one of Embodiments **E-G01** to **E-G09,** wherein the antibody (or antibody derivative/antigen binding fragment thereof) comprises
∘ in the light chain variable region THREE (VL-3) the amino acid sequence of SEQ ID NO.26 or an amino acid sequence of at least 80% sequence identity thereof.

**E-G16** The (isolated) antibody (or antibody derivative/antigen binding fragment thereof) according to Embodiment **E-G15,** wherein the antibody (or antibody derivative/antigen binding fragment thereof) comprises
∘ in the light chain variable region ONE (VL-1) the amino acid sequence of SEQ ID NO.26 or an amino acid sequence of at least 85% sequence identity thereof, preferably of at least 90% sequence identity thereof, more preferably of at least 95% sequence identity thereof, most preferably of at least 98% sequence identity thereof.

**E-G17** The (isolated) antibody (or antibody derivative/antigen binding fragment thereof) according to any one of Embodiments **E-G01** to **E-G09,** wherein the antibody (or antibody derivative/antigen binding fragment thereof) comprises
∘ a light chain variable region THREE (VL-3) of the amino acid sequence of SEQ ID NO.26.

**E-G18** The (isolated) antibody (or antibody derivative/antigen binding fragment thereof) according to any one of Embodiments **E-G01** to **E-G09,** wherein the antibody (or antibody derivative/antigen binding fragment thereof) comprises
∘ a heavy chain variable region ONE (VH-1) having amino acid sequence of SEQ ID NO. 21, and
∘ in the light chain variable region ONE (VL-1) having amino acid sequence of SEQ ID NO.26.

**E-G19** The (isolated) antibody (or antibody derivative/antigen binding fragment thereof) according to any one of Embodiments **E-G01** to **E-G09,** wherein the antibody (or antibody derivative/antigen binding fragment thereof) comprises
∘ a heavy chain variable region THREE (VH-3) encoded by nucleic acid sequence of SEQ ID NO. 25, and
∘ a light chain variable region THREE (VL-3) encoded by nucleic acid sequence of SEQ ID NO. 30.

**E-G20** The (isolated) antibody (or antibody derivative/antigen binding fragment thereof) according to any one of Embodiments **E-G01** to **E-G09,** wherein the antibody (or antibody derivative/antigen binding fragment thereof) comprises
∘ a heavy chain variable region THREE (VH-3) having amino acid sequence of SEQ ID NO. 21, and a light chain variable region THREE (VL-3) having amino acid sequence of SEQ ID NO.26; or
∘ a heavy chain variable region THREE (VH-3) encoded by nucleic acid sequence of SEQ ID NO.25, and a light chain variable region THREE (VL-3) encoded by nucleic acid sequence of SEQ ID NO. 30.

**E-G21** An (isolated) antibody (or antibody derivative/antigen binding fragment thereof) binding to HMGI-C, wherein the antibody (or antibody derivative/antigen binding fragment) comprises
(a) a heavy chain (or heavy chain variable region HCVR) having an amino acid sequence with at least 98%, 99% or 100% sequence identity to SEQ ID NO: 21 and a light chain (or light chain variable region LCVR) with at least 98%, 99% or 100% sequence identity to SEQ ID NO: 26; or
(b) a heavy chain (or heavy chain variable region HCVR) encoded by a nucleic acid sequence with at least 98%, 99% or 100% sequence identity to SEQ ID NO: 25 and a light chain (or light chain variable region LCVR) encoded by a nucleic acid sequence with at least 98%, 99% or 100% sequence identity to SEQ ID NO: 30;

**E-G22** The (isolated) antibody (or antibody derivative/antigen binding fragment thereof) according to **E-G21,** wherein the antibody (or antibody derivative/antigen binding fragment) comprises
(a) a heavy chain (or heavy chain variable region HCVR) having the amino acid sequence of SEQ ID NO: 21 and a light chain (or light chain variable region LCVR) having the amino acid sequence of SEQ ID NO: 26; or
(b) a heavy chain (or heavy chain variable region HCVR) encoded by a nucleic acid sequence of SEQ ID NO: 25 and a light chain (or light chain variable region LCVR) encoded by a nucleic acid sequence of SEQ ID NO: 30;

**E-G23** The (isolated) antibody (or antibody derivative/antigen binding fragment thereof) according to any one of Embodiments **E-G01** to **E-G22,** wherein the antibody (or antibody derivative/antigen binding fragment thereof) binds to Epitope 3 of SEQ ID NO. 34

**E-G24** The (isolated) antibody (or antibody derivative/antigen binding fragment thereof) according to Embodiment **E-G23,** wherein the antibody (or antibody derivative/antigen binding fragment thereof) binds to Epitope 3 of SEQ ID NO. 34 of HMGI-C, preferably of human HMGI-C.

**E-G25** The antibody (or antibody derivative/antigen binding fragment thereof) according to Embodiments **E-G23** or **E-G24,** wherein the antibody (or antibody derivative/antigen binding fragment thereof) also binds to Epitope 1 of SEQ ID NO. 32, preferably also binds to Epitope 1 of SEQ ID NO. 32 of HMGI-C, preferably of human HMGI-C.

**E-G26** The (isolated) antibody (or antibody derivative) according to Embodiments **E-G23** to **E-G25,** wherein the antibody (or antibody derivative) binds to Epitope 3 of SEQ ID NO. 34 of human HMGI-C and binds also to Epitope 1 of SEQ ID NO. 32 of human HMGI-C.

**E-G27** The (isolated) antibody (or antibody derivative) according to Embodiments **E-G23** to **E-G26,** wherein the antibody (or antibody derivative) binds to the amino acid sequence of the overlap between Epitope 1 and Epitope 3 of human HMGI-C of SEQ ID NO. 35.

**E-G28** The (isolated) antibody (or antibody derivative/antigen binding fragment thereof) according to any one of Embodiments **E-G01** to **E-G27,** wherein the antibody is a single chain antibody, a single domain antibody, or a chimeric antibody.

**E-G29** The (isolated) antibody or antibody derivative/antigen binding fragment thereof according to any one of Embodiments **E-G01** to **E-G27,** wherein said antibody derivative/antigen binding fragment thereof is a Fab fragment.

**E-G30** The antibody or antibody derivative for use according to any one of Embodiments **E-G01** to **E-G27,** wherein said antibody is a murine antibody, such as an IgG, or wherein said antibody is a human or humanized antibody, in particular wherein said humanized antibody is an IgG.

**E-G31** The antibody (or antibody derivative/antigen binding fragment thereof) according to any one of Embodiment **E-G01** to **E-G27,** wherein the antibody is a monoclonal antibody.

**E-G32** The antibody (or antibody derivative/antigen binding fragment thereof) according to any one of Embodiments **E-G01** to **E-G27,** wherein the antibody is a humanized antibody.

**E-G33** The (isolated) antibody (or antibody derivative/antigen binding fragment thereof) according to any one of Embodiments **E-G01** to **E-G32,** wherein the antibody (or antibody derivative/antigen binding fragment thereof) is effective to detect HMGI-C, preferably is effective to detect HMGI-C in a fluid.

**E-G34** The (isolated) antibody (or antibody derivative/antigen binding fragment thereof) according to Embodiment **E-G33,** wherein the fluid, is a body fluid, preferably is a body fluid of human origin.

**E-G35** The (isolated) antibody (or antibody derivative/antigen binding fragment thereof) according to any one of Embodiments **E-G33** or **E-G34,** wherein the fluid is selected from blood, urine, semen, sputum, saliva; preferably is blood including whole blood, blood serum or blood plasma.

**E-G36** The (isolated) antibody (or antibody derivative/antigen binding fragment thereof) according to any one of Embodiments **E-G01** to **E-G32,** wherein the antibody (or antibody derivative/antigen binding fragment thereof) binds (to) human HMGI-C and is effective to identify the presence of human HMGI-C in liquids, especially *in vitro.*

**E-G39** The (isolated) antibody (or antibody derivative/antigen binding fragment thereof) according to any one of Embodiments **E-G01** to **E-G34,** wherein the antibody (or antibody derivative/antigen binding fragment thereof) is effective to treat cancer related to HMGI-C, preferably is effective to treat cancer related to human HMGI-C *in vivo.*

**E-G40** The (isolated) antibody (or antibody derivative/antigen binding fragment thereof) according to Embodiments **E-G39,** wherein cancer related to HMGI-C is selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.

**E-G41** The (isolated) antibody (or antibody derivative/antigen binding fragment thereof) according to any one of Embodiments **E-G39** to **E-G40**, wherein the treatment involves a HMGI-C-positive cancer cell.

**E-G42** The (isolated) antibody (or antibody derivative/antigen binding fragment thereof) according to any one of Embodiments **E-G01** to **E-G24,** wherein the antibody (or antibody derivative/antigen binding fragment thereof) binds to a cancer cell-associated HMGI-C, preferably binds to a human cancer cell-associated HMGI-C.

**E-G43** The (isolated) antibody (or antibody derivative/antigen binding fragment thereof) according to any one of Embodiments **E-G41** to **E-G42,** wherein the human cancer cell to which the cancer cell-associated HMGI-C is associated or the HMGI-C-positive cancer cell is a solid tumor cancer cell, in particular a soft tissue cancer cell, especially an ovarian cancer, like epithelial ovarian cancer cell; nasopharyngeal cancer cell, renal cancer cell, epithelial cancer cell, prostate cancer cell, endometrial cancer cell, pancreatic cancer cell, laryngeal squamous cancer cell, bladder cancer cell, oral squamous cancer cell, cervical cancer cell, head and neck squamous cancer cell, hepatocellular cancer cell, tongue squamous cancer cell, especially oral tongue squamous cancer cell carcinoma; and osteosarcoma cell.

**E-G44** An isolated nucleic acid that encodes the antibody (or antibody derivative/antigen binding fragment thereof) according to any one of Embodiments **E-G01** to **E-G32.**

**E-G45** An expression vector encoding the (isolated) antibody (or antibody derivative/antigen binding fragment thereof) according to any one of Embodiments **E-G01** to **E-G32.**

**E-G46** A host cell comprising a nucleic acid according to Embodiment **E-G44** or an expression vector according to Embodiment **E-G45.**

**E-G47** The host cell of Embodiment **E-G46** that produces the (isolated) antibody (or antibody derivative/antigen binding fragment thereof) according to any one of Embodiments **E-G01** to **E-G32.**

**E-G48** A method of producing the (isolated) antibody (or antibody derivative/antigen binding fragment thereof) according to any one of Embodiments **E-G01** to **E-G32** comprising culturing the host cell according to Embodiment **E-G47** that produces the antibody and recovering the antibody from the cell culture.

**E-G49** A composition comprising the (isolated) antibody (or antibody derivative/antigen binding fragment thereof) according to any one of Embodiments **E-G01** to **E-G32** and a carrier.

**E-G50** The composition of Embodiment **E-G49** wherein the carrier is a pharmaceutically acceptable carrier.

**E-G51** An article of manufacture or medical device comprising a container and a composition contained therein, wherein the composition comprises an (isolated) antibody (or antibody derivative/antigen binding fragment thereof) according to any of the Embodiments **E-G01** to **E-G32,** preferably wherein the medical device is a lateral flow device for detection of an antigen, most preferably wherein the medical device is a lateral flow device for detection of the presence of human HMGI-C in a body fluid, like blood, whole blood, blood plasma or serum.

**E-G52** A method of treating a HMGI-C positive cancer, comprising administering to a patient suffering from the cancer, a therapeutically effective amount of the (isolated) antibody (or antibody derivative/antigen binding fragment thereof) according to any one of Embodiments **E-G01** to **E-G32.**

**E-G53** An (isolated) antibody (or antibody derivative/antigen binding fragment thereof) according to any one of Embodiments **E-G01** to **E-G32** for use in a method of treating HMGI-C positive cancer, wherein the method comprises the step of contacting a HMGI-C-positive cancer in a subject with said antibody or antibody derivative.

**EG54** A lateral flow device for the detection of a first protein released during an event leading to an acute aortic dissection (1-AAD-protein) in a sample of bodily fluid of a mammal, the device comprising:
a) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
      wherein the 1-AAD-protein is human HMGI-C and the protein antibody binding to said 1-AAD-protein is an antibody of any one of the Embodiments **E-G10** to **E-G34.**

The invention is further described with reference to the following examples. It will be appreciated that the invention as claimed is not intended to be limited in any way by these examples.

### EXAMPLES:

### EXAMPLE 1) Development of monoclonal antibodies against HMGI-C derived peptides

It was a

### A. Antibody Development

### 1. Animal Immunization

**Species:** Mouse
**Number of animals:** 5
**Antigen:** Peptide mix
**Immunization protocol:** Standard 45 days, 4 injections

| **Peptide/ Epitope** | **1** | **2** | **3** |
|---|---|---|---|
| **Sequence** | C-QKRGRGRPRKQQQE | C-IFSRDRVSP | C-RGRPRKWAGVQWYNLGSLQ |
| **MW** | **1855.12** | **1179.35** | **2375.73** |
| **Purity** | **>90%** | **>90%** | **>90%** |

### ELISA titer results after the last boost (before fusion)

NB: Negative = pre-immune serum: 1:2000 > 0.2; Standard ELISA without optimization for the respective antigen

### Coating: BSA-C-QKRGRGRPRKQQQE (5µg/ml)

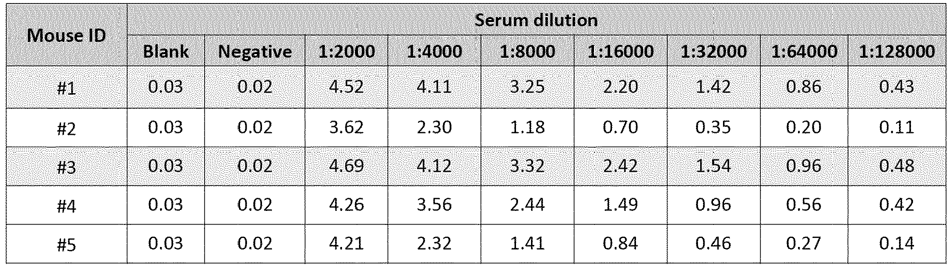

### Coating: BSA-C-RGRPRKWAGVQWYNLGSLQ (5µg/ml)

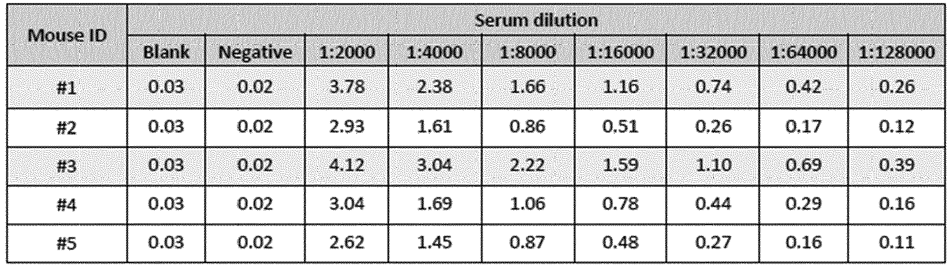

### Coating: IFSRDRVSP-C-BSA (5µg/ml)

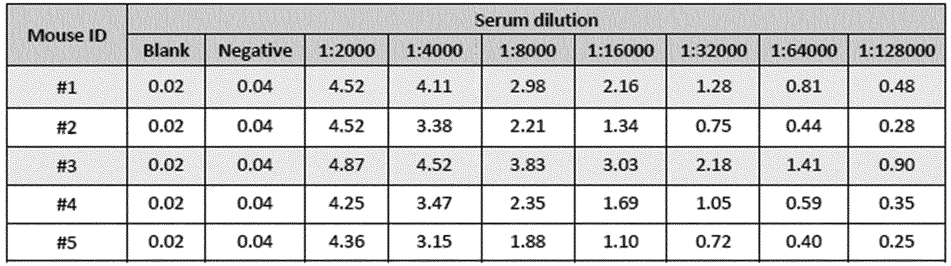

**Animals chosen for the fusion:** MOUSE #1 & MOUSE #3

### 2. Fusion * clone screening/selection

### Screening strategy:

- Screen against each of the three peptides individually
- Application: ELISA
- Additional positive screening against protein/peptide different from antigen: No
- Additional negative screening; No

### Clone selection:

- **Clones selected after fusion:** 3 clones
   ∘ Clone 4 with good specific binding against peptide QKRGRGRPRKQQQE
   ∘ Clone 43 with good specific binding against peptide IFSRDRVSP
   ∘ No clone was originally identified with specific binding against peptide RGRPRKWAGVQWYNLGSLQ. Mouse #1 was given booster shots specifically with peptide RGRPRKWAGVQWYNLGSLQ to obtain clones against this peptide. Clone #141 was later identified as specific for this peptide
- 2-3 rounds of sub-cloning performed

### Final clones obtained:

### Final clone: 4-H2-2-G6-E12

with good specific binding against peptide QKRGRGRPRKQQQE lsotype:lgG1
- 3 rounds of sub-cloning

### Final clone: 43-3-C5-A6

with good specific binding against peptide IFSRDRVSP Isotype: lgG2b
- 2 rounds of sub-cloning

### Final clone: 141-3-F2-H4

- with good binding to **RGRPRK**WAGVQWYNLGSLQ but also displays binding to QKRG**RGRPRK**QQQE (peptide 1 above) due to the overlap in 6 shared amino acids (see SEQ ID NO: 35 and underlined)
   Isotype: lgG2b
- 2 rounds of sub-cloning

### ELISA results of final clones (supernatants)

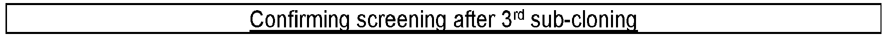

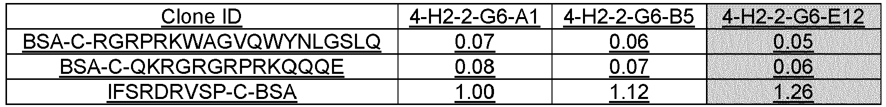

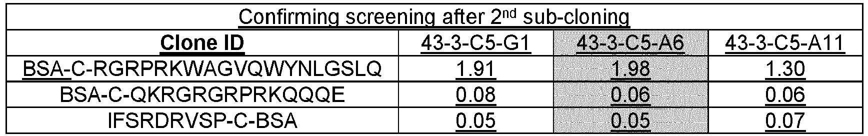

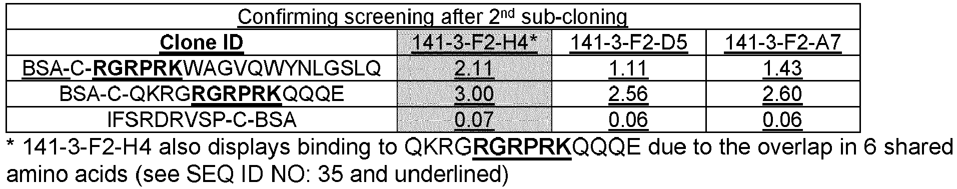

### PURIFICATION SHOWN IN SDS PAGE:

**Fig. 13A**) shows an SDS PAGE (QC) and shows the purified antibody production from the final clones 4-H2-2-G6-E12 and 43-3-C5-A6 with:
- lane no. 1 showing antibody-43-3-C5-A6 (2µg, 10% reduce SDS-PAGE)
- lane no. 2 showing antibody-4-H2-2-G6-E12 (2µg, 10% reduce SDS-PAGE)
- lane no. 3 showing antibody-43-3-C5-A6 (2µg, 10% non-reduce SDS-PAGE)
- lane no. 4 showing antibody-4-H2-2-G6-E12 (2µg, 10% non-reduce SDS-PAGE).

**Fig. 13B**) shows an SDS PAGE (QC) and shows the purified antibody production from the final clone 141-3-F2-H4 with:
- lane no. 1 showing antibody-141-3-F2-H4 (2µg, 10% reduce SDS-PAGE)
- lane no. 2 showing antibody-141-3-F2-H4 (2µg, 10% non-reduce SDS-PAGE)

### EXAMPLE 2) Sequencing of full-length HC and LC genes of hybridomas

Full-length HC and LC genes of hybridomas were sequenced. As already laid out in Example 1), these hybrodomas (clones) were named 4-H2-2-G6-E12 (abbreviated hereinafter "4-H2"), 43-3-C5-A6 (abbreviated hereinafter "43-3") and 141-3-F2-H4 (abbreviated hereinafter "141-3"). Hybrodoma 4-H2 was designed against the peptide QKRGRGRPRKQQQE. This peptide is the Epitope 1 (SEQ ID NO. 32) of HMGI-C. Hybrodoma 43-3 was designed against peptide IFSRDRVSP. This peptide is the Epitope 2 (SEQ ID NO. 33) of human HMGI-C. Hybrodoma 141-3 was designed against peptide RGRPRKWAGVQWYNLGSLQ. This peptide is the Epitope 3 (SEQ ID NO. 34) of human HMGI-C.

### Overview

Total RNA was extracted from the hybridoma cells and cDNA subsequently synthesized. Antibody full length genes were then amplified by isotype-specific PCR, subcloned into a standard cloning vector separately and sequenced.

### Materials

- Hybridoma clones, commercial
- TaKaRa MiniBEST Universal RNA Extraction Kit (Takara)
- PrimeScript RT reagent Kit with gDNA Eraser (Takara)
- TA-cloning Kit (Takara)

### Methods

Total RNA was extracted separately from the hybridoma cell line, cDNA was then synthetized by reverse transcription using oligo-dT primers and HC/LC were finally amplified by PCR. HC/LC fragments, respectively amplified by IgG degenerate primers and kappa specific primers, could be observed by gel electrophoresis. The PCR products were then sub-cloned into a standard vector, followed by clone picking and validation by PCR and finally sequencing of positive clones. Sequences were analyzed via Vbase2 and IMGT/V-QUEST.

The discovered sequences are found in the Sequence Listing below.

| **Sequence** | **Number of Clones sequenced** | **Clones with <99% sequence Identity** |
|---|---|---|
| **4-H2-2-G6-E12-HC** | 5 | 100% |
| **4-H2-2-G6-E12-LC** | 5 | 100% |
| **43-3-C5-A6-HC** | 5 | 100% |
| **43-3-C5-A6-LC** | 5 | 100% |
| **141-3-F2-H4-HC** | 5 | 100% |
| **141-3-F2-H4-LC** | 5 | 100% |

### Publications:

1. Isselbacher EM. Disease of the aorta. Heart Disease, 6th edn. Braunwald E, Zipes DP, Libby P, Eds. W.B. Saunders Company, Philadelphia, 2001;1422-56.
2. Nienaber CA, Fattori R, Mehta RH, Richartz BM, Evangelista A, Petzsch M, et al. The International Registry of Acute Aortic Dissection (IRAD). Gender-Related differences in acute aortic dissection. Circulation. 2003;109:3014-21.
3. Pearson GD, Devereux R, Loeys B, Maslen C, Milewicz D, Pyeritz R, Ramirez F, et al. Report of the National Heart, Lung, and Blood Insti- tute and National Marfan Foundation Working Group on research in Marfan syndrome and related disorders. Circulation. 2008;118: 785-91.
4. Parapia LA, Jackson C. Ehlers-Danlos syndrome-a historical review. Br J Haematol. 2008;141:32-5.
5. Callewaert B, Malfait F, Loeys B, De Paepe A. Ehlers-Danlos syn- dromes and Marfan syndrome. Best Pract Res Clin Rheumatol. 2008;22:165-89.
6. Roberts CS, Roberts WC. Dissection of the aorta with congenital malformation of the aortic valve. J Am Coll Cardiol. 1991;17: 712-6.
7. Mehta RH, Suzuki T, Hagan PG, et al. International Registry of Acute Aortic Dissection (IRAD). Investigators. Predicting death in patients with acute type a aortic dissection. Circulation. 2002;105: 200-6.
8. Daily PO, Trueblood HW, Stinson EB, Wuerflein RD, Shumway NE. Management of acute aortic dissections. Ann Thorac Surg. 1970;10: 237-47.
9. DeBakey ME, McCollum CH, Crawford ES, Morris GC, Howell J, Noon GP, Lawrie G. Dissection and dissecting aneurysms of the aorta: twenty-year follow-up of five hundred twenthy-seven patients treated surgically. Surgery. 1980;92:1118-34.
10. Anagnostopoulos CE, Prabhakar MJS, Kittle CF. Aortic dissections and dissecting aneurysms. Am J Cardiol. 1972;30:263-73.
11. Estrera AL, Huynh TT, Porat EE, Miller CC 3rd, Smith JJ, Safi HJ. Is acute type A aortic dissection a true surgical emergency? Semin Vasc Surg. 2002;15:75-82.
12. Miller DC. Acute dissection of the aorta: continuing need for earlier diagnosis and treatment. Mod Con Cardiovasc Dis. 1985;54:51-55.
13. Roberts WC. Aortic dissection: anatomy, consequences, and causes. Am Heart J. 1981;101:195-214.
14. König et al. Aggrecan: a new biomarker for acute type A aortic dissection. Sci Rep 11, 10371 (2021). https://doi.org/10.1038/s41598-021-89653-y.
15. Belge et al. Upregulation of the high mobility group AT-hook 2 gene in acute aortic dissection is potentially associated with endothelial-mesenchymal transition. Histol Histopathol. 2011 Aug;26(8):1029-37. doi: 10.14670/HH-26.1029.
16. Fusco A. and Fedele M., Roles of HGA proteins in cancer, Nature Reviews/Cancer, Vol. 7, December 2007: 899-910
17. Ma et al. HMGA2 promotes cancer metastasis by regulating epithelial-mesenchymal transition, Frontiers in Oncology, 01 February 2024, 01-10

### SEQUENCE LISTING

### SEQ ID NO: 1

Amino acid sequence of the heavy chain variable domain (VH) of the antibody 4-H2 with the CDRs underlined.

EVQLQQSGPE LVKPGASVRI SCKASGYSFT GHYMHWVKQS PENSLEWIGE INPTTGGITY
NQKFKGKATL TVDKSSITAY MHLKSLTSED SAVYYCTGGG DHFDYWGQGT TLTVSSAKTT
PPSVYPLAPG SAAQTNSMVT LGCLVKGYFP EPVTVTWNSG SLSSGVHTFP AVLQSDLYTL
SSSVTVPSST WPSETVTCNV AHPASSTKVD KKIVPRDCGC KPCICTVPEV SSVFIFPPKP
KDVLTITLTP KVTCVVVDIS KDDPEVQFSW FVDDVEVHTA QTQPREEQFN STFRSVSELP
IMHQDWLNGK EFKCRVNSAA FPAPIEKTIS KTKGRPKAPQ VYTIPPPKEQ MAKDKVSLTC
MITDFFPEDI TVEWQWNGQP AENYKNTQPI MDTDGSYFVY SKLNVQKSNW EAGNTFTCSV
LHEGLHNHHT EKSLSHSPGK

### SEQ ID NO: 2

Amino acid sequence of **CDR1** of the heavy chain variable domain (VH) of the antibody 4-H2:
GYSFTGHY

### SEQ ID NO: 3

Amino acid sequence of **CDR2** of the heavy chain variable domain (VH) of the antibody 4-H2:
**INPTTGGI**

### SEQ ID NO: 4

Amino acid sequence of **CDR3** of the heavy chain variable domain (VH) of the antibody 4-H2:
**TGGGDHFDY**

### SEQ ID NO: 5

**DNA** sequence of the heavy chain variable domain (VH) of the antibody 4-H2:

GAGGTCCAAC TGCAGCAGTC TGGACCTGAG CTGGTGAAGC CTGGGGCTTC AGTGAGGATA
TCCTGCAAGG CTTCTGGTTA CTCATTCACT GGCCACTACA TGCACTGGGT GAAGCAAAGT
CCTGAAAATA GTCTTGAGTG GATTGGAGAG ATTAATCCTA CCACTGGGGG TATTACCTAC
AACCAGAAGT TCAAGGGCAA GGCCACATTA ACTGTAGATA AATCCTCCAT CACAGCCTAC
ATGCACCTCA AGAGCCTGAC ATCTGAAGAC TCTGCAGTCT ATTACTGTAC AGGCGGGGGG
GACCACTTTG ACTACTGGGG CCAAGGCACC ACTCTCACAG TCTCCTCAGC CAAAACGACA
CCCCCATCTG TCTATCCACT GGCCCCTGGA TCTGCTGCCC AAACTAACTC CATGGTGACC
CTGGGATGCC TGGTCAAGGG CTATTTCCCT GAGCCAGTGA CAGTGACCTG GAACTCTGGA
TCCCTGTCCA GCGGTGTGCA CACCTTCCCA GCTGTCCTGC AGTCTGACCT CTACACTCTG
AGCAGCTCAG TGACTGTCCC CTCCAGCACC TGGCCCAGCG AGACCGTCAC CTGCAACGTT
GCCCACCCGG CCAGCAGCAC CAAGGTGGAC AAGAAAATTG TGCCCAGGGA TTGTGGTTGT
AAGCCTTGCA TATGTACAGT CCCAGAAGTA TCATCTGTCT TCATCTTCCC CCCAAAGCCC
AAGGATGTGC TCACCATTAC TCTGACTCCT AAGGTCACGT GTGTTGTGGT AGACATCAGC
AAGGATGATC CCGAGGTCCA GTTCAGCTGG TTTGTAGATG ATGTGGAGGT GCACACAGCT
CAGACGCAAC CCCGGGAGGA GCAGTTCAAC AGCACTTTCC GCTCAGTCAG TGAACTTCCC
ATCATGCACC AGGACTGGCT CAATGGCAAG GAGTTCAAAT GCAGGGTCAA CAGTGCAGCT
TTCCCTGCCC CCATCGAGAA AACCATCTCC AAAACCAAAG GCAGACCGAA GGCTCCACAG
GTGTACACCA TTCCACCTCC CAAGGAGCAG ATGGCCAAGG ATAAAGTCAG TCTGACCTGC
ATGATAACAG ACTTCTTCCC TGAAGACATT ACTGTGGAGT GGCAGTGGAA TGGGCAGCCA
GCGGAGAACT ACAAGAACAC TCAGCCCATC ATGGACACAG ATGGCTCTTA CTTCGTCTAC
AGCAAGCTCA ATGTGCAGAA GAGCAACTGG GAGGCAGGAA ATACTTTCAC CTGCTCTGTG
TTACATGAGG GCCTGCACAA CCACCATACT GAGAAGAGCC TCTCCCACTC TCCTGGTAAA

### SEQ ID NO: 6

Amino acid sequence of the light chain variable domain (VL) of the antibody 4-H2 with the CDRs underlined.

DVSMTQSPLS LPVSLGDQVS ISCRSSQSIL HSNGNTYLEW FLQRPGQSPK
LLIYKVSNRF SGVPDRFSGS GSGTDFTLKI SRVEAEDLGV YYCFQGSHDP
PTFGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC

### SEQ ID NO: 7

Amino acid sequence of **CDR1** of the light chain variable domain (VL) of the antibody 4-H2:
**QSILHSNGNTY**

### SEQ ID NO: 8

Amino acid sequence of **CDR2** of the light chain variable domain (VL) of the antibody 4-H2:
**KVS**

### SEQ ID NO: 9

Amino acid sequence of **CDR3** of the light chain variable domain (VL) of the antibody 4-H2:
**FQGSHDPPT**

### SEQ ID NO: 10

**DNA** sequence of the light chain variable domain (VL) of the antibody 4-H2:

GATGTTTCGA TGACCCAAAG TCCACTCTCC CTGCCTGTCA GTCTTGGAGA
TCAAGTCTCC ATCTCTTGCA GATCCAGTCA GAGCATTTTA CATAGTAACG
GAAACACCTA TTTAGAATGG TTCCTGCAGA GACCAGGCCA GTCTCCAAAG
CTCCTGATCT ACAAAGTTTC CAACCGATTT TCTGGGGTCC CAGACAGGTT
CAGTGGCAGT GGATCAGGGA CAGATTTCAC ACTCAAGATC AGCAGAGTGG
AGGCTGAGGA TCTGGGAGTT TATTACTGCT TTCAAGGTTC ACATGATCCT
CCGACGTTCG GTGGAGGCAC CAAGCTGGAA ATCAAACGGG CTGATGCTGC
ACCAACTGTA TCCATCTTCC CACCATCCAG TGAGCAGTTA ACATCTGGAG
GTGCCTCAGT CGTGTGCTTC TTGAACAACT TCTACCCCAA AGACATCAAT
GTCAAGTGGA AGATTGATGG CAGTGAACGA CAAAATGGCG TCCTGAACAG
TTGGACTGAT CAGGACAGCA AAGACAGCAC CTACAGCATG AGCAGCACCC
TCACGTTGAC CAAGGACGAG TATGAACGAC ATAACAGCTA TACCTGTGAG

GCCACTCACA AGACATCAAC TTCACCCATT GTCAAGAGCT TCAACAGGAA
TGAGTGT

### SEQ ID NO: 11

Amino acid sequence of the heavy chain variable domain (VH) of the antibody 43-3 with the CDRs underlined.

QIQLVQSGPE LKKPGETVKI SCKASGYTFT NYGMNWVKQA PGKDLKWMGW
INTYTGEPTY ADDFKGRFVF SLETSASTAY LQINNLNNKD TATYFCAREG
RLLYFHVWGA GTTVTVSSAK TTPPSVYPLA PGCGDTTGSS VTLGCLVKGY
FPESVTVTWN SGSLSSSVHT FPALLQSGLY TMSSSVTVPS STWPSQTVTC
SVAHPASSTT VDKKLEPSGP ISTINPCPPC KECHKCPAPN LEGGPSVFIF
PPNIKDVLMI SLTPKVTCVV VDVSEDDPDV RISWFVNNVE VHTAQTQTHR
EDYNSTIRVV SALPIQHQDW MSGKEFKCKV NNKDLPSPIE RTISKIKGLV
RAPQVYILPP PAEQLSRKDV SLTCLVVGFN PGDISVEWTS NGHTEENYKD
TAPVLDSDGS YFIYSKLDIK TSKWEKTDSF SCNVRHEGLK NYYLKKTISR SPGK

### SEQ ID NO. 12

Amino acid sequence of **CDR1** of the heavy chain variable domain (VH) of the antibody 43-3:
**GYTFTNYG**

### SEQ ID NO. 13

Amino acid sequence of **CDR2** of the heavy chain variable domain (VH) of the antibody 43-3:
**INTYTGEP**

### SEQ ID NO. 14

Amino acid sequence of **CDR3** of the heavy chain variable domain (VH) of the antibody 43-3:
**AREGRLLYFHV**

### SEQ ID NO. 15

**DNA** sequence of the heavy chain variable domain (VH) of the antibody 43-3:

CAGATCCAGT TGGTGCAGTC TGGACCTGAG CTGAAGAAGC CTGGAGAGAC
AGTCAAGATC TCCTGCAAGG CTTCTGGGTA TACCTTCACA AACTATGGAA
TGAACTGGGT GAAGCAGGCT CCAGGAAAGG ATTTAAAGTG GATGGGCTGG
ATAAACACCT ACACTGGAGA GCCAACATAT GCTGATGACT TCAAGGGACG
GTTTGTCTTC TCTTTGGAAA CCTCTGCCAG CACTGCCTAT TTGCAGATCA
ACAACCTCAA CAATAAAGAC ACGGCTACAT ATTTCTGTGC AAGAGAGGGG
AGGCTACTAT ATTTCCATGT CTGGGGCGCA GGGACCACGG TCACCGTCTC
CTCAGCTAAA ACAACACCCC CATCAGTCTA TCCACTGGCC CCTGGGTGTG
GAGATACAAC TGGTTCCTCT GTGACTCTGG GATGCCTGGT CAAGGGCTAC
TTCCCTGAGT CAGTGACTGT GACTTGGAAC TCTGGATCCC TGTCCAGCAG
TGTGCACACC TTCCCAGCTC TCCTGCAGTC TGGACTCTAC ACTATGAGCA
GCTCAGTGAC TGTCCCCTCC AGCACCTGGC CAAGTCAGAC CGTCACCTGC
AGCGTTGCTC ACCCAGCCAG CAGCACCACG GTGGACAAAA AACTTGAGCC
CAGCGGGCCC ATTTCAACAA TCAACCCCTG TCCTCCATGC AAGGAGTGTC
ACAAATGCCC AGCTCCTAAC CTCGAGGGTG GACCATCCGT CTTCATCTTC
CCTCCAAATA TCAAGGATGT ACTCATGATC TCCCTGACAC CCAAGGTCAC
GTGTGTGGTG GTGGATGTGA GCGAGGATGA CCCAGACGTC CGGATCAGCT
GGTTTGTGAA CAACGTGGAA GTACACACAG CTCAGACACA AACCCATAGA
GAGGATTACA ACAGTACTAT CCGGGTGGTC AGTGCCCTCC CCATCCAGCA
CCAGGACTGG ATGAGTGGCA AGGAGTTCAA ATGCAAGGTC AACAACAAAG
ACCTCCCATC ACCCATCGAG AGAACCATCT CAAAAATTAA AGGGCTAGTC
AGAGCTCCAC AAGTATACAT CTTGCCGCCA CCAGCAGAGC AGTTGTCCAG
GAAAGATGTC AGTCTCACTT GCCTGGTCGT GGGCTTCAAC CCTGGAGACA
TCAGTGTGGA GTGGACCAGC AATGGGCATA CTGAGGAGAA CTACAAGGAC
ACCGCACCAG TCCTGGACTC TGACGGTTCT TACTTCATAT ACAGCAAGCT
CGATATAAAA ACAAGCAAGT GGGAGAAAAC AGATTCCTTC TCATGCAACG
TGAGACACGA GGGTCTGAAA AATTACTACC TGAAGAAGAC CATCTCCCGG
TCTCCGGGTA AA

### SEQ ID NO: 16

Amino acid sequence of the light chain variable domain (VL) of the antibody 43-3 with the CDRs underlined.

DVLMTQTPLS LPVSLGDQAS ISCRSSQRIV HSNGNTYLEW YLQKPGQSPK
LLIYKVSNRF SGVPDRFSGS GSGTDFTLKI TRVEAEDLGV YYCFQGSHVP
YTFGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC

### SEQ ID NO: 17

Amino acid sequence of **CDR1** of the light chain variable domain (VL) of the antibody 43-3:
**QRIVHSNGNTY**

### SEQ ID NO: 18

Amino acid sequence of **CDR2** of the light chain variable domain (VL) of the antibody 43-3:
**KVS**

### SEQ ID NO: 19

Amino acid sequence of **CDR3** of the light chain variable domain (VL) of the antibody 43-3:
FQGSHVPYT

### SEQ ID NO: 20

**DNA** sequence of the light chain variable domain (VL) of the antibody 43-3:

GATGTTTTGA TGACCCAAAC TCCACTCTCC CTGCCTGTCA GTCTTGGAGA
TCAAGCCTCC ATCTCTTGCA GATCTAGTCA GCGCATTGTA CATAGTAATG
GAAACACGTA TTTAGAATGG TACCTGCAGA AACCAGGCCA GTCTCCAAAG

CTCCTGATCT ACAAAGTTTC CAACCGATTT TCTGGGGTCC CAGACAGGTT
CAGTGGCAGT GGATCAGGGA CAGATTTCAC ACTCAAGATC ACCAGAGTGG
AGGCTGAGGA TCTGGGAGTT TATTACTGCT TTCAAGGTTC ACATGTTCCG
TACACATTCG GAGGGGGGAC CAAGCTGGAA ATAAAACGGG CTGATGCTGC
ACCAACTGTA TCCATCTTCC CACCATCCAG TGAGCAGTTA ACATCTGGAG
GTGCCTCAGT CGTGTGCTTC TTGAACAACT TCTACCCCAA AGACATCAAT
GTCAAGTGGA AGATTGATGG CAGTGAACGA CAAAATGGCG TCCTGAACAG
TTGGACTGAT CAGGACAGCA AAGACAGCAC CTACAGCATG AGCAGCACCC
TCACGTTGAC CAAGGACGAG TATGAACGAC ATAACAGCTA TACCTGTGAG
GCCACTCACA AGACATCAAC TTCACCCATT GTCAAGAGCT TCAACAGGAA
TGAGTGT

### SEQ ID NO: 21

Amino acid sequence of the heavy chain variable domain (VH) of the antibody 141-3 with the CDRs underlined.

EVKLEQSGPE LKKPGETVKI SCMASGYTFT DYSIHWVRQA PGKGLKWMAW
INTETGETTY ADDFKGRFAF SLETSASTAY LQINNLENED TATYYCARWG
YDYYDGMDYW GQGTSVTVSS AKTTPPSVYP LAPGCGDTTG SSVTLGCLVK
GYFPESVTVT WNSGSLSSSV HTFPALLQSG LYTMSSSVTV PSSTWPSQTV
TCSVAHPASS TTVDKKLEPS GPISTINPCP PCKECHKCPA PNLEGGPSVF
IFPPNIKDVL MISLTPKVTC VVVDVSEDDP DVRISWFVNN VEVHTAQTQT
HREDYNSTIR VVSALPIQHQ DWMSGKEFKC KVNNKDLPSP IERTISKIKG
LVRAPQVYIL PPPAEQLSRK DVSLTCLVVG FNPGDISVEW TSNGHTEENY
KDTAPVLDSD GSYFIYSKLD IKTSKWEKTD SFSCNVRHEG LKNYYLKKTI
SRSPGK

### SEQ ID NO. 22

Amino acid sequence of **CDR1** of the heavy chain variable domain (VH) of the antibody 141-3:
**GYTFTDYS**

### SEQ ID NO. 23

Amino acid sequence of **CDR2** of the heavy chain variable domain (VH) of the antibody 141-3:
**INTETGET**

### SEQ ID NO. 24

Amino acid sequence of **CDR3** of the heavy chain variable domain (VH) of the antibody 141-3:
**ARWGYDYYDGMDY**

### SEQ ID NO. 25

DNA sequence of the heavy chain variable domain (VH) of the antibody 141-3:

GAGGTGAAGC TGGAGCAGTC AGGACCTGAG CTGAAGAAGC CTGGAGAGAC
AGTCAAGATC TCCTGCATGG CTTCTGGTTA CACCTTCACA GACTATTCAA
TTCACTGGGT GAGGCAGGCT CCAGGAAAGG GTTTAAAGTG GATGGCCTGG
ATAAACACTG AGACTGGTGA GACAACATAT GCAGATGACT TCAAGGGACG
GTTTGCCTTC TCTTTGGAAA CCTCTGCCAG CACGGCCTAT TTGCAGATCA
ACAACCTCGA AAATGAGGAC ACGGCTACAT ATTACTGTGC TAGGTGGGGG
TATGATTACT ACGATGGTAT GGACTACTGG GGTCAAGGAA CTTCAGTCAC
CGTCTCCTCA GCTAAAACAA CACCCCCATC AGTCTATCCA CTGGCCCCTG
GGTGTGGAGA TACAACTGGT TCCTCTGTGA CTCTGGGATG CCTGGTCAAG
GGCTACTTCC CTGAGTCAGT GACTGTGACT TGGAACTCTG GATCCCTGTC
CAGCAGTGTG CACACCTTCC CAGCTCTCCT GCAGTCTGGA CTCTACACTA
TGAGCAGCTC AGTGACTGTC CCCTCCAGCA CCTGGCCAAG TCAGACCGTC
ACCTGCAGCG TTGCTCACCC AGCCAGCAGC ACCACGGTGG ACAAAAAACT
TGAGCCCAGC GGGCCCATTT CAACAATCAA CCCCTGTCCT CCATGCAAGG
AGTGTCACAA ATGCCCAGCT CCTAACCTCG AGGGTGGACC ATCCGTCTTC
ATCTTCCCTC CAAATATCAA GGATGTACTC ATGATCTCCC TGACACCCAA
GGTCACGTGT GTGGTGGTGG ATGTGAGCGA GGATGACCCA GACGTCCGGA
TCAGCTGGTT TGTGAACAAC GTGGAAGTAC ACACAGCTCA GACACAAACC
CATAGAGAGG ATTACAACAG TACTATCCGG GTGGTCAGTG CCCTCCCCAT
CCAGCACCAG GACTGGATGA GTGGCAAGGA GTTCAAATGC AAGGTCAACA

ACAAAGACCT CCCATCACCC ATCGAGAGAA CCATCTCAAA AATTAAAGGG
CTAGTCAGAG CTCCACAAGT ATACATCTTG CCGCCACCAG CAGAGCAGTT
GTCCAGGAAA GATGTCAGTC TCACTTGCCT GGTCGTGGGC TTCAACCCTG
GAGACATCAG TGTGGAGTGG ACCAGCAATG GGCATACTGA GGAGAACTAC
AAGGACACCG CACCAGTCCT GGACTCTGAC GGTTCTTACT TCATATACAG
CAAGCTCGAT ATAAAAACAA GCAAGTGGGA GAAAACAGAT TCCTTCTCAT
GCAACGTGAG ACACGAGGGT CTGAAAAATT ACTACCTGAA GAAGACCATC
TCCCGGTCTC CGGGTAAA

### SEQ ID NO: 26

Amino acid sequence of the light chain variable domain (VL) of the antibody 141-3 with the CDRs underlined.

DVQITQSPSY LAASPGETIT INCRASKRIS KNLAWYQEKP GKTIKLLIYS
GSTLQSGIPS RFSGSGSGRD FTLIISSLEP EDIAMYYCQQ NNEYPLTFGA
GTKLELKRAD AAPTVSIFPP SSEQLTSGGA SVVCFLNNFY PKDINVKWKI
DGSERQNGVL NSWTDQDSKD STYSMSSTLT LTKDEYERHN SYTCEATHKT
STSPIVKSFN RNEC

### SEQ ID NO: 27

Amino acid sequence of **CDR1** of the light chain variable domain (VL) of the antibody 141-3:
**KRISKN**

### SEQ ID NO: 28

Amino acid sequence of **CDR2** of the light chain variable domain (VL) of the antibody 141-3:
**SGS**

### SEQ ID NO: 29

Amino acid sequence of **CDR3** of the light chain variable domain (VL) of the antibody 141-3:
**QQNNEYPLT**

### SEQ ID NO: 30

**DNA** sequence of the light chain variable domain (VL) of the antibody 141-3:

GATGTCCAGA TAACCCAGTC TCCATCTTAT CTTGCTGCAT CTCCTGGAGA AACCATTACT
ATTAATTGCA GGGCAAGTAA GAGAATTAGC AAAAATTTAG CCTGGTATCA GGAGAAACCT
GGGAAAACTA TTAAGCTTCT TATCTACTCT GGATCCACTT TGCAATCTGG AATTCCATCA
AGGTTCAGTG GCAGTGGATC TGGTAGAGAT TTCACTCTCA TCATCAGTAG CCTGGAGCCT
GAAGATATTG CAATGTATTA CTGTCAACAG AATAATGAAT ACCCGCTCAC GTTCGGTGCT
GGGACCAAGC TGGAGCTGAA ACGGGCTGAT GCTGCACCAA CTGTATCCAT CTTCCCACCA
TCCAGTGAGC AGTTAACATC TGGAGGTGCC TCAGTCGTGT GCTTCTTGAA CAACTTCTAC
CCCAAAGACA TCAATGTCAA GTGGAAGATT GATGGCAGTG AACGACAAAA TGGCGTCCTG
AACAGTTGGA CTGATCAGGA CAGCAAAGAC AGCACCTACA GCATGAGCAG CACCCTCACG
TTGACCAAGG ACGAGTATGA ACGACATAAC AGCTATACCT GTGAGGCCAC TCACAAGACA
TCAACTTCAC CCATTGTCAA GAGCTTCAAC AGGAATGAGT GT

### SEQ ID NO. 31:

Amino acid sequence of human HMGI-C (High mobility group protein) with Epitope 1, Epitope 3 and Epitope 2 underlined.

1      MSARGEGAGQ PSTSAQGQPA APAPQKRGRG RPRKQQQEPT GEPSPKRPRG
51     RPKGSKNKSP SKAAQKKAEA TGEKRPRGRP RKWAGVQWYN LGSLQPPPPR
101    FKQFSCLRLL SSWDYRHPPP HPANFCIFSR DRVSPCWPGW SRTPDLR

Just for declaration purpose, the same amino acid sequence of human HMGI-C (High mobility group protein) with Epitope 1, Epitope 3 and Epitope 2 underlined and the identical part in in Epitope 1 and Epitope 3 highlighted in grey.

1     MSARGEGAGQ PSTSAQGQPA APAPQKRGRG RPRKQQQEPT GEPSPKRPRG
51    RPKGSKNKSP SKAAQKKAEA TGEKRPRGRP RKWAGVQWYN LGSLQPPPPR
101   FKQFSCLRLL SSWDYRHPPP HPANFCIFSR DRVSPCWPGW SRTPDLR

### SEQ ID NO. 32:

Amino acid sequence of Epitope 1 of human HMGI-C (High mobility group protein)
QKRGRGRPRKQQQE

### SEQ ID NO. 33:

Amino acid sequence of Epitope 2 of human HMGI-C (High mobility group protein)
IFSRDRVSP

### SEQ ID NO. 34:

Amino acid sequence of Epitope 3 of human HMGI-C (High mobility group protein)
RGRPRKWAGVQWYNLGSLQ

### SEQ ID NO. 35:

Amino acid sequence of the overlap between Epitope 1 and Epitope 3 of human HMGI-C (High mobility group protein)
RGRPRK

### SEQ ID NO: 36

Amino acid sequence of **FR1** of the heavy chain variable domain (VH) of the antibody 4-H2:
EVQLQQSGPELVKPGASVRISCKAS

### SEQ ID NO: 37

Amino acid sequence of **FR2** of the heavy chain variable domain (VH) of the antibody 4-H2:
MHWVKQSPENSLEWIGE

### SEQ ID NO: 38

Amino acid sequence of **FR3** of the heavy chain variable domain (VH) of the antibody 4-H2:
TYNQKFKGKATLTVDKSSITAYMHLKSLTSEDSAVYYC

### SEQ ID NO: 39

Amino acid sequence of **FR4** of the heavy chain variable domain (VH) of the antibody 4-H2:

WGQGT TLTVSSAKTT PPSVYPLAPG SAAQTNSMVT LGCLVKGYFP
EPVTVTWNSG SLSSGVHTFP AVLQSDLYTL SSSVTVPSST WPSETVTCNV
AHPASSTKVD KKIVPRDCGC KPCICTVPEV SSVFIFPPKP KDVLTITLTP
KVTCVVVDIS KDDPEVQFSW FVDDVEVHTA QTQPREEQFN STFRSVSELP
IMHQDWLNGK EFKCRVNSAA FPAPIEKTIS KTKGRPKAPQ VYTIPPPKEQ
MAKDKVSLTC MITDFFPEDI TVEWQWNGQP AENYKNTQPI MDTDGSYFVY
SKLNVQKSNW EAGNTFTCSV LHEGLHNHHT EKSLSHSPGK

### SEQ ID NO: 40

Amino acid sequence of **FR1** of the light chain variable domain (VL) of the antibody 4-H2:
DVSMTQSPLSLPVSLGDQVSISCRSS

### SEQ ID NO: 41

Amino acid sequence of **FR2** of the light chain variable domain (VL) of the antibody 4-H2:
LEWFLQRPGQSPKLLIY

### SEQ ID NO: 42

Amino acid sequence of **FR3** of the light chain variable domain (VL) of the antibody 4-H2:
NRFSGVPDRFSGSGSGTDFTLKISRVEAEDLGVYYC

### SEQ ID NO: 43

Amino acid sequence of **FR4** of the light chain variable domain (VL) of the antibody 4-H2:

FGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC

### SEQ ID NO: 44

Amino acid sequence of **FR1** of the heavy chain variable domain (VH) of the antibody 43-3:
QIQLVQSGPELKKPGETVKISCKAS

### SEQ ID NO: 45

Amino acid sequence of **FR2** of the heavy chain variable domain (VH) of the antibody 43-3:
MNWVKQAPGKDLKWMGW

### SEQ ID NO: 46

Amino acid sequence of **FR3** of the heavy chain variable domain (VH) of the antibody 43-3:
YADDFKGRFVFSLETSASTAYLQINNLNNKDTATYFC

### SEQ ID NO: 47

Amino acid sequence of **FR4** of the heavy chain variable domain (VH) of the antibody 43-3:

WGA

GTTVTVSSAK TTPPSVYPLA PGCGDTTGSS VTLGCLVKGY FPESVTVTWN

SGSLSSSVHT FPALLQSGLY TMSSSVTVPS STWPSQTVTC SVAHPASSTT

VDKKLEPSGP ISTINPCPPC KECHKCPAPN LEGGPSVFIF PPNIKDVLMI

SLTPKVTCVV VDVSEDDPDV RISWFVNNVE VHTAQTQTHR EDYNSTIRVV

SALPIQHQDW MSGKEFKCKV NNKDLPSPIE RTISKIKGLV RAPQVYILPP

PAEQLSRKDV SLTCLVVGFN PGDISVEWTS NGHTEENYKD TAPVLDSDGS

YFIYSKLDIK TSKWEKTDSF SCNVRHEGLK NYYLKKTISR SPGK

### SEQ ID NO: 48

Amino acid sequence of **FR1** of the light chain variable domain (VL) of the antibody 43-3:
DVLMTQTPLSLPVSLGDQASISCRSS

### SEQ ID NO: 49

Amino acid sequence of **FR2** of the light chain variable domain (VL) of the antibody 43-3:
LEWYLQKPGQSPKLLIY

### SEQ ID NO: 50

Amino acid sequence of **FR3** of the light chain variable domain (VL) of the antibody 43-3:
NRFSGVPDRFSGSGSGTDFTLKITRVEAEDLGVYYC

### SEQ ID NO: 51

Amino acid sequence of **FR4** of the light chain variable domain (VL) of the antibody 43-3:

FGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC

### SEQ ID NO: 52

Amino acid sequence of **FR1** of the heavy chain variable domain (VH) of the antibody 141-3:
EVKLEQSGPE LKKPGETVKI SCMAS

### SEQ ID NO: 53

Amino acid sequence of **FR2** of the heavy chain variable domain (VH) of the antibody 141-3:
IHWVRQAPGK GLKWMAW

### SEQ ID NO: 54

Amino acid sequence of **FR3** of the heavy chain variable domain (VH) of the antibody 141-3:
TYADDFKGRF AFSLETSAST AYLQINNLEN EDTATYYC

### SEQ ID NO: 55

Amino acid sequence of **FR4** of the heavy chain variable domain (VH) of the antibody 141-3:

W
GQGTSVTVSS AKTTPPSVYP LAPGCGDTTG SSVTLGCLVK GYFPESVTVT
WNSGSLSSSV HTFPALLQSG LYTMSSSVTV PSSTWPSQTV TCSVAHPASS
TTVDKKLEPS GPISTINPCP PCKECHKCPA PNLEGGPSVF IFPPNIKDVL
MISLTPKVTC VVVDVSEDDP DVRISWFVNN VEVHTAQTQT HREDYNSTIR
VVSALPIQHQ DWMSGKEFKC KVNNKDLPSP IERTISKIKG LVRAPQVYIL
PPPAEQLSRK DVSLTCLVVG FNPGDISVEW TSNGHTEENY KDTAPVLDSD
GSYFIYSKLD IKTSKWEKTD SFSCNVRHEG LKNYYLKKTI SRSPGK

### SEQ ID NO: 56

Amino acid sequence of **FR1** of the light chain variable domain (VL) of the antibody 141-3:
DVQITQSPSY LAASPGETIT INCRAS

### SEQ ID NO: 57

Amino acid sequence of **FR2** of the light chain variable domain (VL) of the antibody 43-3:
LAWYQEKPGK TIKLLIY

### SEQ ID NO: 58

Amino acid sequence of **FR3** of the light chain variable domain (VL) of the antibody 141-3:
TLQSGIPSRF SGSGSGRDFT LIISSLEPED IAMYYC

### SEQ ID NO: 59

Amino acid sequence of **FR4** of the light chain variable domain (VL) of the antibody 141-3:

FGA GTKLELKRAD AAPTVSIFPP SSEQLTSGGA SVVCFLNNFY PKDINVKWKI
DGSERQNGVL NSWTDQDSKD STYSMSSTLT LTKDEYERHN SYTCEATHKT STSPIVKSFN
RNEC

## Claims

1. An antibody or an antibody derivative that bind to human HMGI-C, wherein the antibody comprises
- in the heavy chain variable region ONE (VH-1) one amino acid CDR sequence CDR3 of SEQ ID NO. 4;
OR
- in the heavy chain variable region TWO (VH-2) at least one amino acid CDR sequence CDR3 of SEQ ID NO. 14.

2. The antibody or antibody derivative according to claim 1, wherein the antibody or antibody derivative is effective to identify the presence of human HMGI-C in liquids *in vitro;* and/or
wherein the antibody or antibody derivative is effective to treat cancer related to human HMGI-C.

3. The antibody or antibody derivative according to claims 1 or 2, wherein the antibody or antibody derivative comprises
- in the heavy chain variable region ONE (VH-1) together with the amino acid sequence CDR3 of SEQ ID NO. 4 the amino acid sequence CDR1 of SEQ ID NO. 2 and the amino acid sequence CDR2 of SEQ ID NO. 3;
OR
- in the heavy chain variable region TWO (VH-2) together with the amino acid sequence CDR3 of SEQ ID NO. 14 the amino acid sequence CDR1 of SEQ ID NO. 12 and the amino acid sequence CDR2 of SEQ ID NO. 13.

4. The antibody or antibody derivative according to any one of claims 1 to 3, wherein the antibody or antibody derivative comprises
- in the heavy chain variable region ONE (VH-1) amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
OR
- in the heavy chain variable region TWO (VH-2) amino acid sequence CDR1 of SEQ ID NO.12, amino acid sequence CDR2 of SEQ ID NO. 13, and amino acid sequence CDR3 of SEQ ID NO. 14 and
- in the light chain variable region TWO (VL-2) amino acid sequence CDR1 of SEQ ID NO. 17, amino acid sequence CDR2 of SEQ ID NO. 18 and amino acid sequence CDR3 of SEQ ID NO. 19.

5. The antibody or antibody derivative according to any one of claims 1 to 4, wherein the antibody or antibody derivative comprises:
a) a heavy chain variable region ONE (VH-1) encoded by the nucleic acid sequence of SEQ ID NO. 5 and a light chain variable region ONE (VL-1) encoded by the nucleic acid sequence of SEQ ID NO. 10; or
b) a heavy chain variable region ONE (VH-1) having amino acid sequence of SEQ ID NO. 1 and a light chain variable region ONE (VL-1) having amino acid sequence of SEQ ID NO. 6;
OR
a) a heavy chain variable region TWO (VH-2) encoded by nucleic acid sequences of SEQ ID NO. 15 and a light chain variable region TWO (VL-2) encoded by nucleic acid sequences of SEQ ID NO. 20; or
b) a heavy chain variable region TWO (VH-2) having amino acid sequence of SEQ ID NO. 11 and a light chain variable region TWO (VL-2) having amino acid sequence of SEQ ID NO. 16.

6. The antibody or antibody derivative according to any one of claims 1 to 5, wherein
- for the antibody with the heavy chain variable region ONE (VH-1) with the CDR sequence CDR3 of SEQ ID NO. 4, the antibody or antibody derivative comprises a heavy chain variable region with at least 95%, 99% or 100% sequence identity to SEQ ID NO: 1 and a light chain variable region with at least 95%, 99% or 100% sequence identity to SEQ ID NO: 6;
OR
- for the antibody with the heavy chain variable region TWO (VH-2) with the CDR sequence CDR3 of SEQ ID NO. 14, the antibody or antibody derivative comprises a heavy chain variable region with at least 95%, 99% or 100% sequence identity to SEQ ID NO: 11 and a light chain variable region with at least 95%, 99% or 100% sequence identity to SEQ ID NO: 16.

7. An antibody or an antibody derivative that bind to human HMGI-C, wherein the antibody comprises
- in the heavy chain variable region THREE (VH-3) (the three CDRs of) amino acid sequence CDR1 of SEQ ID NO.22, amino acid sequence CDR2 of SEQ ID NO.23, and amino acid sequence CDR3 of SEQ ID NO.24 and
- in the light chain variable region THREE (VL-3) amino acid sequence CDR1 of SEQ ID NO. 27, amino acid sequence CDR2 of SEQ ID NO. 28 and amino acid sequence CDR3 of SEQ ID NO. 29.

8. The antibody or antibody derivative according to claim 7, wherein the antibody or antibody derivative comprises a heavy chain variable region with at least 95%, 99% or 100% sequence identity to SEQ ID NO: 21 and a light chain variable region with at least 95%, 99% or 100% sequence identity to SEQ ID NO: 26.

9. The antibody or antibody derivative according to any one of claims 1 to 8,
- wherein the antibody is a murine antibody, such as an IgG, or
- wherein said antibody is a human or humanized antibody, especially a humanized antibody, in particular wherein said humanized antibody is an IgG and/or
- wherein said antibody is a monoclonal antibody; and/or
- wherein said antibody derivative is a Fab fragment; and/or
- wherein the said antibody or the said antibody derivative
∘ which comprises in the heavy chain variable region ONE (VH-1) amino acid CDR sequence CDR3 of SEQ ID NO. 4, binds to Epitope 1 of SEQ ID NO. 32;
∘ which comprises in the heavy chain variable region TWO (VH-2) amino acid CDR sequence CDR3 of SEQ ID NO. 14, binds to Epitope 2 of SEQ ID NO. 33.

10. An isolated nucleic acid or an expression vector that encodes the antibody or antibody derivative according to any one of claims 1 to 9.

11. A host cell comprising the nucleic acid or the expression vector according to claim 10, preferably wherein the host cell produces the antibody or antibody derivative according to any one of claims 1 to 9.

12. A method of producing the antibody or antibody derivative according to any one of claims 1 to 9, comprising culturing the host cell according to claim 11 that produces the antibody and recovering the antibody from the cell culture.

13. A composition comprising the antibody or antibody derivative according to any one of claims 1 to 9 and a carrier, preferably a pharmaceutically acceptable carrier.

14. An article of manufacture or medical device comprising a container and a composition contained therein, wherein the composition comprises an antibody or antibody derivative according to any one of claims 1 to 9, preferably wherein the medical device is a lateral flow device for detection of an antigen, most preferably herein the medical device is a lateral flow device for detection of the presence of human HMGI-C in a body fluid, like blood, whole blood, blood plasma or serum.

15. An antibody according to any one of claims 1 to 9 for use in a method of treating HMGI-C positive cancer, wherein the method comprises the step of contacting a HMGI-C-positive cancer in a subject with said antibody or antibody derivative, preferably wherein said HMGI-C positive cancer is selected from ovarian cancer, especially epithelial ovarian cancer; nasopharyngeal carcinoma, renal cell carcinoma, epithelial cancer, prostate cancer, endometrial cancer, pancreatic cancer, laryngeal squamous cell carcinoma, bladder cancer, oral squamous cell carcinoma, cervical cancer, head and neck squamous cell carcinoma, hepatocellular carcinoma, tongue squamous cell carcinoma, especially oral tongue squamous cell carcinoma; and osteosarcoma.
